# EUROPEAN PATENT APPLICATION

(11) **EP 2 966 085 A1**
(43) Date of publication of application: **13.01.2016**
(21) Application number: 14176746.7
(22) Date of filing: 11.07.2014
(51) Int. Cl.: C07K 16/00, C07K 16/28

(54) **Antibody IgG1 with a modified heavy chain constant region**

(71) Applicant: Boehringer Ingelheim International GmbH, 55216 Ingelheim am Rhein (DE)
(72) Inventor: Enenkel, Barbara, 88447 Warthausen (DE); Koenitzer, Jennifer, 89073 Ulm (DE)
(74) Representative: Oetke, Cornelia

(57) **Abstract**

The present invention *inter alia* relates to modified antibodies and in particular to therapeutic modified antibodies of the IgG1 subtype. The antibodies are modified in the CH1 domain and/or upper hinge region and show enhanced direct cytotoxic activity compared to the corresponding unmodified antibody. Said antibodies therefore are useful in therapy, particularly in cancer therapy.

SEQUENCE LISTING

<110> Boehringer Ingelheim International GmbH

<120> MODIFIED ANTIBODIES

<130> BOE14880EP

<160> 162

<170> BiSSAP 1.3

<210> 1
<211> 330
<212> PRT
<213> Homo sapiens

<220>
<223> IgG1 a1 residues 118-447

<400> 1
Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Arg Val Glu Pro Lys Ser Cys Asp Lys Thr His Thr Cys Pro Pro Cys
            100                 105                 110
Pro Ala Pro Glu Leu Leu Gly Gly Pro Ser Val Phe Leu Phe Pro Pro
        115                 120                 125
Lys Pro Lys Asp Thr Leu Met Ile Ser Arg Thr Pro Glu Val Thr Cys
    130                 135                 140
Val Val Val Asp Val Ser His Glu Asp Pro Glu Val Lys Phe Asn Trp
145                 150                 155                 160
Tyr Val Asp Gly Val Glu Val His Asn Ala Lys Thr Lys Pro Arg Glu
                165                 170                 175
Glu Gln Tyr Asn Ser Thr Tyr Arg Val Val Ser Val Leu Thr Val Leu
            180                 185                 190
His Gln Asp Trp Leu Asn Gly Lys Glu Tyr Lys Cys Lys Val Ser Asn
        195                 200                 205
Lys Ala Leu Pro Ala Pro Ile Glu Lys Thr Ile Ser Lys Ala Lys Gly
    210                 215                 220
Gln Pro Arg Glu Pro Gln Val Tyr Thr Leu Pro Pro Ser Arg Asp Glu
225                 230                 235                 240
Leu Thr Lys Asn Gln Val Ser Leu Thr Cys Leu Val Lys Gly Phe Tyr
                245                 250                 255
Pro Ser Asp Ile Ala Val Glu Trp Glu Ser Asn Gly Gln Pro Glu Asn
            260                 265                 270
Asn Tyr Lys Thr Thr Pro Pro Val Leu Asp Ser Asp Gly Ser Phe Phe
        275                 280                 285
Leu Tyr Ser Lys Leu Thr Val Asp Lys Ser Arg Trp Gln Gln Gly Asn
    290                 295                 300
Val Phe Ser Cys Ser Val Met His Glu Ala Leu His Asn His Tyr Thr
305                 310                 315                 320
Gln Lys Ser Leu Ser Leu Ser Pro Gly Lys
                325                 330

<210> 2
<211> 330
<212> PRT
<213> Homo sapiens

<220>
<223> IgG1 a2 residues 118-447

<400> 2
Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Arg Val Glu Pro Lys Ser Cys Asp Lys Thr His Thr Cys Pro Pro Cys
            100                 105                 110
Pro Ala Pro Glu Leu Leu Gly Gly Pro Ser Val Phe Leu Phe Pro Pro
        115                 120                 125
Lys Pro Lys Asp Thr Leu Met Ile Ser Arg Thr Pro Glu Val Thr Cys
    130                 135                 140
Val Val Val Asp Val Ser His Glu Asp Pro Glu Val Lys Phe Asn Trp
145                 150                 155                 160
Tyr Val Asp Gly Val Glu Val His Asn Ala Lys Thr Lys Pro Arg Glu
                165                 170                 175
Glu Gln Tyr Asn Ser Thr Tyr Arg Val Val Ser Val Leu Thr Val Leu
            180                 185                 190
His Gln Asp Trp Leu Asn Gly Lys Glu Tyr Lys Cys Lys Val Ser Asn
        195                 200                 205
Lys Ala Leu Pro Ala Pro Ile Glu Lys Thr Ile Ser Lys Ala Lys Gly
    210                 215                 220
Gln Pro Arg Glu Pro Gln Val Tyr Thr Leu Pro Pro Ser Arg Glu Glu
225                 230                 235                 240
Met Thr Lys Asn Gln Val Ser Leu Thr Cys Leu Val Lys Gly Phe Tyr
                245                 250                 255
Pro Ser Asp Ile Ala Val Glu Trp Glu Ser Asn Gly Gln Pro Glu Asn
            260                 265                 270
Asn Tyr Lys Thr Thr Pro Pro Val Leu Asp Ser Asp Gly Ser Phe Phe
        275                 280                 285
Leu Tyr Ser Lys Leu Thr Val Asp Lys Ser Arg Trp Gln Gln Gly Asn
    290                 295                 300
Val Phe Ser Cys Ser Val Met His Glu Ala Leu His Asn His Tyr Thr
305                 310                 315                 320
Gln Lys Ser Leu Ser Leu Ser Pro Gly Lys
                325                 330

<210> 3
<211> 326
<212> PRT
<213> Homo sapiens

<220>
<223> IgG2 residues 118-443

<400> 3
Ala Ser Thr Lys Gly Pro Ser Val Phe Pro Leu Ala Pro Cys Ser Arg

1               5                   10                  15
Ser Thr Ser Glu Ser Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Asn Phe Gly Thr Gln Thr
65                  70                  75                  80
Tyr Thr Cys Asn Val Asp His Lys Pro Ser Asn Thr Lys Val Asp Lys
                85                  90                  95
Thr Val Glu Arg Lys Cys Cys Val Glu Cys Pro Pro Cys Pro Ala Pro
            100                 105                 110
Pro Val Ala Gly Pro Ser Val Phe Leu Phe Pro Pro Lys Pro Lys Asp
        115                 120                 125
Thr Leu Met Ile Ser Arg Thr Pro Glu Val Thr Cys Val Val Val Asp
    130                 135                 140
Val Ser His Glu Asp Pro Glu Val Gln Phe Asn Trp Tyr Val Asp Gly
145                 150                 155                 160
Val Glu Val His Asn Ala Lys Thr Lys Pro Arg Glu Glu Gln Phe Asn
                165                 170                 175
Ser Thr Phe Arg Val Val Ser Val Leu Thr Val Val His Gln Asp Trp
            180                 185                 190
Leu Asn Gly Lys Glu Tyr Lys Cys Lys Val Ser Asn Lys Gly Leu Pro
        195                 200                 205
Ala Pro Ile Glu Lys Thr Ile Ser Lys Thr Lys Gly Gln Pro Arg Glu
    210                 215                 220
Pro Gln Val Tyr Thr Leu Pro Pro Ser Arg Glu Glu Met Thr Lys Asn
225                 230                 235                 240
Gln Val Ser Leu Thr Cys Leu Val Lys Gly Phe Tyr Pro Ser Asp Ile
                245                 250                 255
Ala Val Glu Trp Glu Ser Asn Gly Gln Pro Glu Asn Asn Tyr Lys Thr
            260                 265                 270
Thr Pro Pro Met Leu Asp Ser Asp Gly Ser Phe Phe Leu Tyr Ser Lys
        275                 280                 285
Leu Thr Val Asp Lys Ser Arg Trp Gln Gln Gly Asn Val Phe Ser Cys
    290                 295                 300
Ser Val Met His Glu Ala Leu His Asn His Tyr Thr Gln Lys Ser Leu
305                 310                 315                 320
Ser Leu Ser Pro Gly Lys
                325

<210> 4
<211> 327
<212> PRT
<213> Homo sapiens

<220>
<223> IgG4P residues 118-444

<400> 4
Ala Ser Thr Lys Gly Pro Ser Val Phe Pro Leu Ala Pro Cys Ser Arg
1               5                   10                  15
Ser Thr Ser Glu Ser Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Lys Thr
65                  70                  75                  80
Tyr Thr Cys Asn Val Asp His Lys Pro Ser Asn Thr Lys Val Asp Lys
                85                  90                  95

Arg Val Glu Ser Lys Tyr Gly Pro Pro Cys Pro Pro Cys Pro Ala Pro
            100                 105                 110
Glu Phe Leu Gly Gly Pro Ser Val Phe Leu Phe Pro Pro Lys Pro Lys
        115                 120                 125
Asp Thr Leu Met Ile Ser Arg Thr Pro Glu Val Thr Cys Val Val Val
    130                 135                 140
Asp Val Ser Gln Glu Asp Pro Glu Val Gln Phe Asn Trp Tyr Val Asp
145                 150                 155                 160
Gly Val Glu Val His Asn Ala Lys Thr Lys Pro Arg Glu Glu Gln Phe
                165                 170                 175
Asn Ser Thr Tyr Arg Val Val Ser Val Leu Thr Val Leu His Gln Asp
            180                 185                 190
Trp Leu Asn Gly Lys Glu Tyr Lys Cys Lys Val Ser Asn Lys Gly Leu
        195                 200                 205
Pro Ser Ser Ile Glu Lys Thr Ile Ser Lys Ala Lys Gly Gln Pro Arg
    210                 215                 220
Glu Pro Gln Val Tyr Thr Leu Pro Pro Ser Gln Glu Glu Met Thr Lys
225                 230                 235                 240
Asn Gln Val Ser Leu Thr Cys Leu Val Lys Gly Phe Tyr Pro Ser Asp
                245                 250                 255
Ile Ala Val Glu Trp Glu Ser Asn Gly Gln Pro Glu Asn Asn Tyr Lys
            260                 265                 270
Thr Thr Pro Pro Val Leu Asp Ser Asp Gly Ser Phe Phe Leu Tyr Ser
        275                 280                 285
Arg Leu Thr Val Asp Lys Ser Arg Trp Gln Glu Gly Asn Val Phe Ser
    290                 295                 300
Cys Ser Val Met His Glu Ala Leu His Asn His Tyr Thr Gln Lys Ser
305                 310                 315                 320
Leu Ser Leu Ser Leu Gly Lys
                325

<210> 5
<211> 98
<212> PRT
<213> Homo sapiens

<220>
<223> IgG1 residues 118-215

<400> 5
Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Arg Val


<210> 6
<211> 10
<212> PRT
<213> Homo sapiens

<220>
<223> IgG1 residues 216-225

<400> 6
Glu Pro Lys Ser Cys Asp Lys Thr His Thr
1               5                   10

<210> 7
<211> 15
<212> PRT
<213> Homo sapiens

<220>
<223> IgG1 residues 216-230

<400> 7
Glu Pro Lys Ser Cys Asp Lys Thr His Thr Cys Pro Pro Cys Pro
1               5                   10                  15

<210> 8
<211> 110
<212> PRT
<213> Homo sapiens

<220>
<223> IgG1 residues 231-340

<400> 8
Ala Pro Glu Leu Leu Gly Gly Pro Ser Val Phe Leu Phe Pro Pro Lys
1               5                   10                  15
Pro Lys Asp Thr Leu Met Ile Ser Arg Thr Pro Glu Val Thr Cys Val
            20                  25                  30
Val Val Asp Val Ser His Glu Asp Pro Glu Val Lys Phe Asn Trp Tyr
        35                  40                  45
Val Asp Gly Val Glu Val His Asn Ala Lys Thr Lys Pro Arg Glu Glu
    50                  55                  60
Gln Tyr Asn Ser Thr Tyr Arg Val Val Ser Val Leu Thr Val Leu His
65                  70                  75                  80
Gln Asp Trp Leu Asn Gly Lys Glu Tyr Lys Cys Lys Val Ser Asn Lys
                85                  90                  95
Ala Leu Pro Ala Pro Ile Glu Lys Thr Ile Ser Lys Ala Lys
            100                 105                 110

<210> 9
<211> 22
<212> PRT
<213> Artificial Sequence

<220>
<223> D221 deletion

<400> 9
Glu Pro Lys Ser Cys Lys Thr His Thr Cys Pro Pro Cys Pro Ala Pro
1               5                   10                  15
Glu Leu Leu Gly Gly Pro
            20

<210> 10
<211> 22
<212> PRT
<213> Artificial Sequence

<220>
<223> K222 deletion

<400> 10

Glu Pro Lys Ser Cys Asp Thr His Thr Cys Pro Pro Cys Pro Ala Pro
1               5                   10                  15
Glu Leu Leu Gly Gly Pro
            20

<210> 11
<211> 22
<212> PRT
<213> Artificial Sequence

<220>
<223> T223 deletion

<400> 11
Glu Pro Lys Ser Cys Asp Lys His Thr Cys Pro Pro Cys Pro Ala Pro
1               5                   10                  15
Glu Leu Leu Gly Gly Pro
            20

<210> 12
<211> 21
<212> PRT
<213> Artificial Sequence

<220>
<223> DT deletion

<400> 12
Glu Pro Lys Ser Cys Lys His Thr Cys Pro Pro Cys Pro Ala Pro Glu
1               5                   10                  15
Leu Leu Gly Gly Pro
            20

<210> 13
<211> 21
<212> PRT
<213> Artificial Sequence

<220>
<223> DK deletion

<400> 13
Glu Pro Lys Ser Cys Thr His Thr Cys Pro Pro Cys Pro Ala Pro Glu
1               5                   10                  15
Leu Leu Gly Gly Pro
            20

<210> 14
<211> 21
<212> PRT
<213> Artificial Sequence

<220>
<223> KT deletion

<400> 14
Glu Pro Lys Ser Cys Asp His Thr Cys Pro Pro Cys Pro Ala Pro Glu
1               5                   10                  15
Leu Leu Gly Gly Pro
            20

<210> 15
<211> 20

<212> PRT
<213> Artificial Sequence

<220>
<223> DKT deletion

<400> 15
Glu Pro Lys Ser Cys His Thr Cys Pro Pro Cys Pro Ala Pro Glu Leu
1               5                   10                  15
Leu Gly Gly Pro
            20

<210> 16
<211> 22
<212> PRT
<213> Artificial Sequence

<220>
<223> lh2

<400> 16
Glu Pro Lys Ser Cys Asp Lys Thr His Thr Cys Pro Pro Cys Pro Ala
1               5                   10                  15
Pro Pro Val Ala Gly Pro
            20

<210> 17
<211> 23
<212> PRT
<213> Artificial Sequence

<220>
<223> lh4

<400> 17
Glu Pro Lys Ser Cys Asp Lys Thr His Thr Cys Pro Pro Cys Pro Ala
1               5                   10                  15
Pro Glu Phe Leu Gly Gly Pro
            20

<210> 18
<211> 22
<212> PRT
<213> Artificial Sequence

<220>
<223> uch2

<400> 18
Thr Val Glu Arg Lys Cys Cys Val Glu Cys Pro Pro Cys Pro Ala Pro
1               5                   10                  15
Glu Leu Leu Gly Gly Pro
            20

<210> 19
<211> 20
<212> PRT
<213> Artificial Sequence

<220>
<223> uch4

<400> 19

Glu Ser Lys Tyr Gly Pro Pro Cys Pro Pro Cys Pro Ala Pro Glu Leu
1               5                   10                  15
Leu Gly Gly Pro
            20

<210> 20
<211> 23
<212> PRT
<213> Artificial Sequence

<220>
<223> P217R

<400> 20
Glu Arg Lys Ser Cys Asp Lys Thr His Thr Cys Pro Pro Cys Pro Ala
1               5                   10                  15
Pro Glu Leu Leu Gly Gly Pro
            20

<210> 21
<211> 23
<212> PRT
<213> Artificial Sequence

<220>
<223> P217S

<400> 21
Glu Ser Lys Ser Cys Asp Lys Thr His Thr Cys Pro Pro Cys Pro Ala
1               5                   10                  15
Pro Glu Leu Leu Gly Gly Pro
            20

<210> 22
<211> 23
<212> PRT
<213> Artificial Sequence

<220>
<223> S219C

<400> 22
Glu Pro Lys Cys Cys Asp Lys Thr His Thr Cys Pro Pro Cys Pro Ala
1               5                   10                  15
Pro Glu Leu Leu Gly Gly Pro
            20

<210> 23
<211> 23
<212> PRT
<213> Artificial Sequence

<220>
<223> S219Y

<400> 23
Glu Pro Lys Tyr Cys Asp Lys Thr His Thr Cys Pro Pro Cys Pro Ala
1               5                   10                  15
Pro Glu Leu Leu Gly Gly Pro
            20

<210> 24
<211> 23

<212> PRT
<213> Artificial Sequence

<220>
<223> C220G

<400> 24
Glu Pro Lys Ser Gly Asp Lys Thr His Thr Cys Pro Pro Cys Pro Ala
1               5                   10                  15
Pro Glu Leu Leu Gly Gly Pro
            20

<210> 25
<211> 23
<212> PRT
<213> Artificial Sequence

<220>
<223> S219C-C220G

<400> 25
Glu Pro Lys Cys Gly Asp Lys Thr His Thr Cys Pro Pro Cys Pro Ala
1               5                   10                  15
Pro Glu Leu Leu Gly Gly Pro
            20

<210> 26
<211> 20
<212> PRT
<213> Artificial Sequence

<220>
<223> DKT-S219C

<400> 26
Glu Pro Lys Cys Cys His Thr Cys Pro Pro Cys Pro Ala Pro Glu Leu
1               5                   10                  15
Leu Gly Gly Pro
            20

<210> 27
<211> 21
<212> PRT
<213> Artificial Sequence

<220>
<223> DK-S219C

<400> 27
Glu Pro Lys Cys Cys Thr His Thr Cys Pro Pro Cys Pro Ala Pro Glu
1               5                   10                  15
Leu Leu Gly Gly Pro
            20

<210> 28
<211> 21
<212> PRT
<213> Artificial Sequence

<220>
<223> DT-S219C

<400> 28

Glu Pro Lys Cys Cys Lys His Thr Cys Pro Pro Cys Pro Ala Pro Glu
1               5                   10                  15
Leu Leu Gly Gly Pro
            20

<210> 29
<211> 21
<212> PRT
<213> Artificial Sequence

<220>
<223> KT-S219C

<400> 29
Glu Pro Lys Cys Cys Asp His Thr Cys Pro Pro Cys Pro Ala Pro Glu
1               5                   10                  15
Leu Leu Gly Gly Pro
            20

<210> 30
<211> 23
<212> PRT
<213> Artificial Sequence

<220>
<223> H224V

<400> 30
Glu Pro Lys Ser Cys Asp Lys Thr Val Thr Cys Pro Pro Cys Pro Ala
1               5                   10                  15
Pro Glu Leu Leu Gly Gly Pro
            20

<210> 31
<211> 23
<212> PRT
<213> Artificial Sequence

<220>
<223> H224P

<400> 31
Glu Pro Lys Ser Cys Asp Lys Thr Pro Thr Cys Pro Pro Cys Pro Ala
1               5                   10                  15
Pro Glu Leu Leu Gly Gly Pro
            20

<210> 32
<211> 23
<212> PRT
<213> Artificial Sequence

<220>
<223> T225P

<400> 32
Glu Pro Lys Ser Cys Asp Lys Thr His Pro Cys Pro Pro Cys Pro Ala
1               5                   10                  15
Pro Glu Leu Leu Gly Gly Pro
            20

<210> 33
<211> 23

<212> PRT
<213> Artificial Sequence

<220>
<223> T225E

<400> 33
Glu Pro Lys Ser Cys Asp Lys Thr His Glu Cys Pro Pro Cys Pro Ala
1               5                   10                  15
Pro Glu Leu Leu Gly Gly Pro
            20

<210> 34
<211> 23
<212> PRT
<213> Artificial Sequence

<220>
<223> H224P-T225P

<400> 34
Glu Pro Lys Ser Cys Asp Lys Thr Pro Pro Cys Pro Pro Cys Pro Ala
1               5                   10                  15
Pro Glu Leu Leu Gly Gly Pro
            20

<210> 35
<211> 23
<212> PRT
<213> Artificial Sequence

<220>
<223> H224V-T225E

<400> 35
Glu Pro Lys Ser Cys Asp Lys Thr Val Glu Cys Pro Pro Cys Pro Ala
1               5                   10                  15
Pro Glu Leu Leu Gly Gly Pro
            20

<210> 36
<211> 84
<212> PRT
<213> Artificial Sequence

<220>
<223> 1CH1

<400> 36
Ser Ser Lys Ser Thr Ser Gly Gly Thr Ala Ala Leu Gly Cys Leu Val
1               5                   10                  15
Lys Asp Tyr Phe Pro Glu Pro Val Thr Val Ser Trp Asn Ser Gly Ala
            20                  25                  30
Leu Thr Ser Gly Val His Thr Phe Pro Ala Val Leu Gln Ser Ser Gly
        35                  40                  45
Leu Tyr Ser Leu Ser Ser Val Val Thr Val Pro Ser Ser Ser Leu Gly
    50                  55                  60
Thr Gln Thr Tyr Thr Cys Asn Val Asp His Lys Pro Ser Asn Thr Lys
65                  70                  75                  80
Val Asp Lys Arg

<210> 37

<211> 84
<212> PRT
<213> Artificial Sequence

<220>
<223> uCH1

<400> 37
Cys Ser Lys Ser Thr Ser Glu Ser Thr Ala Ala Leu Gly Cys Leu Val
1               5                   10                  15
Lys Asp Tyr Phe Pro Glu Pro Val Thr Val Ser Trp Asn Ser Gly Ala
            20                  25                  30
Leu Thr Ser Gly Val His Thr Phe Pro Ala Val Leu Gln Ser Ser Gly
        35                  40                  45
Leu Tyr Ser Leu Ser Ser Val Val Thr Val Pro Ser Ser Ser Leu Gly
    50                  55                  60
Thr Gln Thr Tyr Ile Cys Asn Val Asn His Lys Pro Ser Asn Thr Lys
65                  70                  75                  80
Val Asp Lys Arg


<210> 38
<211> 84
<212> PRT
<213> Artificial Sequence

<220>
<223> S131C

<400> 38
Cys Ser Lys Ser Thr Ser Gly Gly Thr Ala Ala Leu Gly Cys Leu Val
1               5                   10                  15
Lys Asp Tyr Phe Pro Glu Pro Val Thr Val Ser Trp Asn Ser Gly Ala
            20                  25                  30
Leu Thr Ser Gly Val His Thr Phe Pro Ala Val Leu Gln Ser Ser Gly
        35                  40                  45
Leu Tyr Ser Leu Ser Ser Val Val Thr Val Pro Ser Ser Ser Leu Gly
    50                  55                  60
Thr Gln Thr Tyr Ile Cys Asn Val Asn His Lys Pro Ser Asn Thr Lys
65                  70                  75                  80
Val Asp Lys Arg


<210> 39
<211> 84
<212> PRT
<213> Artificial Sequence

<220>
<223> K133R

<400> 39
Ser Ser Arg Ser Thr Ser Gly Gly Thr Ala Ala Leu Gly Cys Leu Val
1               5                   10                  15
Lys Asp Tyr Phe Pro Glu Pro Val Thr Val Ser Trp Asn Ser Gly Ala
            20                  25                  30
Leu Thr Ser Gly Val His Thr Phe Pro Ala Val Leu Gln Ser Ser Gly
        35                  40                  45
Leu Tyr Ser Leu Ser Ser Val Val Thr Val Pro Ser Ser Ser Leu Gly
    50                  55                  60
Thr Gln Thr Tyr Ile Cys Asn Val Asn His Lys Pro Ser Asn Thr Lys
65                  70                  75                  80
Val Asp Lys Arg

<210> 40
<211> 84
<212> PRT
<213> Artificial Sequence

<220>
<223> S131C-K133R

<400> 40
Cys Ser Arg Ser Thr Ser Gly Gly Thr Ala Ala Leu Gly Cys Leu Val
1               5                   10                  15
Lys Asp Tyr Phe Pro Glu Pro Val Thr Val Ser Trp Asn Ser Gly Ala
            20                  25                  30
Leu Thr Ser Gly Val His Thr Phe Pro Ala Val Leu Gln Ser Ser Gly
        35                  40                  45
Leu Tyr Ser Leu Ser Ser Val Val Thr Val Pro Ser Ser Ser Leu Gly
    50                  55                  60
Thr Gln Thr Tyr Ile Cys Asn Val Asn His Lys Pro Ser Asn Thr Lys
65                  70                  75                  80
Val Asp Lys Arg


<210> 41
<211> 84
<212> PRT
<213> Artificial Sequence

<220>
<223> G137E-G138S

<400> 41
Ser Ser Lys Ser Thr Ser Glu Ser Thr Ala Ala Leu Gly Cys Leu Val
1               5                   10                  15
Lys Asp Tyr Phe Pro Glu Pro Val Thr Val Ser Trp Asn Ser Gly Ala
            20                  25                  30
Leu Thr Ser Gly Val His Thr Phe Pro Ala Val Leu Gln Ser Ser Gly
        35                  40                  45
Leu Tyr Ser Leu Ser Ser Val Val Thr Val Pro Ser Ser Ser Leu Gly
    50                  55                  60
Thr Gln Thr Tyr Ile Cys Asn Val Asn His Lys Pro Ser Asn Thr Lys
65                  70                  75                  80
Val Asp Lys Arg


<210> 42
<211> 84
<212> PRT
<213> Artificial Sequence

<220>
<223> S192N

<400> 42
Ser Ser Lys Ser Thr Ser Gly Gly Thr Ala Ala Leu Gly Cys Leu Val
1               5                   10                  15
Lys Asp Tyr Phe Pro Glu Pro Val Thr Val Ser Trp Asn Ser Gly Ala
            20                  25                  30
Leu Thr Ser Gly Val His Thr Phe Pro Ala Val Leu Gln Ser Ser Gly
        35                  40                  45
Leu Tyr Ser Leu Ser Ser Val Val Thr Val Pro Ser Ser Asn Leu Gly
    50                  55                  60

Thr Gln Thr Tyr Ile Cys Asn Val Asn His Lys Pro Ser Asn Thr Lys
65                  70                  75                  80
Val Asp Lys Arg

<210> 43
<211> 84
<212> PRT
<213> Artificial Sequence

<220>
<223> L193F

<400> 43
Ser Ser Lys Ser Thr Ser Gly Gly Thr Ala Ala Leu Gly Cys Leu Val
1               5                   10                  15
Lys Asp Tyr Phe Pro Glu Pro Val Thr Val Ser Trp Asn Ser Gly Ala
            20                  25                  30
Leu Thr Ser Gly Val His Thr Phe Pro Ala Val Leu Gln Ser Ser Gly
        35                  40                  45
Leu Tyr Ser Leu Ser Ser Val Val Thr Val Pro Ser Ser Ser Phe Gly
    50                  55                  60
Thr Gln Thr Tyr Ile Cys Asn Val Asn His Lys Pro Ser Asn Thr Lys
65                  70                  75                  80
Val Asp Lys Arg

<210> 44
<211> 84
<212> PRT
<213> Artificial Sequence

<220>
<223> S192N-L193F

<400> 44
Ser Ser Lys Ser Thr Ser Gly Gly Thr Ala Ala Leu Gly Cys Leu Val
1               5                   10                  15
Lys Asp Tyr Phe Pro Glu Pro Val Thr Val Ser Trp Asn Ser Gly Ala
            20                  25                  30
Leu Thr Ser Gly Val His Thr Phe Pro Ala Val Leu Gln Ser Ser Gly
        35                  40                  45
Leu Tyr Ser Leu Ser Ser Val Val Thr Val Pro Ser Ser Asn Phe Gly
    50                  55                  60
Thr Gln Thr Tyr Ile Cys Asn Val Asn His Lys Pro Ser Asn Thr Lys
65                  70                  75                  80
Val Asp Lys Arg

<210> 45
<211> 84
<212> PRT
<213> Artificial Sequence

<220>
<223> Q196K

<400> 45
Ser Ser Lys Ser Thr Ser Gly Gly Thr Ala Ala Leu Gly Cys Leu Val
1               5                   10                  15
Lys Asp Tyr Phe Pro Glu Pro Val Thr Val Ser Trp Asn Ser Gly Ala
            20                  25                  30
Leu Thr Ser Gly Val His Thr Phe Pro Ala Val Leu Gln Ser Ser Gly

35                  40                  45
Leu Tyr Ser Leu Ser Ser Val Val Thr Val Pro Ser Ser Ser Leu Gly
    50                  55                  60
Thr Lys Thr Tyr Ile Cys Asn Val Asn His Lys Pro Ser Asn Thr Lys
65                  70                  75                  80
Val Asp Lys Arg


<210> 46
<211> 84
<212> PRT
<213> Artificial Sequence

<220>
<223> R214T

<400> 46
Ser Ser Lys Ser Thr Ser Gly Gly Thr Ala Ala Leu Gly Cys Leu Val
1               5                   10                  15
Lys Asp Tyr Phe Pro Glu Pro Val Thr Val Ser Trp Asn Ser Gly Ala
            20                  25                  30
Leu Thr Ser Gly Val His Thr Phe Pro Ala Val Leu Gln Ser Ser Gly
        35                  40                  45
Leu Tyr Ser Leu Ser Ser Val Val Thr Val Pro Ser Ser Ser Leu Gly
    50                  55                  60
Thr Gln Thr Tyr Ile Cys Asn Val Asn His Lys Pro Ser Asn Thr Lys
65                  70                  75                  80
Val Asp Lys Thr


<210> 47
<211> 105
<212> PRT
<213> Artificial Sequence

<220>
<223> uch2-S131C

<400> 47
Cys Ser Lys Ser Thr Ser Gly Gly Thr Ala Ala Leu Gly Cys Leu Val
1               5                   10                  15
Lys Asp Tyr Phe Pro Glu Pro Val Thr Val Ser Trp Asn Ser Gly Ala
            20                  25                  30
Leu Thr Ser Gly Val His Thr Phe Pro Ala Val Leu Gln Ser Ser Gly
        35                  40                  45
Leu Tyr Ser Leu Ser Ser Val Val Thr Val Pro Ser Ser Ser Leu Gly
    50                  55                  60
Thr Gln Thr Tyr Ile Cys Asn Val Asn His Lys Pro Ser Asn Thr Lys
65                  70                  75                  80
Val Asp Lys Thr Val Glu Arg Lys Cys Cys Val Glu Cys Pro Pro Cys
                85                  90                  95
Pro Ala Pro Glu Leu Leu Gly Gly Pro
            100                 105

<210> 48
<211> 105
<212> PRT
<213> Artificial Sequence

<220>
<223> uch4-S131C

<400> 48

Cys Ser Lys Ser Thr Ser Gly Gly Thr Ala Ala Leu Gly Cys Leu Val
1               5                   10                  15
Lys Asp Tyr Phe Pro Glu Pro Val Thr Val Ser Trp Asn Ser Gly Ala
            20                  25                  30
Leu Thr Ser Gly Val His Thr Phe Pro Ala Val Leu Gln Ser Ser Gly
        35                  40                  45
Leu Tyr Ser Leu Ser Ser Val Val Thr Val Pro Ser Ser Ser Leu Gly
    50                  55                  60
Thr Gln Thr Tyr Ile Cys Asn Val Asn His Lys Pro Ser Asn Thr Lys
65                  70                  75                  80
Val Asp Lys Arg Val Glu Ser Lys Tyr Gly Pro Pro Cys Pro Pro Cys
                85                  90                  95
Pro Ala Pro Glu Leu Leu Gly Gly Pro
            100                 105

<210> 49
<211> 105
<212> PRT
<213> Artificial Sequence

<220>
<223> DKT-S131C

<400> 49
Cys Ser Lys Ser Thr Ser Gly Gly Thr Ala Ala Leu Gly Cys Leu Val
1               5                   10                  15
Lys Asp Tyr Phe Pro Glu Pro Val Thr Val Ser Trp Asn Ser Gly Ala
            20                  25                  30
Leu Thr Ser Gly Val His Thr Phe Pro Ala Val Leu Gln Ser Ser Gly
        35                  40                  45
Leu Tyr Ser Leu Ser Ser Val Val Thr Val Pro Ser Ser Ser Leu Gly
    50                  55                  60
Thr Gln Thr Tyr Ile Cys Asn Val Asn His Lys Pro Ser Asn Thr Lys
65                  70                  75                  80
Val Asp Lys Arg Val Glu Pro Lys Ser Cys His Thr Cys Pro Pro Cys
                85                  90                  95
Pro Ala Pro Glu Leu Leu Gly Gly Pro
            100                 105

<210> 50
<211> 106
<212> PRT
<213> Artificial Sequence

<220>
<223> DK-S131C

<400> 50
Cys Ser Lys Ser Thr Ser Gly Gly Thr Ala Ala Leu Gly Cys Leu Val
1               5                   10                  15
Lys Asp Tyr Phe Pro Glu Pro Val Thr Val Ser Trp Asn Ser Gly Ala
            20                  25                  30
Leu Thr Ser Gly Val His Thr Phe Pro Ala Val Leu Gln Ser Ser Gly
        35                  40                  45
Leu Tyr Ser Leu Ser Ser Val Val Thr Val Pro Ser Ser Ser Leu Gly
    50                  55                  60
Thr Gln Thr Tyr Ile Cys Asn Val Asn His Lys Pro Ser Asn Thr Lys
65                  70                  75                  80
Val Asp Lys Arg Val Glu Pro Lys Ser Cys Thr His Thr Cys Pro Pro
                85                  90                  95
Cys Pro Ala Pro Glu Leu Leu Gly Gly Pro
            100                 105

<210> 51
<211> 106
<212> PRT
<213> Artificial Sequence

<220>
<223> KT-S131C

<400> 51
Cys Ser Lys Ser Thr Ser Gly Gly Thr Ala Ala Leu Gly Cys Leu Val
1               5                   10                  15
Lys Asp Tyr Phe Pro Glu Pro Val Thr Val Ser Trp Asn Ser Gly Ala
            20                  25                  30
Leu Thr Ser Gly Val His Thr Phe Pro Ala Val Leu Gln Ser Ser Gly
        35                  40                  45
Leu Tyr Ser Leu Ser Ser Val Val Thr Val Pro Ser Ser Ser Leu Gly
    50                  55                  60
Thr Gln Thr Tyr Ile Cys Asn Val Asn His Lys Pro Ser Asn Thr Lys
65                  70                  75                  80
Val Asp Lys Arg Val Glu Pro Lys Ser Cys Asp His Thr Cys Pro Pro
                85                  90                  95
Cys Pro Ala Pro Glu Leu Leu Gly Gly Pro
            100                 105

<210> 52
<211> 106
<212> PRT
<213> Artificial Sequence

<220>
<223> DT-S131C

<400> 52
Cys Ser Lys Ser Thr Ser Gly Gly Thr Ala Ala Leu Gly Cys Leu Val
1               5                   10                  15
Lys Asp Tyr Phe Pro Glu Pro Val Thr Val Ser Trp Asn Ser Gly Ala
            20                  25                  30
Leu Thr Ser Gly Val His Thr Phe Pro Ala Val Leu Gln Ser Ser Gly
        35                  40                  45
Leu Tyr Ser Leu Ser Ser Val Val Thr Val Pro Ser Ser Ser Leu Gly
    50                  55                  60
Thr Gln Thr Tyr Ile Cys Asn Val Asn His Lys Pro Ser Asn Thr Lys
65                  70                  75                  80
Val Asp Lys Arg Val Glu Pro Lys Ser Cys Lys His Thr Cys Pro Pro
                85                  90                  95
Cys Pro Ala Pro Glu Leu Leu Gly Gly Pro
            100                 105

<210> 53
<211> 108
<212> PRT
<213> Artificial Sequence

<220>
<223> S131C-S219C

<400> 53
Cys Ser Lys Ser Thr Ser Gly Gly Thr Ala Ala Leu Gly Cys Leu Val
1               5                   10                  15
Lys Asp Tyr Phe Pro Glu Pro Val Thr Val Ser Trp Asn Ser Gly Ala
            20                  25                  30
Leu Thr Ser Gly Val His Thr Phe Pro Ala Val Leu Gln Ser Ser Gly
        35                  40                  45

Leu Tyr Ser Leu Ser Ser Val Val Thr Val Pro Ser Ser Ser Leu Gly
    50                  55                  60
Thr Gln Thr Tyr Ile Cys Asn Val Asn His Lys Pro Ser Asn Thr Lys
65                  70                  75                  80
Val Asp Lys Arg Val Glu Pro Lys Cys Cys Asp Lys Thr His Thr Cys
                85                  90                  95
Pro Pro Cys Pro Ala Pro Glu Leu Leu Gly Gly Pro
            100                 105

<210> 54
<211> 105
<212> PRT
<213> Artificial Sequence

<220>
<223> DKT-S131C-S219C

<400> 54
Cys Ser Lys Ser Thr Ser Gly Gly Thr Ala Ala Leu Gly Cys Leu Val
1               5                   10                  15
Lys Asp Tyr Phe Pro Glu Pro Val Thr Val Ser Trp Asn Ser Gly Ala
            20                  25                  30
Leu Thr Ser Gly Val His Thr Phe Pro Ala Val Leu Gln Ser Ser Gly
        35                  40                  45
Leu Tyr Ser Leu Ser Ser Val Val Thr Val Pro Ser Ser Ser Leu Gly
    50                  55                  60
Thr Gln Thr Tyr Ile Cys Asn Val Asn His Lys Pro Ser Asn Thr Lys
65                  70                  75                  80
Val Asp Lys Arg Val Glu Pro Lys Cys Cys His Thr Cys Pro Pro Cys
                85                  90                  95
Pro Ala Pro Glu Leu Leu Gly Gly Pro
            100                 105

<210> 55
<211> 106
<212> PRT
<213> Artificial Sequence

<220>
<223> DK-S131C-S219C

<400> 55
Cys Ser Lys Ser Thr Ser Gly Gly Thr Ala Ala Leu Gly Cys Leu Val
1               5                   10                  15
Lys Asp Tyr Phe Pro Glu Pro Val Thr Val Ser Trp Asn Ser Gly Ala
            20                  25                  30
Leu Thr Ser Gly Val His Thr Phe Pro Ala Val Leu Gln Ser Ser Gly
        35                  40                  45
Leu Tyr Ser Leu Ser Ser Val Val Thr Val Pro Ser Ser Ser Leu Gly
    50                  55                  60
Thr Gln Thr Tyr Ile Cys Asn Val Asn His Lys Pro Ser Asn Thr Lys
65                  70                  75                  80
Val Asp Lys Arg Val Glu Pro Lys Cys Cys Thr His Thr Cys Pro Pro
                85                  90                  95
Cys Pro Ala Pro Glu Leu Leu Gly Gly Pro
            100                 105

<210> 56
<211> 106
<212> PRT
<213> Artificial Sequence

<220>

<223> KT-S131C-S219C

<400> 56
Cys Ser Lys Ser Thr Ser Gly Gly Thr Ala Ala Leu Gly Cys Leu Val
1               5                   10                  15
Lys Asp Tyr Phe Pro Glu Pro Val Thr Val Ser Trp Asn Ser Gly Ala
            20                  25                  30
Leu Thr Ser Gly Val His Thr Phe Pro Ala Val Leu Gln Ser Ser Gly
        35                  40                  45
Leu Tyr Ser Leu Ser Ser Val Val Thr Val Pro Ser Ser Ser Leu Gly
    50                  55                  60
Thr Gln Thr Tyr Ile Cys Asn Val Asn His Lys Pro Ser Asn Thr Lys
65                  70                  75                  80
Val Asp Lys Arg Val Glu Pro Lys Cys Cys Asp His Thr Cys Pro Pro
                85                  90                  95
Cys Pro Ala Pro Glu Leu Leu Gly Gly Pro
            100                 105

<210> 57
<211> 106
<212> PRT
<213> Artificial Sequence

<220>
<223> DT-S131C-S219C

<400> 57
Cys Ser Lys Ser Thr Ser Gly Gly Thr Ala Ala Leu Gly Cys Leu Val
1               5                   10                  15
Lys Asp Tyr Phe Pro Glu Pro Val Thr Val Ser Trp Asn Ser Gly Ala
            20                  25                  30
Leu Thr Ser Gly Val His Thr Phe Pro Ala Val Leu Gln Ser Ser Gly
        35                  40                  45
Leu Tyr Ser Leu Ser Ser Val Val Thr Val Pro Ser Ser Ser Leu Gly
    50                  55                  60
Thr Gln Thr Tyr Ile Cys Asn Val Asn His Lys Pro Ser Asn Thr Lys
65                  70                  75                  80
Val Asp Lys Arg Val Glu Pro Lys Cys Cys Lys His Thr Cys Pro Pro
                85                  90                  95
Cys Pro Ala Pro Glu Leu Leu Gly Gly Pro
            100                 105

<210> 58
<211> 108
<212> PRT
<213> Artificial Sequence

<220>
<223> R214T-P217R

<400> 58
Ser Ser Lys Ser Thr Ser Gly Gly Thr Ala Ala Leu Gly Cys Leu Val
1               5                   10                  15
Lys Asp Tyr Phe Pro Glu Pro Val Thr Val Ser Trp Asn Ser Gly Ala
            20                  25                  30
Leu Thr Ser Gly Val His Thr Phe Pro Ala Val Leu Gln Ser Ser Gly
        35                  40                  45
Leu Tyr Ser Leu Ser Ser Val Val Thr Val Pro Ser Ser Ser Leu Gly
    50                  55                  60
Thr Gln Thr Tyr Ile Cys Asn Val Asn His Lys Pro Ser Asn Thr Lys
65                  70                  75                  80
Val Asp Lys Thr Val Glu Arg Lys Ser Cys Asp Lys Thr His Thr Cys
                85                  90                  95

Pro Pro Cys Pro Ala Pro Glu Leu Leu Gly Gly Pro
            100                 105

<210> 59
<211> 108
<212> PRT
<213> Artificial Sequence

<220>
<223> R214T-P217S

<400> 59
Ser Ser Lys Ser Thr Ser Gly Gly Thr Ala Ala Leu Gly Cys Leu Val
1               5                   10                  15
Lys Asp Tyr Phe Pro Glu Pro Val Thr Val Ser Trp Asn Ser Gly Ala
            20                  25                  30
Leu Thr Ser Gly Val His Thr Phe Pro Ala Val Leu Gln Ser Ser Gly
        35                  40                  45
Leu Tyr Ser Leu Ser Ser Val Val Thr Val Pro Ser Ser Ser Leu Gly
    50                  55                  60
Thr Gln Thr Tyr Ile Cys Asn Val Asn His Lys Pro Ser Asn Thr Lys
65                  70                  75                  80
Val Asp Lys Thr Val Glu Ser Lys Ser Cys Asp Lys Thr His Thr Cys
                85                  90                  95
Pro Pro Cys Pro Ala Pro Glu Leu Leu Gly Gly Pro
            100                 105

<210> 60
<211> 38
<212> DNA
<213> Artificial Sequence

<220>
<223> primer

<400> 60
gagcccaaat cttgtgacac tcacacatgc ccaccgtg                              38


<210> 61
<211> 38
<212> DNA
<213> Artificial Sequence

<220>
<223> primer

<400> 61
cacggtgggc atgtgtgagt gtcacaagat ttgggctc                              38


<210> 62
<211> 38
<212> DNA
<213> Artificial Sequence

<220>
<223> primer

<400> 62
cccaaatctt gtgacaaaca cacatgccca ccgtgccc                              38

<210> 63
<211> 38
<212> DNA
<213> Artificial Sequence

<220>
<223> primer

<400> 63
gggcacggtg ggcatgtgtg tttgtcacaa gatttggg                              38


<210> 64
<211> 53
<212> DNA
<213> Artificial Sequence

<220>
<223> primer

<400> 64
caagagagtt gagcccaaat cttgtaaaca cacatgccca ccgtgcccag cac             53


<210> 65
<211> 53
<212> DNA
<213> Artificial Sequence

<220>
<223> primer

<400> 65
gtgctgggca cggtgggcat gtgtgtttac aagatttggg ctcaactctc ttg             53


<210> 66
<211> 54
<212> DNA
<213> Artificial Sequence

<220>
<223> primer

<400> 66
ggacaagaga gttgagccca aatcttgtac tcacacatgc ccaccgtgcc cagc            54


<210> 67
<211> 54
<212> DNA
<213> Artificial Sequence

<220>
<223> primer

<400> 67
gctgggcacg gtgggcatgt gtgagtacaa gatttgggct caactctctt gtcc            54


<210> 68
<211> 42
<212> DNA

<213> Artificial Sequence

<220>
<223> primer

<400> 68
gagttgagcc caaatcttgt gaccacacat gcccaccgtg cc                          42


<210> 69
<211> 42
<212> DNA
<213> Artificial Sequence

<220>
<223> primer

<400> 69
ggcacggtgg gcatgtgtgg tcacaagatt tgggctcaac tc                          42


<210> 70
<211> 38
<212> DNA
<213> Artificial Sequence

<220>
<223> primer

<400> 70
gttgagccca aatcttgtca cacatgccca ccgtgccc                               38


<210> 71
<211> 38
<212> DNA
<213> Artificial Sequence

<220>
<223> primer

<400> 71
gggcacggtg ggcatgtgtg acaagatttg ggctcaac                               38


<210> 72
<211> 45
<212> DNA
<213> Artificial Sequence

<220>
<223> primer

<400> 72
gcccaccgtg cccagcacct cccgtggcgg gaccgtcagt cttcc                       45


<210> 73
<211> 45
<212> DNA
<213> Artificial Sequence

<220>

<223> primer

<400> 73
gaggaagact gacggtcccg ccacgggagg tgctgggcac ggtgg                45


<210> 74
<211> 39
<212> DNA
<213> Artificial Sequence

<220>
<223> primer

<400> 74
ccgtgcccag cacctgaatt cctgggggga ccgtcagtc                       39


<210> 75
<211> 39
<212> DNA
<213> Artificial Sequence

<220>
<223> primer

<400> 75
gactgacggt ccccccagga attcaggtgc tgggcacgg                       39


<210> 76
<211> 60
<212> DNA
<213> Artificial Sequence

<220>
<223> primer

<400> 76
ccaaggtgga caagacagtt gagaggaaat gttgtgtcga atgcccaccg tgcccagcac    60


<210> 77
<211> 60
<212> DNA
<213> Artificial Sequence

<220>
<223> primer

<400> 77
gtgctgggca cggtgggcat tcgacacaac atttcctctc aactgtcttg tccaccttgg    60


<210> 78
<211> 55
<212> DNA
<213> Artificial Sequence

<220>
<223> primer

<400> 78

gtggacaaga gagttgagtc caaatatggt cccccatgcc caccgtgccc agcac          55

<210> 79
<211> 55
<212> DNA
<213> Artificial Sequence

<220>
<223> primer

<400> 79
gtgctgggca cggtgggcat gggggaccat atttggactc aactctcttg tccac          55

<210> 80
<211> 41
<212> DNA
<213> Artificial Sequence

<220>
<223> primer

<400> 80
ggtggacaag agagttgagc gcaaatcttg tgacaaaact c                         41

<210> 81
<211> 41
<212> DNA
<213> Artificial Sequence

<220>
<223> primer

<400> 81
gagttttgtc acaagatttg cgctcaactc tcttgtccac c                         41

<210> 82
<211> 38
<212> DNA
<213> Artificial Sequence

<220>
<223> primer

<400> 82
ggacaagaga gttgagagca aatcttgtga caaaactc                             38

<210> 83
<211> 38
<212> DNA
<213> Artificial Sequence

<220>
<223> primer

<400> 83
gagttttgtc acaagatttg ctctcaactc tcttgtcc                             38

<210> 84
<211> 35
<212> DNA
<213> Artificial Sequence

<220>
<223> primer

<400> 84
gagagttgag cccaaatgtt gtgacaaaac tcaca                                35


<210> 85
<211> 35
<212> DNA
<213> Artificial Sequence

<220>
<223> primer

<400> 85
tgtgagtttt gtcacaacat ttgggctcaa ctctc                                35


<210> 86
<211> 35
<212> DNA
<213> Artificial Sequence

<220>
<223> primer

<400> 86
gagagttgag cccaaatatt gtgacaaaac tcaca                                35


<210> 87
<211> 35
<212> DNA
<213> Artificial Sequence

<220>
<223> primer

<400> 87
tgtgagtttt gtcacaatat ttgggctcaa ctctc                                35


<210> 88
<211> 36
<212> DNA
<213> Artificial Sequence

<220>
<223> primer

<400> 88
agttgagccc aaatctggtg acaaaactca cacatg                               36


<210> 89
<211> 36
<212> DNA

<213> Artificial Sequence

<220>
<223> primer

<400> 89
catgtgtgag ttttgtcacc agatttgggc tcaact                                36


<210> 90
<211> 46
<212> DNA
<213> Artificial Sequence

<220>
<223> primer

<400> 90
ggacaagaga gttgagccca aatgtggtga caaaactcac acatgc                     46


<210> 91
<211> 46
<212> DNA
<213> Artificial Sequence

<220>
<223> primer

<400> 91
gcatgtgtga gttttgtcac cacatttggg ctcaactctc ttgtcc                     46


<210> 92
<211> 36
<212> DNA
<213> Artificial Sequence

<220>
<223> primer

<400> 92
gagagttgag cccaaatgtt gtcacacatg cccacc                                36


<210> 93
<211> 36
<212> DNA
<213> Artificial Sequence

<220>
<223> primer

<400> 93
ggtgggcatg tgtgacaaca tttgggctca actctc                                36


<210> 94
<211> 35
<212> DNA
<213> Artificial Sequence

<220>

<223> primer

<400> 94
gagagttgag cccaaatgtt gtactcacac atgcc                                35


<210> 95
<211> 35
<212> DNA
<213> Artificial Sequence

<220>
<223> primer

<400> 95
ggcatgtgtg agtacaacat ttgggctcaa ctctc                                35


<210> 96
<211> 35
<212> DNA
<213> Artificial Sequence

<220>
<223> primer

<400> 96
gagagttgag cccaaatgtt gtaaacacac atgcc                                35


<210> 97
<211> 35
<212> DNA
<213> Artificial Sequence

<220>
<223> primer

<400> 97
ggcatgtgtg tttacaacat ttgggctcaa ctctc                                35


<210> 98
<211> 35
<212> DNA
<213> Artificial Sequence

<220>
<223> primer

<400> 98
gagagttgag cccaaatgtt gtgaccacac atgcc                                35


<210> 99
<211> 35
<212> DNA
<213> Artificial Sequence

<220>
<223> primer

<400> 99

ggcatgtgtg gtcacaacat ttgggctcaa ctctc 35

<210> 100
<211> 36
<212> DNA
<213> Artificial Sequence

<220>
<223> primer

<400> 100
aatcttgtga caaaactgtc acatgcccac cgtgcc 36

<210> 101
<211> 36
<212> DNA
<213> Artificial Sequence

<220>
<223> primer

<400> 101
ggcacggtgg gcatgtgaca gttttgtcac aagatt 36

<210> 102
<211> 32
<212> DNA
<213> Artificial Sequence

<220>
<223> primer

<400> 102
cttgtgacaa aactcccaca tgcccaccgt gc 32

<210> 103
<211> 32
<212> DNA
<213> Artificial Sequence

<220>
<223> primer

<400> 103
gcacggtggg catgtgggag ttttgtcaca ag 32

<210> 104
<211> 33
<212> DNA
<213> Artificial Sequence

<220>
<223> primer

<400> 104
gtgacaaaac tcacccatgc ccaccgtgcc cag 33

<210> 105
<211> 33
<212> DNA
<213> Artificial Sequence

<220>
<223> primer

<400> 105
ctgggcacgg tgggcatggg tgagttttgt cac                                    33


<210> 106
<211> 35
<212> DNA
<213> Artificial Sequence

<220>
<223> primer

<400> 106
gtgacaaaac tcacgaatgc ccaccgtgcc cagca                                  35


<210> 107
<211> 35
<212> DNA
<213> Artificial Sequence

<220>
<223> primer

<400> 107
tgctgggcac ggtgggcatt cgtgagtttt gtcac                                  35


<210> 108
<211> 43
<212> DNA
<213> Artificial Sequence

<220>
<223> primer

<400> 108
ccaaatcttg tgacaaaact gtcgaatgcc caccgtgccc agc                         43


<210> 109
<211> 43
<212> DNA
<213> Artificial Sequence

<220>
<223> primer

<400> 109
gctgggcacg gtgggcattc gacagttttg tcacaagatt tgg                         43


<210> 110
<211> 43
<212> DNA

<213> Artificial Sequence

<220>
<223> primer

<400> 110
ccaaatcttg tgacaaaact cccccatgcc caccgtgccc agc                43


<210> 111
<211> 43
<212> DNA
<213> Artificial Sequence

<220>
<223> primer

<400> 111
gctgggcacg gtgggcatgg gggagttttg tcacaagatt tgg                43


<210> 112
<211> 57
<212> DNA
<213> Artificial Sequence

<220>
<223> primer

<400> 112
cttccccctg gcaccctgct ccaggagcac ctctgagagc acagcggccc tgggctg        57


<210> 113
<211> 57
<212> DNA
<213> Artificial Sequence

<220>
<223> primer

<400> 113
cagcccaggg ccgctgtgct ctcagaggtg ctcctggagc agggtgccag ggggaag        57


<210> 114
<211> 48
<212> DNA
<213> Artificial Sequence

<220>
<223> primer

<400> 114
gggcacccag acctacacct gcaacgtgga tcacaagccc agcaacac            48


<210> 115
<211> 48
<212> DNA
<213> Artificial Sequence

<220>

<223> primer

<400> 115
gtgttgctgg gcttgtgatc cacgttgcag gtgtaggtct gggtgccc                48


<210> 116
<211> 45
<212> DNA
<213> Artificial Sequence

<220>
<223> primer

<400> 116
cttccccctg gcaccctcct ccaggagcac ctctgggggc acagc                    45


<210> 117
<211> 45
<212> DNA
<213> Artificial Sequence

<220>
<223> primer

<400> 117
gctgtgcccc cagaggtgct cctggaggag ggtgccaggg ggaag                   45


<210> 118
<211> 44
<212> DNA
<213> Artificial Sequence

<220>
<223> primer

<400> 118
cggtcttccc cctggcaccc tgctccaaga gcacctctgg gggc                    44


<210> 119
<211> 44
<212> DNA
<213> Artificial Sequence

<220>
<223> primer

<400> 119
gcccccagag gtgctcttgg agcagggtgc cagggggaag accg                    44


<210> 120
<211> 34
<212> DNA
<213> Artificial Sequence

<220>
<223> primer

<400> 120

cccctggcac cctgctccag gagcacctct gggg                                34

<210> 121
<211> 34
<212> DNA
<213> Artificial Sequence

<220>
<223> primer

<400> 121
ccccagaggt gctcctggag cagggtgcca gggg                                34

<210> 122
<211> 52
<212> DNA
<213> Artificial Sequence

<220>
<223> primer

<400> 122
gcaccctcct ccaagagcac ctctgagagc acagcggccc tgggctgcct gg            52

<210> 123
<211> 52
<212> DNA
<213> Artificial Sequence

<220>
<223> primer

<400> 123
ccaggcagcc cagggccgct gtgctctcag aggtgctctt ggaggagggt gc            52

<210> 124
<211> 37
<212> DNA
<213> Artificial Sequence

<220>
<223> primer

<400> 124
ggtgaccgtg ccctccagca acttgggcac ccagacc                             37

<210> 125
<211> 37
<212> DNA
<213> Artificial Sequence

<220>
<223> primer

<400> 125
ggtctgggtg cccaagttgc tggagggcac ggtcacc                             37

<210> 126
<211> 40
<212> DNA
<213> Artificial Sequence

<220>
<223> primer

<400> 126
cgtgccctcc agcagctttg gcacccagac ctacatctgc                40


<210> 127
<211> 40
<212> DNA
<213> Artificial Sequence

<220>
<223> primer

<400> 127
gcagatgtag gtctgggtgc caaagctgct ggagggcacg                40


<210> 128
<211> 46
<212> DNA
<213> Artificial Sequence

<220>
<223> primer

<400> 128
ggtgaccgtg ccctccagca actttggcac ccagacctac atctgc          46


<210> 129
<211> 46
<212> DNA
<213> Artificial Sequence

<220>
<223> primer

<400> 129
gcagatgtag gtctgggtgc caaagttgct ggagggcacg gtcacc          46


<210> 130
<211> 36
<212> DNA
<213> Artificial Sequence

<220>
<223> primer

<400> 130
ccagcagctt gggcacccaa acctacatct gcaacg                    36


<210> 131
<211> 36
<212> DNA

<213> Artificial Sequence

<220>
<223> primer

<400> 131
cgttgcagat gtaggtttgg gtgcccaagc tgctgg                                36


<210> 132
<211> 51
<212> DNA
<213> Artificial Sequence

<220>
<223> primer

<400> 132
gcaacaccaa ggtggacaag acagttgagc caaaatcttg tgacaaaact c               51


<210> 133
<211> 51
<212> DNA
<213> Artificial Sequence

<220>
<223> primer

<400> 133
gagttttgtc acaagatttt ggctcaactg tcttgtccac cttggtgttg c               51


<210> 134
<211> 44
<212> DNA
<213> Artificial Sequence

<220>
<223> primer

<400> 134
cggtcttccc cctggcaccc tgctccaaga gcacctctgg gggc                       44


<210> 135
<211> 44
<212> DNA
<213> Artificial Sequence

<220>
<223> primer

<400> 135
gcccccagag gtgctcttgg agcagggtgc cagggggaag accg                       44


<210> 136
<211> 44
<212> DNA
<213> Artificial Sequence

<220>

<223> primer

<400> 136
cggtcttccc cctggcaccc tgctccaaga gcacctctgg gggc                        44


<210> 137
<211> 44
<212> DNA
<213> Artificial Sequence

<220>
<223> primer

<400> 137
gcccccagag gtgctcttgg agcagggtgc cagggggaag accg                        44


<210> 138
<211> 44
<212> DNA
<213> Artificial Sequence

<220>
<223> primer

<400> 138
cggtcttccc cctggcaccc tgctccaaga gcacctctgg gggc                        44


<210> 139
<211> 44
<212> DNA
<213> Artificial Sequence

<220>
<223> primer

<400> 139
gcccccagag gtgctcttgg agcagggtgc cagggggaag accg                        44


<210> 140
<211> 44
<212> DNA
<213> Artificial Sequence

<220>
<223> primer

<400> 140
cggtcttccc cctggcaccc tgctccaaga gcacctctgg gggc                        44


<210> 141
<211> 44
<212> DNA
<213> Artificial Sequence

<220>
<223> primer

<400> 141

gcccccagag gtgctcttgg agcagggtgc cagggggaag accg                    44

<210> 142
<211> 44
<212> DNA
<213> Artificial Sequence

<220>
<223> primer

<400> 142
cggtcttccc cctggcaccc tgctccaaga gcacctctgg gggc                    44

<210> 143
<211> 44
<212> DNA
<213> Artificial Sequence

<220>
<223> primer

<400> 143
gcccccagag gtgctcttgg agcagggtgc cagggggaag accg                    44

<210> 144
<211> 44
<212> DNA
<213> Artificial Sequence

<220>
<223> primer

<400> 144
cggtcttccc cctggcaccc tgctccaaga gcacctctgg gggc                    44

<210> 145
<211> 44
<212> DNA
<213> Artificial Sequence

<220>
<223> primer

<400> 145
gcccccagag gtgctcttgg agcagggtgc cagggggaag accg                    44

<210> 146
<211> 35
<212> DNA
<213> Artificial Sequence

<220>
<223> primer

<400> 146
gagagttgag cccaaatgtt gtgacaaaac tcaca                              35

<210> 147
<211> 35
<212> DNA
<213> Artificial Sequence

<220>
<223> primer

<400> 147
tgtgagtttt gtcacaacat ttgggctcaa ctctc                                35


<210> 148
<211> 44
<212> DNA
<213> Artificial Sequence

<220>
<223> primer

<400> 148
cggtcttccc cctggcaccc tgctccaaga gcacctctgg gggc                      44


<210> 149
<211> 44
<212> DNA
<213> Artificial Sequence

<220>
<223> primer

<400> 149
gcccccagag gtgctcttgg agcagggtgc cagggggaag accg                      44


<210> 150
<211> 44
<212> DNA
<213> Artificial Sequence

<220>
<223> primer

<400> 150
cggtcttccc cctggcaccc tgctccaaga gcacctctgg gggc                      44


<210> 151
<211> 44
<212> DNA
<213> Artificial Sequence

<220>
<223> primer

<400> 151
gcccccagag gtgctcttgg agcagggtgc cagggggaag accg                      44


<210> 152
<211> 44
<212> DNA

<213> Artificial Sequence

<220>
<223> primer

<400> 152
cggtcttccc cctggcaccc tgctccaaga gcacctctgg gggc                        44


<210> 153
<211> 44
<212> DNA
<213> Artificial Sequence

<220>
<223> primer

<400> 153
gcccccagag gtgctcttgg agcagggtgc cagggggaag accg                        44


<210> 154
<211> 44
<212> DNA
<213> Artificial Sequence

<220>
<223> primer

<400> 154
cggtcttccc cctggcaccc tgctccaaga gcacctctgg gggc                        44


<210> 155
<211> 44
<212> DNA
<213> Artificial Sequence

<220>
<223> primer

<400> 155
gcccccagag gtgctcttgg agcagggtgc cagggggaag accg                        44


<210> 156
<211> 43
<212> DNA
<213> Artificial Sequence

<220>
<223> primer

<400> 156
ccaaggtgga caagacagtt gagcgcaaat cttgtgacaa aac                         43


<210> 157
<211> 43
<212> DNA
<213> Artificial Sequence

<220>

<223> primer

<400> 157
gttttgtcac aagatttgcg ctcaactgtc ttgtccacct tgg                43


<210> 158
<211> 43
<212> DNA
<213> Artificial Sequence

<220>
<223> primer

<400> 158
ccaaggtgga caagacagtt gagagcaaat cttgtgacaa aac                43


<210> 159
<211> 43
<212> DNA
<213> Artificial Sequence

<220>
<223> primer

<400> 159
gttttgtcac aagatttgct ctcaactgtc ttgtccacct tgg                43


<210> 160
<211> 106
<212> PRT
<213> Artificial Sequence

<220>
<223> constant region of kappa light chain

<400> 160
Thr Val Ala Ala Pro Ser Val Phe Ile Phe Pro Pro Ser Asp Glu Gln
1               5                   10                  15
Leu Lys Ser Gly Thr Ala Ser Val Val Cys Leu Leu Asn Asn Phe Tyr
            20                  25                  30
Pro Arg Glu Ala Lys Val Gln Trp Lys Val Asp Asn Ala Leu Gln Ser
        35                  40                  45
Gly Asn Ser Gln Glu Ser Val Thr Glu Gln Asp Ser Lys Asp Ser Thr
    50                  55                  60
Tyr Ser Leu Ser Ser Thr Leu Thr Leu Ser Lys Ala Asp Tyr Glu Lys
65                  70                  75                  80
His Lys Val Tyr Ala Cys Glu Val Thr His Gln Gly Leu Ser Ser Pro
                85                  90                  95
Val Thr Lys Ser Phe Asn Arg Gly Glu Cys
            100                 105

<210> 161
<211> 451
<212> PRT
<213> Artificial Sequence

<220>
<223> Rituximab HC

<400> 161

Gln Val Gln Leu Gln Gln Pro Gly Ala Glu Leu Val Lys Pro Gly Ala
1               5                   10                  15
Ser Val Lys Met Ser Cys Lys Ala Ser Gly Tyr Thr Phe Thr Ser Tyr
            20                  25                  30
Asn Met His Trp Val Lys Gln Thr Pro Gly Arg Gly Leu Glu Trp Ile
        35                  40                  45
Gly Ala Ile Tyr Pro Gly Asn Gly Asp Thr Ser Tyr Asn Gln Lys Phe
    50                  55                  60
Lys Gly Lys Ala Thr Leu Thr Ala Asp Lys Ser Ser Ser Thr Ala Tyr
65                  70                  75                  80
Met Gln Leu Ser Ser Leu Thr Ser Glu Asp Ser Ala Val Tyr Tyr Cys
                85                  90                  95
Ala Arg Ser Thr Tyr Tyr Gly Gly Asp Trp Tyr Phe Asn Val Trp Gly
            100                 105                 110
Ala Gly Thr Thr Val Thr Val Ser Ala Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Arg Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450

<210> 162
<211> 213
<212> PRT
<213> Artificial Sequence

<220>
<223> Rituximab LC

<400> 162
Gln Ile Val Leu Ser Gln Ser Pro Ala Ile Leu Ser Ala Ser Pro Gly
1               5                   10                  15
Glu Lys Val Thr Met Thr Cys Arg Ala Ser Ser Ser Val Ser Tyr Ile
            20                  25                  30
His Trp Phe Gln Gln Lys Pro Gly Ser Ser Pro Lys Pro Trp Ile Tyr
        35                  40                  45
Ala Thr Ser Asn Leu Ala Ser Gly Val Pro Val Arg Phe Ser Gly Ser
    50                  55                  60
Gly Ser Gly Thr Ser Tyr Ser Leu Thr Ile Ser Arg Val Glu Ala Glu
65                  70                  75                  80
Asp Ala Ala Thr Tyr Tyr Cys Gln Gln Trp Thr Ser Asn Pro Pro Thr
                85                  90                  95
Phe Gly Gly Gly Thr Lys Leu Glu Ile Lys Arg Thr Val Ala Ala Pro
            100                 105                 110
Ser Val Phe Ile Phe Pro Pro Ser Asp Glu Gln Leu Lys Ser Gly Thr
        115                 120                 125
Ala Ser Val Val Cys Leu Leu Asn Asn Phe Tyr Pro Arg Glu Ala Lys
    130                 135                 140
Val Gln Trp Lys Val Asp Asn Ala Leu Gln Ser Gly Asn Ser Gln Glu
145                 150                 155                 160
Ser Val Thr Glu Gln Asp Ser Lys Asp Ser Thr Tyr Ser Leu Ser Ser
                165                 170                 175
Thr Leu Thr Leu Ser Lys Ala Asp Tyr Glu Lys His Lys Val Tyr Ala
            180                 185                 190
Cys Glu Val Thr His Gln Gly Leu Ser Ser Pro Val Thr Lys Ser Phe
        195                 200                 205
Asn Arg Gly Glu Cys
    210

## Description

### Field of the invention

The invention relates to modified antibodies and in particular to therapeutic modified antibodies of the IgG1 subtype and uses thereof. The invention further relates to methods for enhancing the cytotoxic activity of an IgG1 antibody.

### Background of the Invention

Monoclonal antibodies (mAbs) currently play a leading role in the provision of therapeutics by the pharmaceutical industry. There are over 30 antibody drugs already on the market for use in the treatment of a variety of conditions such as cancer, inflammation and cardiovascular disease. The interest in the development of mAbs as therapeutics is motivated by several major advantages. First of all, antibodies inherently possess the potential to act in a very specific manner and can therefore achieve potent on-target effects. Due to their specificity, mAbs are furthermore expected to cause fewer side effects than small molecule drugs. mAbs also have a long half-life in serum and they can be used as shuttles for conjugated drug molecules by which the drug is more efficiently delivered to a specific target cell. Therefore, mAb-based therapeutics offer high potency and efficacy.

mAbs are used extensively for the treatment of cancer. After binding to their target, a variety of mechanisms are thought to play important roles in mediating the observed effects of mAbs, such as i) complement-dependent cytotoxicity (CDC), ii) the recruitment of effector cells leading to antibody-dependent cell-mediated cytotoxicity (ADCC) or phagocytosis (ADCP), and iii) (programmed) cell death (also termed as direct cell death/cytotoxicity or apoptosis). Therapeutic efficacy may be due to a combination of these mechanisms, with their relative importance in clinical therapy possibly dependent on the particular type of diseases. Most therapeutic antibodies that have been licensed and developed as medical agent are of the human IgG1 isotype, which generally induce strong ADCC and CDC when compared with other heavy chain isotypes of the human antibody. However, several issues have been emerging in antibody therapy, such as high cost and insufficient drug action. Advances in the understanding of the interactions of the Abs with their receptors on immune effector cells or with complement protein C1q has provided a rationale for generating engineered Abs with improved effector function such as improved ADCC and CDC to improve effectiveness of the antibody and simultaneously to reduce the amount needed, which potentially results in lower costs. However, little is known on how to improve the direct cytotoxic activity of an antibody.

In recent years, much attention in the cancer field has been directed to CD20 as a promising target for mAbs, particularly in regard to lymphoid malignancies. CD20 is expressed at high levels on most normal and neoplastic B cells, making it an attractive target. Perhaps because of the attention it has received, CD20 has so far been the most effective unconjugated antibody target for the treatment of lymphoid malignancies. B cell depletion therapy using mAbs against CD20, particularly the mAb rituximab, has revolutionized the treatment of these disorders, greatly improving overall survival in patients. The Fc region of rituximab plays a critical role in triggering the cellular events that lead to B-cell elimination *in vivo.* Depending on their ability to redistribute CD20 into lipid rafts and to induce CDC, or to induce cell death and homotypic adhesion, anti-CD20 mAbs can be classified as type I or II anti-CD20 antibodies. Rituximab belongs to the first generation of chimeric anti-CD20 antibodies recognizing a type I epitope.

Several other cell-surface receptors on malignant B cells have been the subject of intense research within the past decade. Among those, CD37 is a promising target in B-cell malignancies because of its high expression in many lymphoid cancers and on chronic lymphocytic leukemia (CLL) cells. Antibodies against various B cell receptors may also be useful in the treatment of non-cancer indications, e.g., for multiple sclerosis, rheumatoid arthritis, and systemic lupus erythematosus. Unfortunately, many patients relapse after mAb therapy and these patients may be resistant to further treatment with the same mAbs. In an effort to improve efficacy and possibly overcome the problem of antibody resistance, strategies have been pursued which aim to enhance mAb effector function, i.e. mutagenesis of the Fc region which can either optimize its affinity for activatory Fc-receptors or reduce its affinity for inhibitory Fc-receptors. Such mutagenesis approaches have been shown to enhance ADCC activity (Lazar et al, PNAS 2006).

Accumulating evidence from in vitro studies, animal tumor models and early clinical trials suggest that a significant portion of the tumoricidal effect of anti-CD20 mAb may be mediated by mechanisms independent of complement or ADCC. Thus, in addition to ADCC and CDC, the induction of apoptotic signalling by therapeutic antibodies may be a particularly important mode of action in therapy, specifically in cancer. The mechanism by which these antibodies induce apoptosis may be somewhat atypical since the process appears to be insensitive to Bcl-2, independent of Fas, and only partially blocked by a caspase inhibitor (Shan et al., Cancer Immunol Immunother, 2000; van der Kolk et al., Leukemia, 2002, p 1735-1744).

It has been shown that antibody modifications are capable of enhancing ADCC or ADCP activity. This has been achieved for amino acid mutations in the Fc region, particularly in the CH2 region, such as S239D/A330L/I332E, F243L and G236A (Lazar et al, PNAS, 2006, Stewart et al, Protein Engineering, Desing and Selection, 2011 and Richards et al, Mol Cancer Ther, 2008). These variants showed a significantly higher affinity for recombinant FcγRIIIa and significantly increased binding to natural killer (NK) cells. However, these documents have not shown any increase in direct cytotoxicity and the modifications are in the CH2 region of the antibody.

Antibody modifications are further capable of enhancing CDC activity. For example, mutation of two residues, K326 and E333, which are located at the extreme ends of the C1q binding epicenter in the CH2 domain of a human IgG, results in a dramatic increase in C1q binding and CDC activity (Idusogie et al, J Immunology, 2001). Another example of CDC enhancement is the engineering of the antibody by IgG1 and IgG3 isotype shuffling by shuffling IgG1 and IgG3 sequences within a heavy chain constant region (see, e.g., Natsume et al., Drug Des Devel Ther, 2009). However, these documents have not shown any increase in direct cytotoxicity and the modifications are in the Fc-portion of the antibody.

Recently, Heider et al (Blood, 2011) described an anti-CD37 IgG1 monoclonal antibody (mAb 37.1) in which 2 amino acids have been replaced in the Fc CH2 region to increase affinity to FcγRIIIa. mAb37.1 exhibits both enhanced ADCC and higher pro-apoptotic activity compared to rituximab, but not compared to the unmodified anti-CD37 IgG1 monoclonal antibody. Also the antibody is not modified in the CH1 or upper hinge region.

However, despite the advances made in the field of therapeutic mAbs, many patients still do not or no longer respond to antibody-mediated treatment. Thus, there is an ongoing need for new antibodies with improved efficacy and engineered or enhanced effector function resulting in enhanced therapeutic properties and/or a reduced effective dose of the antibody. In addition to ADCC and CDC, direct cytotoxic activity is another important mode of action for many monoclonal antibodies. Surprisingly, the inventors were able to identify mutations within the CH1 and upper hinge region of IgG1 antibodies that improve direct cytotoxicity of IgG1 antibodies. The finding that mutations in the sequence of the CH1 and upper hinge region of IgG1 antibodies enhance the direct cytotoxic activity of the antibody allows to tailor the specific effector function of the antibody according to the needs in a specific disease. Thus, IgG1 antibodies carrying those mutations have the potential to be more effective in therapy or prophylaxis and to allow reducing the effective dose. This would not only result in a more cost effective treatment, but also potentially reduced side effects.

### Summary of the Invention

The present invention *inter alia* provides antibodies carrying certain amino acid modifications which display a surprisingly superior direct cytotoxic activity compared to unmodified antibodies.

In one aspect, the invention relates to an antibody having a variant human IgG1 heavy chain constant (CH) region comprising at least a CH1 and/or upper hinge region, wherein the CH1 region has the amino acid sequence of SEQ ID NO: 5 corresponding to residues 118-215 of the Eu numbering and the upper hinge region has the amino acid sequence of SEQ ID NO: 6 corresponding to residues 216-225 of the Eu numbering; and one or more amino acid modification(s) within said CH1 and/or upper hinge region, wherein at least one of said one or more amino acid modification(s) is selected from the group consisting of (i) substitution S131C, (ii) substitutions I199T and N203D, (iii) substitution S219C, (iv) deletion of at least two of D221, K222 and T223, and (v) any combinations thereof, wherein the numbering is according to the Eu numbering. Said antibody has an enhanced direct cytotoxic activity compared to an antibody without said amino acid modification(s). In one embodiment the at least one of said one or more amino acid modification(s) is selected from the group consisting of (i) substitution S131C, (ii) substitutions I199T and N203D, (iv) deletion of at least two of D221, K222 and T223, and (v) any combinations thereof and optionally further comprises the substitution S219Y. The enhanced direct cytotoxic activity is preferably detected by increased Annexin V staining.

The antibody according to the invention may further comprise the substitution (a) R214T, (b) P217R or P217S, (c) H224V or H224 P and/or (d) T225E or T225P. In one embodiment the antibody comprises at least the substitutions R214T, P217R, S219C, H224V and T225E and the deletions of D221, K222 and T223. In another embodiment the antibody comprises at least the substitutions S131C, S219C and the deletion of at least two of D221, K222 and T223. In yet another embodiment the antibody comprises the substitution S219C and the deletions of at least two of D221, K222 and T223. In yet another embodiment the antibody comprising at least the substitutions P217S, S219Y, H224P and T225P and the deletions of D221, K222 and T223; or the substitution S219Y and the deletions of at least two of D221, K222 and T223.

The deletions of at least two of D221, K222 and T223 in any of the antibodies according to the invention may be a deletion of D221 and K222, of D221 and T223, of K222 and T223 or of D221, K222 and T223, preferably the deletion is a deletion of D221, K222 and T223.

The antibody according to the invention may further comprise the substitution K133R, preferably the antibody comprises at least the substitutions S131C and K133R; and/or the substitutions G137E and/or G138S.

The present invention relates to antibodies wherein preferably at least the CH1 or the hinge region comprises no more than 6 amino acid modifications, preferably 4, more preferably 3 or less amino acid modifications. Hence, the invention relates to antibodies wherein the CH1 region comprises 6 or less amino acid modifications, i.e., 6, 5, 4, 3, 2 or 1 amino acid modifications and/or the hinge region comprises 6 or less amino acid modifications, i.e., 6, 5, 4, 3, 2 or 1 amino acid modifications.

The antibody of the invention may be a chimeric antibody, a humanized antibody or a human antibody. Further, the antibody may additionally comprise one or more substitutions in the Fc region to alter other effector functions such as ADCC, ADCP or CDC. Also, the antibody may be a single chain antibody, a bispecific antibody or an antibody fragment, preferably a F(ab')2 fragment.

In a preferred embodiment the antibody of the invention binds to a cancer-related antigen, such as CD20, CD22, CD30, CD33, CD37, CD44v6, CD52, HER2 or HER1.

In another aspect the invention relates to a DNA molecule comprising a sequence encoding the heavy chain of the antibody according to the first aspect.

In yet another aspect the invention relates to a vector comprising the DNA of the second aspect. In one embodiment the vector further comprises a DNA encoding the light chain of the antibody. Alternatively, the DNA encoding the light chain of the antibody may be on a separate vector.

In a fourth aspect the invention relates to a pharmaceutical composition comprising the antibody according to the first aspect and a pharmaceutically acceptable carrier and optionally pharmaceutically acceptable excipients.

The antibody of the first aspect or the pharmaceutical composition of the fourth aspect may further be used in therapy, preferably for use in treating cancer. The antibody of the invention thereby promotes or induces direct cytotoxicity *in vivo,* particularly in treating cancer. Alternatively the antibody may also be used for inducing direct cytotoxicity *in vitro.*

In a fifth aspect the invention relates to a method for enhancing the direct cytotoxicity of an antibody having a human IgG1 heavy chain constant (CH) region comprising at least a CH1 and/or upper hinge region, comprising a step of modifying one or more amino acid(s) of the CH1 and/or upper hinge region. The method results in an antibody having enhanced direct cytotoxic activity compared to an antibody without said amino acid modification(s). The enhanced direct cytotoxic activity is preferably detected by increased Annexin V staining. The method comprises modifying one or more amino acid(s) within said CH1 and/or upper hinge region, wherein at least one of said one or more amino acid modifications(s) is selected from the group consisting of: (i) substitution S131C, (ii) substitutions I199T and N203D, (iii) substitution S219C or S219Y, (iv) deletion of at least two of D221, K222 and T223, and (v) combinations thereof wherein the numbering is according to the Eu numbering. The resulting antibody has an enhanced direct cytotoxic activity compared to an antibody without said amino acid modification(s).

In one embodiment the method comprises modifying one or more amino acid(s) selected from the group consisting of: (i) substitution S131C, (ii) substitutions I199T and N203D, (iii) substitution S219C, (iv) deletion of at least two of D221, K222 and T223, and (v) combinations thereof wherein the numbering is according to the Eu numbering. The method results in an antibody having enhanced direct cytotoxic activity compared to an antibody without said amino acid modification(s). In another embodiment the method comprises modifying the amino acids as disclosed for the antibody according to the first aspect.

In yet another aspect the invention relates to a method for producing an antibody according to the first aspect, comprising (a) transfecting a mammalian host cell with the DNA molecule comprising a sequence encoding the heavy chain of the antibody of the invention or the vector of the invention, (b) optionally transfecting the mammalian host cell with a DNA comprising a sequence encoding an antibody light chain, (c) culturing the host cell under conditions suitable for expressing the antibody, and (d) recovering and purifying the antibody molecule. In one embodiment the DNA molecule comprising a sequence encoding the heavy chain of the antibody according to the first aspect. The DNA comprising a sequence encoding an antibody light chain may be on the same or on different vectors.

### Brief Description of the Figures

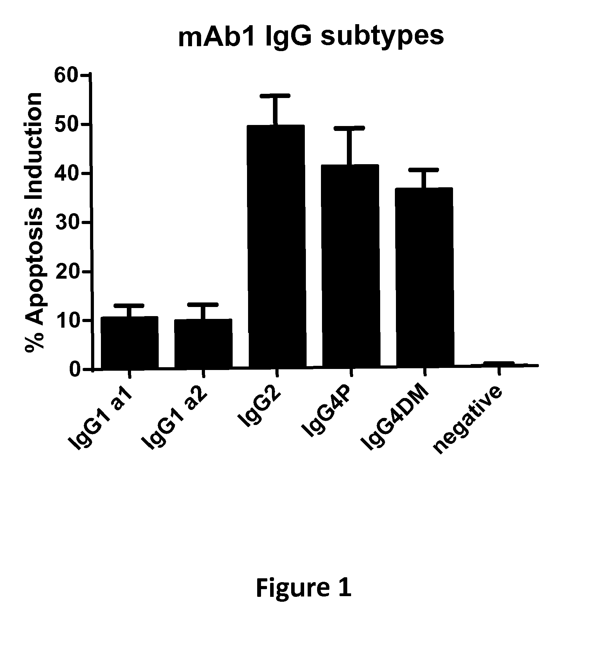

Figure 1: Antibodies were assessed for their apoptosis induction potential. The antibody mAb1 (rituximab) was utilized in the following allotypes: IgG1 a1, IgG1 a2, IgG2, IgG4P and IgG4DM. IgG4P and IgG4DM are IgG4 subtype variants with hinge/CH2 mutations S228P and S228P/L234E, respectively. Shown are the means of the absolute values (percentage of apoptotic cells) ± standard deviations. The numerical data are presented in Table 2.

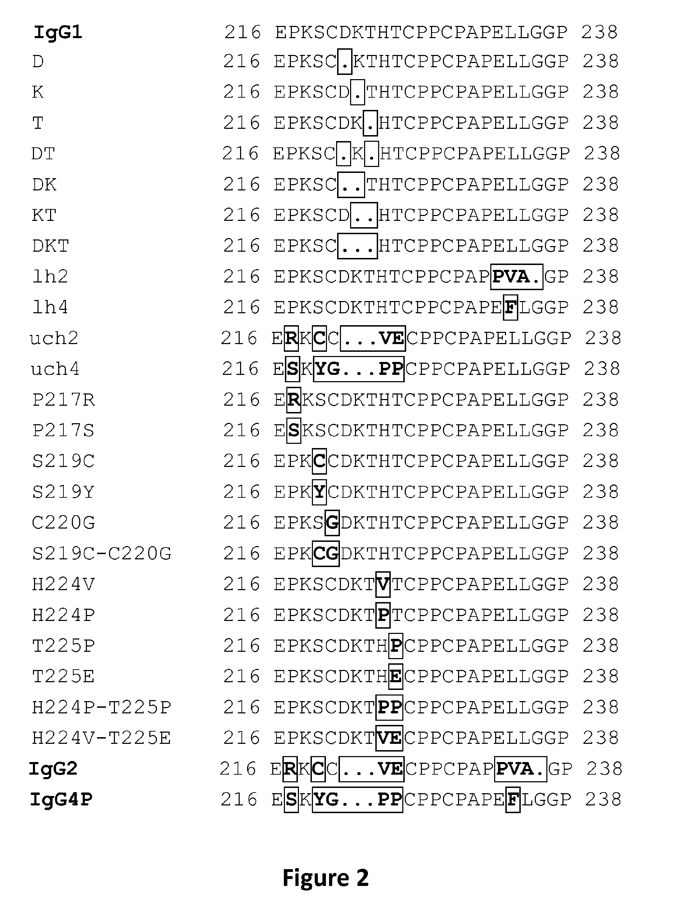

Figure 2: The amino acid alignment illustrates a section of an antibody's hinge and CH2 domains, from positions 216 to 238 according to the Eu numbering of the modified IgG1 antibodies. IgG1, IgG2 and IgG4P are the reference sequences. Substitutions are illustrated in bold, framed letters. Dots indicate amino acid deletions. Mutant uch2 further comprises the mutation R214T (not shown).

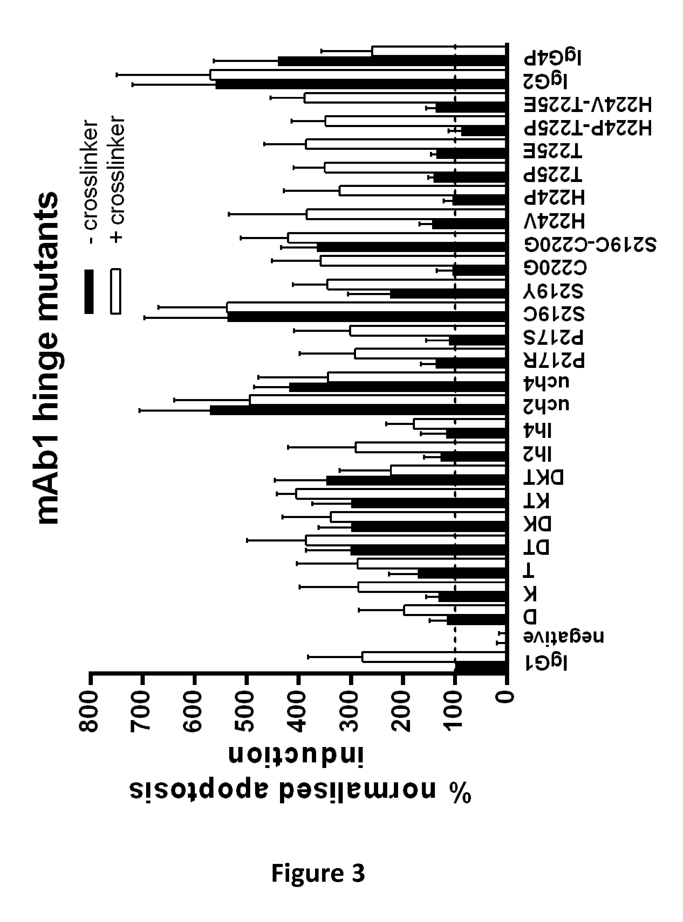

Figure 3: The apoptosis induction potential of the mAb1 hinge mutants depicted in Figure 2 is shown in this figure in relation to IgG1, IgG2 and IgG4P mAb1 controls. An antibody not binding to the target cell was used as negative control (labelled as "negative"). Antibodies were used on their own (black bars) or in conjunction with a cross-linking secondary antibody (white bars). Apoptosis induction was normalised to the IgG1 control in the absence of a cross-linker. The black dashed line indicates 100% normalised apoptosis induction of the mAb1 IgG1 control. Shown are the means ± standard deviations. The numerical data are presented in Table 5.

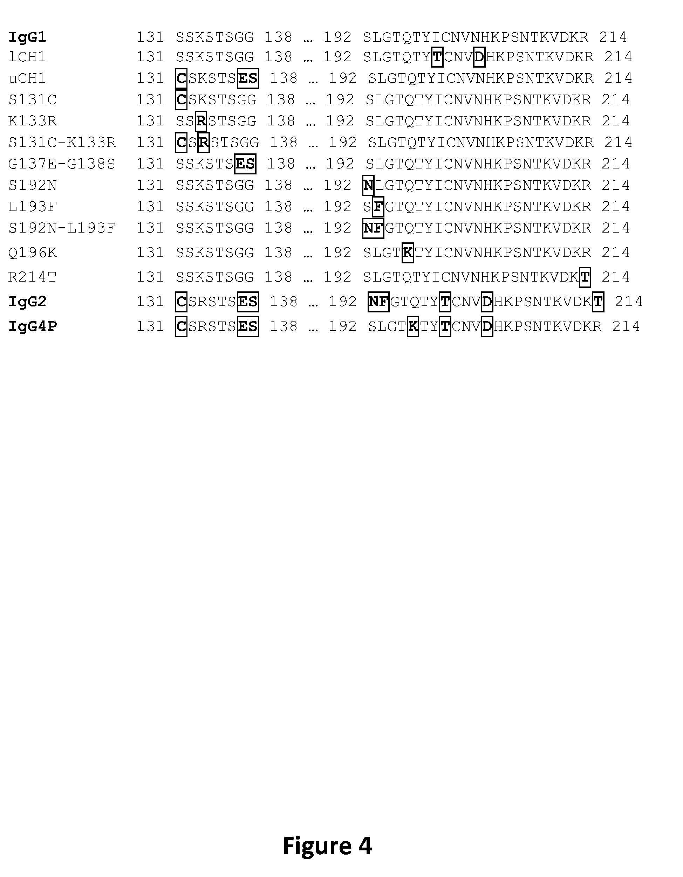

Figure 4: The amino acid alignment illustrates two sections of an antibody's CH1 domain, from positions 131 to 138 and 192 to 214 according to the Eu numbering, respectively, of the modified IgG1 antibodies. IgG1, IgG2 and IgG4P are the reference sequences. Substitutions are illustrated in bold, framed letters.

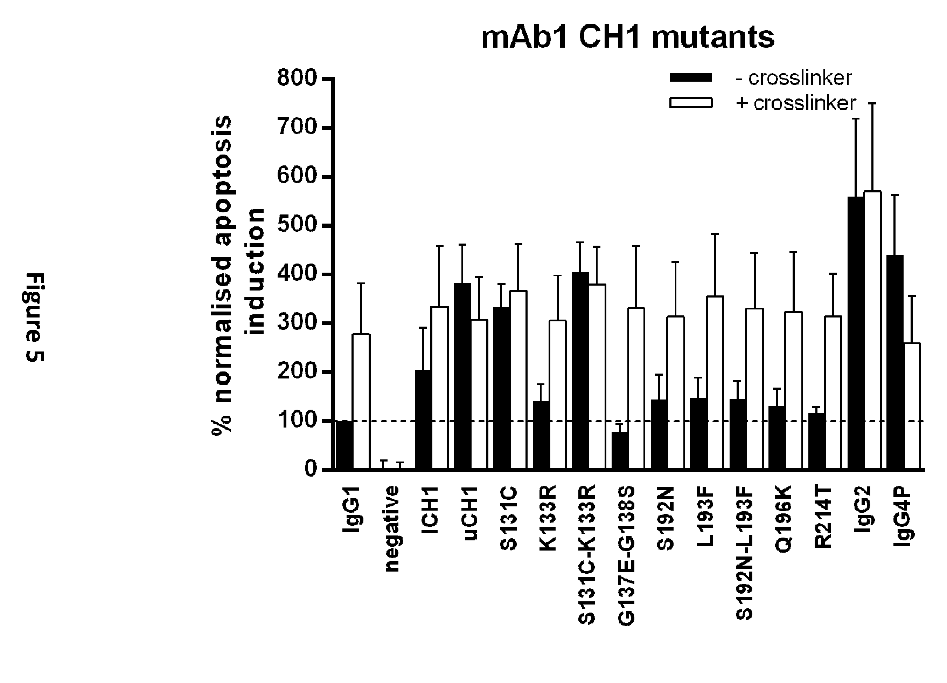

Figure 5: This figure illustrates the apoptosis induction potential of the mAb1 CH1 mutants depicted in
Figure 4 in relation to IgG1, IgG2 and IgG4P mAb1 controls. An antibody not binding to the target cell was used as negative control (labelled as "negative"). Antibodies were used on their own (black bars) or in conjunction with a cross-linking secondary antibody (white bars). Apoptosis induction was normalised to the IgG1 control in the absence of a cross-linker. The black dashed line indicates 100% normalised apoptosis induction of the mAb1 IgG1 control. Shown are the means ± standard deviations. The numerical data are presented in Table 5.

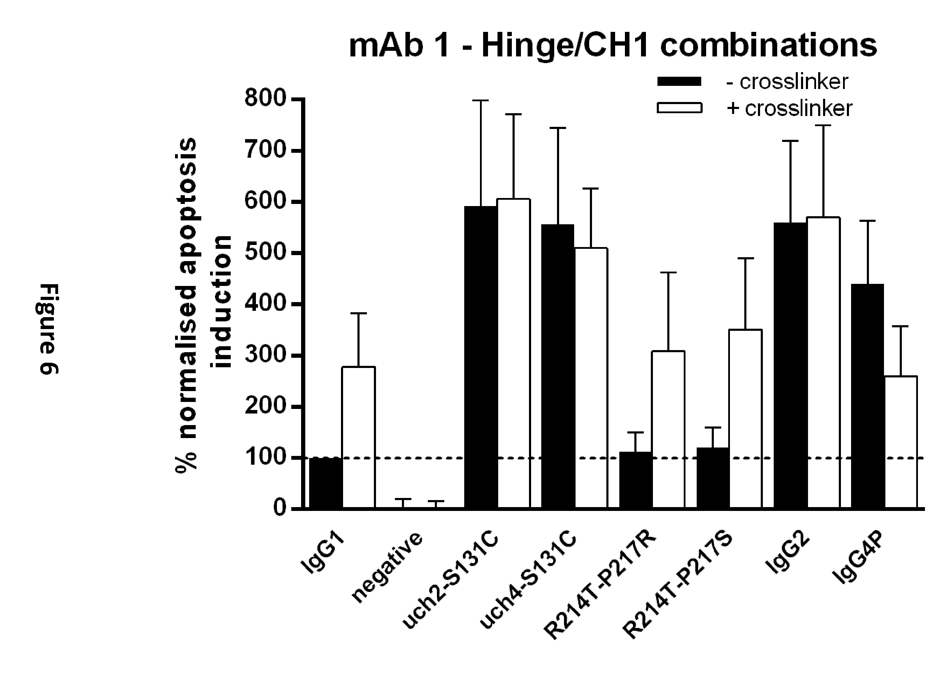

Figure 6: This figure illustrates the apoptosis induction potential of several mAb1 mutants combining hinge and CH1 modifications in comparison to IgG1, IgG2 and IgG4P mAb1 controls. An antibody not binding to the target cell was used as negative control (labelled as "negative"). Antibodies were used on their own (black bars) or in conjunction with a cross-linking secondary antibody (white bars).
Apoptosis induction was normalised to the IgG1 control in the absence of a cross-linker. The black dashed line indicates 100% normalised apoptosis induction of the mAb1 IgG1 control. Shown are the means ± standard deviations. The numerical data are presented in Table 5.

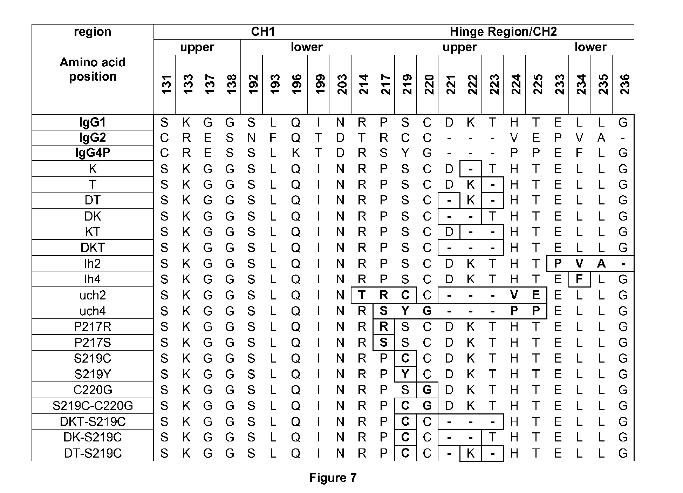

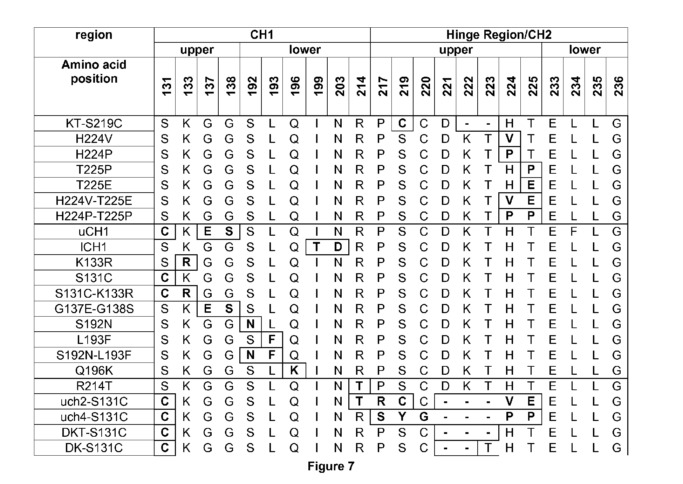

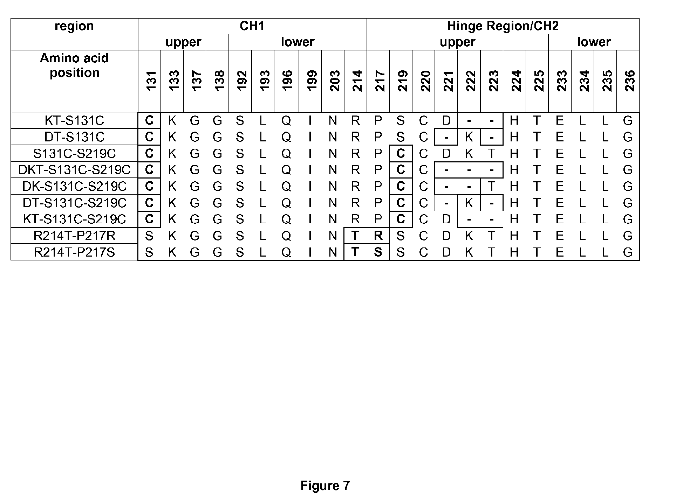

Figure 7: This figure summarizes the modifications of the hinge and/or CH1 mutants by listing the amino acids of the mutants compared to IgG1 at the relevant positions. Modifications are illustrated in bold, framed letters for substitutions and as bold, framed dashes for deletions. The respective amino acids in unmodified IgG1, IgG2 and IgG4P are provided as a reference.

### Detailed Description of the Invention

### Definitions

In order that the present invention may be readily understood, several definitions of terms used in the course of the invention are set forth below.

As used herein, the term "amino acid" refers to one of the 20 naturally occurring amino acids or any non-natural analogues. The term "amino acid modification" refers to an amino acid substitution (mutation), insertion, or deletion in a polypeptide sequence. An amino acid "substitution" is defined as the replacement of an amino acid at a particular position in a parent polypeptide sequence with another amino acid. For example, the substitution S131C refers to a variant polypeptide in which serine at position 131 is replaced with cysteine. An amino acid "insertion" is defined as the addition of an amino acid at a particular position in a parent polypeptide sequence, thereby increasing the length of the polypeptide sequence. An amino acid "deletion" is defined as the removal of an amino acid at a particular position in a parent polypeptide sequence, thereby decreasing the length of the polypeptide sequence. For example, the modification D221Δ refers to a variant polypeptide in which aspartic acid is deleted. The preferred amino acid modifications herein are substitutions and/or deletions. More preferably, the amino acid modification is a substitution and/or deletion to the corresponding IgG2 (SEQ ID NO: 3) or IgG4P (SEQ ID NO: 4) residue (see also Figures 2 and 4). The numbering as used herein is according to the EU numbering of an IgG1 antibody, unless stated otherwise. Residues 118-215 according to the EU numbering correspond to the sequences of SEQ ID NO: 5 and residues 216 to 226 according to the EU numbering correspond to the sequence of SEQ ID NO:6.

The term "protein" is used interchangeably with amino acid residue sequences or polypeptide and refers to polymers of amino acids of any length. These terms also include proteins that are post-translationally modified through reactions that include, but are not limited to, glycosylation, acetylation, phosphorylation or protein processing. Modifications, for example amino acid sequence substitutions, insertions, deletions or fusions to other proteins can be made in the underlying nucleic acid sequence coding for a polypeptide.

As used herein, the term "antibody" refers to a glycoprotein comprising at least two heavy (H) chains and two light (L) chains inter-connected by disulfide bonds, or an antibody fragment (antigen binding portion) thereof. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region (CL). The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host cells or factors, including various cells of the immune system (e.g., effector cells) and the first component (C1q) of the classical complement system.

The term "antibody" is used herein in the broadest sense. Antibodies of the invention include monoclonal antibodies (including full length monoclonal antibodies) and polyclonal antibodies, whole antibodies, chimeric antibodies, humanized antibodies, human antibodies or hybrid antibodies with dual or multiple antigen or epitope specificities, antibody fragments and antibody sub-fragments, e.g., Fab, Fab', F(ab')₂, fragments and the like, including hybrid fragments of any immunoglobulin or any natural, synthetic or genetically engineered protein that acts like an antibody by binding to a specific antigen to form a complex. Antibodies of the invention can also be Fc fusion proteins containing the CH1 (SEQ ID NO: 5) or upper hinge region (SEQ ID NO: 6). Full length "antibodies" or "immunoglobulins" are generally heterotetrameric glycoproteins of about 150 kDa, composed of two identical light and two identical heavy chains. Each light chain is linked to a heavy chain by one covalent disulphide bond, while the number of disulphide linkages varies between the heavy chain of different immunoglobulin isotypes. Each heavy and light chain also has regularly spaced intrachain disulphide bridges. Each heavy chain has an amino terminal variable domain (VH) followed by three carboxy terminal constant domains (CH). Each light chain has a variable N-terminal domain (VL) and a single C-terminal constant domain (CL). The term "antibody" further refers to a type of antibody comprising a plurality of individual antibodies having the same specificity (variable domain) and having the same constant domains (i.e., carrying the same modifications according to the invention).

"Eu numbering" (also known as Eu index) refers to the antibody numbering system (Kabat et al., 1991, Sequences of Proteins of Immunological Interest, 5th Ed., United States Public Health Service, National Institutes of Health, Bethesda), which is based on the sequential numbering of the first human IgG1 sequenced (the Eu antibody; Edelman, et al., 1969, Proc Natl Acad Sci USA 63: 78-85).

The term "CH1 region" as used herein relates to the first (most N-terminal) of three constant domains of the two γ heavy chains of an IgG antibody. In IgG1 it comprises residues 118-215, wherein the numbering is according to the Eu numbering scheme. The IgG1 CH1 region has the amino acid sequence of SEQ ID NO: 5. The term "CH2 region" as used herein relates to the second of three constant domains of the two γ heavy chains of an IgG antibody. Located on the CH2 domain is the carbohydrate moiety of the IgG molecule. The IgG1 CH2 region comprises residues 231-340, wherein the numbering is according to the Eu numbering scheme, and has the amino acid sequence of SEQ ID NO: 8. The term "CH3 domain relates to the third (carboxy terminal portion) of the three constant domains of the two γ heavy chains of an IgG antibody. The terms "region" and "domain" are used interchangeably to refer to the CH1, CH2 and/or CH3 domain or region.

The term "hinge region" refers to the flexible polypeptide comprising the amino acids between the CH1 and CH2 domains of an antibody. The hinge is defined structurally for the purposes of the present invention. The IgG1 "hinge region" as used herein comprises residues 216-230 according to the Eu numbering scheme, and has the amino acid sequence of SEQ ID NO: 7. The term "upper hinge region" as used herein refers to the portion corresponding to the hinge region between residues 216-225 of IgG1 according to the EU numbering, wherein the IgG1 upper hinge region has the amino acid sequence of SEQ ID NO: 6.

The term "IgG1 heavy chain constant region" as used herein refers to a polypeptide comprising CH1, hinge, CH2 and CH3 domain, but lacking the variable domain, having the amino acid sequence of SEQ ID NO: 1 (equivalent to IgG1 a1) or SEQ ID NO: 2 (equivalent to IgG1 a2).

The term "IgG1 light chain constant region" as used herein refers to a polypeptide comprising a CL domain of a light chain either of type k (SEQ ID NO: 160) or type λ, but lacking the variable domain.

The term "Fc region" (fragment crystallizable region) as used herein refers to the C-terminal region of an antibody heavy chain. The Fc region of a full antibody usually comprises two CH2 domains and two CH3 domains. A preferred immunoglobulin CH2 domain within the context of the present invention is a human IgG1 CH2 domain. The CH2 domain of a human IgG1 Fc region usually extends from residues 231-340 according to the EU numbering (SEQ ID NO:8 or residues 114-223 of SEQ ID NOs: 1 or 2) and the CH3 domain of a human IgG1 Fc region usually extends from residues 341-447 according to the EU numbering (residues 224-330 of SEQ ID NO: 1 or SEQ ID NO:2). The CH2 and/or CH3 domain of the antibodies of the present invention are derived from human IgG1 corresponding to residues 114-330 of SEQ ID NOs: 1 or 2. The CH2 and/or CH3 domain may further comprise mutations to enhance effector function such as ADCC, ADCP or CDC known in the art or described herein. Thus, the skilled person understands that the sequence of the CH2 and CH3 domain may vary, but is preferably 60% or more, 70% or more, 80% or more, 90% or more, 95% or more, 98% or more or 99% or more identical to residues 114-330 of the sequence of SEQ ID NOs: 1 or 2.

"Fc-fusion proteins" are defined as proteins which contain or are modified to contain at least the portion of the CH2 domain of the heavy chain immunoglobulin constant region comprising the single N-linked glycosylation site. According to the Eu numbering system, this position is Asn297 in an IgG1, IgG2, IgG3 or IgG4 antibody. This portion is fused (i.e., linked) to the complete or partial sequence of a natural or modified heterologous protein or a composition of such sequences. Fc-fusion proteins can be constructed by genetic engineering approaches by introducing the CH2 domain of the heavy chain immunoglobulin constant region comprising the N-linked glycosylation site into another expression construct comprising, for example, other immunoglobulin domains, enzymatically active protein portions, or effector domains. An Fc-fusion protein may further comprise the hinge region.

"Fab fragments" consist of the variable regions of both chains which are held together by the adjacent constant region of the light chain and the first constant domain (CH1) of the heavy chain. These may be formed by protease digestion, e.g. with papain, from conventional antibodies, but similar Fab fragments may also be produced by genetic engineering. Fab' fragments differ from Fab fragments in that they contain additional residues at the carboxy terminus of the heavy chain CH1 domain including one or more cysteines from the antibody hinge region. Further, antibody fragments include F(ab')2 fragments, which may be prepared by proteolytic cleavage with pepsin or by genetic engineering. The "Fab region" as used herein comprises from the N-terminus to residue 214 of the light chain and from the N-terminus to residue 220 of the heavy chain, wherein the numbering of the C-terminal residues is according to the Eu numbering scheme. Fab may refer to this region in isolation or this region in the context of a full length antibody or antibody fragment. The Fab' and F(ab')2 fragment as used herein comprise from the N-terminus to residue 214 of the light chain and from the N-terminus up to residue 230 of the heavy chain, respectively wherein the numbering of the C-terminal residues is according to the Eu numbering scheme.

The term "monoclonal antibody" or "mAb" as used herein refers to an antibody obtained from a population of substantially homogeneous antibodies based on their amino acid sequence. Monoclonal antibodies are highly specific, being directed against a single antigenic site. Furthermore, in contrast to conventional (polyclonal) antibody preparations which typically include different antibodies directed against different determinants (epitopes) of an antigen, each mAb is directed against a single determinant on the antigen. In addition to their specificity, mAbs are advantageous in that they can be synthesized by cell culture (hybridomas, recombinant cells or the like) uncontaminated by other immunoglobulins. The mAbs herein include for example chimeric, humanized of human antibodies or antibody fragements.

Monoclonal Antibodies in accordance with the present invention may be prepared by methods well known to those skilled in the art. For example, mice may be immunized with an antigen of interest together with adjuvant. Splenocytes are harvested as a pool from the mice that were administered 3 immunizations at 2-week intervals with test bleeds performed on alternate weeks for serum antibody titers. Splenocytes are prepared as 3 aliquots that are either used immediately in fusion experiments or stored in liquid nitrogen for use in future fusions. Fusion experiments are then performed according to the procedure of Stewart & Fuller, J. Immunol. Methods 1989, 123:45-53. Supernatants from wells with growing hybrids are screened by enzyme-linked immunosorbent assay (ELISA) for mAb secretors on 96-well ELISA plates coated with the antigen. ELISA-positive cultures are cloned by limiting dilutions, typically resulting in hybridomas established from single colonies after 2 serial cloning experiments. The ability of an antibody, including an antibody fragment or sub-fragment, to bind to a specific antigen can be determined by binding assays known in the art, for example, using the antigen of interest as the binding partner.

"Chimeric antibodies" are antibodies whose light and/or heavy chain genes have been constructed, typically by genetic engineering, from immunoglobulin variable and constant regions which are identical to, or homologous to, corresponding sequences of different species, such as mouse and human. Alternatively, heavy chain genes derive from a particular antibody class or subclass while the remainder of the chain derives from another antibody class or subclass of the same or a different species. It covers also fragments of such antibodies. For example, a typical therapeutic chimeric antibody is a hybrid protein composed of the variable or antigen-binding domain from a mouse antibody and the constant or effector domain from a human antibody (e.g., ATCC Accession No. CRL 9688 secretes an anti-Tac chimeric antibody), although other mammalian species may be used.

The term "humanized antibodies" according to the present invention refers to specific chimeric antibodies, immunoglobulin chains or fragments thereof (such as Fab, Fab', F(ab')₂, Fv, or other antigen-binding subsequences of antibodies), which contain minimal sequence derived from non-human immunoglobulin. For the most part, humanized antibodies are human immunoglobulins (recipient antibody) in which residues from a complementary-determining region (CDR) of the recipient are replaced by residues from a CDR of a non-human species (donor antibody) such as mouse, rat or rabbit having the desired specificity, affinity and capacity. In some instances, Fv framework residues of the human immunoglobulin need to be replaced by the corresponding non-human residues to increase specificity or affinity. Furthermore, humanized antibodies can comprise residues which are found neither in the recipient antibody nor in the imported CDR or framework sequences. These modifications are made to further refine and maximize antibody performance. In general, the humanized antibody will comprise substantially all of at least one, and typically two, variable domains, in which all or substantially all of the CDR regions correspond to those of a non-human immunoglobulin and all or substantially all of the framework regions are those of a human immunoglobulin consensus sequence. The humanized antibody optimally also will comprise at least a portion of an immunoglobulin constant region, typically that of a human immunoglobulin. Adjustments in framework amino acids might be required to keep antigen binding specificity, affinity and or structure of domain.

Exemplary antibodies within the scope of the present invention include but are not limited to anti-CD2, anti-CD3, anti-CD20, anti-CD22, anti-CD30, anti-CD33, anti-CD37, anti-CD40, anti-CD44, anti-CD44v6, anti-CD49d, anti-CD52, anti-EGFR1 (HER1), anti-EGFR2 (HER2), anti-GD3, anti-IGF, anti-VEGF, anti-TNFalpha, anti-IL2, anti-IL-5R or anti-IgE antibodies. It follows that CD2, CD3, CD20, CD22, CD30, CD33, CD37, CD40, CD44, CD44v6, CD49d, CD52, EGFR1 (HER1), EGFR2 (HER2), GD3, IGF, VEGF, TNFalpha, IL2, IL-5R and IgE are suitable target antigens for the purposes of the present invention.

Antibodies with amino acid modifications according to the present invention are termed "variants" or "mutants" or "modified Antibodies". The term "unmodified antibody" in the context of the present invention refers to an IgG1 antibody or fragment thereof that has no amino acid modifications in the IgG1 upper hinge (SEQ ID NO: 6) and CH1 region (SEQ ID NO: 5). Preferred amino acid modifications in the context of the present invention are substitutions and/or deletions. More preferably, the amino acid modification is a substitution and/or deletion to the corresponding human IgG2 (SEQ ID NO: 3) or IgG4P (SEQ ID NO: 4) residue (see also Figures 2 or 4). Thus, these modifications do not result in epitopes foreign to the patient in need of treatment and hence these modified antibodies are expected to be non-immunogenic.

A "comma" in between two individual amino acid modifications denotes that both modifications are present, e.g., the antibody variant of D221Δ, K222Δ denotes that both D221 and K222 are deleted.

The symbols "/" or "-" are used to distinguish between two or more alternative modifications in a combination of modifications, whereby all modifications are present, e.g., the antibody variant of "S131C / I199T, N203D" means that both the S131C modification and the double modification I199T, N203D are present.

The symbol "Δ" is used to abbreviate an amino acid deletion, e.g., the antibody variant D221Δ denotes that aspartic acid at residue 221 is deleted.

A semicolon denotes the beginning or end of a combination of amino acid modifications, indicating a choice between the different combinations of modifications, e.g., "I199T, N203D / D221Δ, T223Δ; I199T, N203D / K222Δ, T223Δ" corresponds to a choice between two different combinations of modifications, i.e. the choice is between "I199T, N203D / D221Δ, T223Δ" or "I199T, N203D / K222Δ, T223Δ". In this particular example case, each of the different combinations of modifications consists therein of two double modifications.

Preferably, the amino acid modifications are substitutions or deletions in the IgG1 upper hinge and/or CH1 domain into the corresponding IgG2 or IgG4P residue. However, the skilled person understands that for the amino acid modifications disclosed herein, conservative amino acid changes and substitutions may be used instead. Thus, in a specific embodiment the invention also relates to conservative amino acid changes or substitutions of the specific amino acid modifications disclosed herein. In this regard, amino acids belonging to one of the following groups of amino acids represent conservative amino acid changes and substitutions: i) non polar/hydrophob Ala, Val, Leu, Ile, Pro, Met, Phe, Trp; ii) polar/hydrophil Gly, Ser, Thr, Cys, Asn, Gin, Tyer; iii) hydrophylic/acidic: Asp, Glu; iv) hydrophylic/basic: Lys, Arg, His.

"Apoptosis" refers to a process of genetically programmed cell death. It is considered to be irreversible once initiated and it is characterized by distinct biochemical and morphological changes in the cell. Apoptosis can be induced by other cells or by antibodies, either indirectly by a receptor-mediated and a death-domain-dependent mechanism, or directly by a death-domain-independent trigger. Both of these pathways lead to caspase activation, loss of cell membrane integrity, DNA degradation, and formation of apoptotic bodies.

The term "apoptotic potential" refers to the likelihood that a cell will enter and undergo the process of apoptosis when contacted with a specific antibody of antibody fragment. The apoptotic potential of a cell can be influenced by various factors such as the receptors it expresses, its contact with other cells, its source of nutrients and growth factors (i.e. external stimuli) and changes in its physical environment. The term "apoptotic potential" as used herein may also include other forms of direct cytotoxicity, such as "non-apoptotic direct cell death" and non-classical apoptosis".

The term "non-apoptotic direct cell death" as used herein refers to a process which is neither fully necrotic nor apoptotic in nature, but rather may exhibit characteristics of both forms of cell death, e.g. programmed or induced cell death combined with non-apoptotic characteristics such as the rupturing of lysosomes within the cytoplasm, or the generation of reactive oxygen species.

The term "non-classical apoptosis" as used herein refers to a process which is apoptotic in nature, but which does not necessarily invoke the full range of classical apoptosis machinery and associated cascades. For example, non-classical apoptosis may circumvent the anti-apoptotic machinery in cells and hence may be more rapid than classical apoptosis. DNA laddering or caspase dependence may be lacking in this process.

The term "direct cytotoxic activity" as used herein refers to a process which encompasses the processes of apoptosis, non-apoptotic direct cell death and non-classical apoptosis as defined herein mediated by antibody or antibody fragment binding to the target cell, preferably without antibody crosslinking. Direct cytotoxic activity may be detected using Annexin V staining as described herein. Direct cytotoxic activity does not require the presence of an effector cell and is therefore distinct from antibody-dependent cytotoxicity (ADCC). Thus, although the same antibody may induce direct cytotoxicity and ADCC *in vivo* or *in vitro,* these effector functions can be distinguished *in vitro* using methods known in the art. "Direct cytotoxic activity" as used herein refers to the effector function of the antibody or antibody fragment itself and does not require delivery of toxic agents to the target cell. Although antibodies may be used in the form of a conjugate, i.e., an antibody molecule that is chemically coupled to a cytotoxic agent, particularly a cytotoxic agent that induces cytotoxicity (e.g., apoptosis or mitotic arrest) for delivering cytotoxic agents to the target cell *in vivo* or *in vitro,* this is not intended by the term as used herein.

The term "enhanced direct cytotoxic activity" as used herein refers to an improvement in direct cytotoxic activity of a modified antibody compared to an unmodified IgG1 antibody as measured by Annexin V staining, preferably without antibody cross-linking.

The term "% normalized apoptosis induction" or "% apoptosis induction" as used herein refers to all forms of direct cytotoxic activity as measured by Annexin V staining, without specifying the exact pathway to undergo direct cell death. The "% normalized apoptosis induction" is the calculated sum of Annexin V (e.g., Annexin V-FITC) positive and Annexin V/PI double positive cells with the background apoptosis (untreated cells) substracted from all samples given as % apoptosis induction ± SD relative to the unmodified non-crosslinked IgG1 antibody. The "% apoptosis induction" is the calculated sum of Annexin V (e.g., Annexin V-FITC) positive and Annexin V/PI double positive cells with the background apoptosis (untreated cells) substracted from all samples given as % apoptosis induction ± SD.

The term "antibody-dependent cell-mediated cytotoxicity" or "ADCC", as used herein refers to the cell-mediated reaction wherein non-specific cytotoxic cells (effector cells) such as NK cells that express one or more effector ligands (Fc receptors) recognize bound antibody on a target cell and subsequently cause lysis of the target cell. The term "antibody dependent cell-mediated phagocytosis" or "ADCP" as used herein refers to the cell-mediated reaction wherein non-specific cytotoxic cells that express one or more effector ligands recognize bound antibody on a target cell and subsequently cause phagocytosis of the target cell. It has been shown that antibody modifications are capable of enhancing ADCC or ADCP activity. This has been achieved for amino acid mutations in the Fc region, particularly in the CH2 region such as S239D/A330L/I332E, G236A/S239D/I332E, S239D/I332E, G236A/I332E, I332E, F243L or G236A (Lazar et al, PNAS, 2006, Stewart et al, Protein Engineering, Design and Selection, 2011, Richards et al, Mol Cancer Ther, 2008 and WO 2009/019312). These variants showed a significantly higher affinity for recombinant FcγRIIIa and significantly increased binding to natural killer (NK) cells, resulting in enhanced ADCC.

The term "complement-dependent cytotoxicity" or "CDC" as used herein refers to a process of cell killing that is mediated by the complement system. Antibodies may induce complement activation after binding to their target antigen on cells. Initiation of complement activation involves the binding of complement factor C1q to specific motifs within the CH2 domain of antibodies, triggering activation of the complement cascade. It has been shown that antibody modifications are capable of enhancing CDC activity. For example, mutation of two residues, K326 and E333, which are located at the extreme ends of the C1q binding epicenter in the CH2 domain of a human IgG, results in a dramatic increase in C1q binding and CDC activity (Idusogie et al, J Immunology, 2001). Another example of CDC enhancement is the engineering of the heavy chain by shuffling IgG1 and IgG3 sequences within a heavy chain constant region (see, e.g., Natsume et al., Drug Des Devel Ther, 2009).

The term "effector function" as used herein refers to a biochemical event that results from the interaction of an antibody with an effector ligand. Effector functions include but are not limited to ADCC, ADCP, CDC, the release of reactive oxygen species (oxidative burst) and the release of inflammatory mediators.

An "effector cell" as used herein is a cell of the immune system that expresses one or more Fc receptors and mediates one or more effector functions. Effector cells include but are not limited to monocytes, macrophages, neutrophils, dendritic cells, eosinophils, mast cells, platelets, B cells, large granular lymphocytes, Langerhans cells, natural killer (NK) cells, and (CD8+) T cells, and may be from any organism including, but not limited to humans, mice, rats, rabbits, and monkeys.

The term "receptor crosslinking" as used herein refers to the process of bringing two or more cell surface receptors into close proximity with each other or linking them together, thus changing their distribution and/or density on the cell surface. Receptor crosslinking usually has functional consequences, e.g. it stimulates internalization or intracellular signalling. The term "receptor dimerization" refers to the bringing into contact of two cell surface receptors to form a dimer. This may also have functional consequences.

A "cross-linking secondary antibody" as used in accordance with the present invention is a secondary antibody reacting with the respective primary antibody. For example, a primary human IgG1 antibody binding to the target cell may be cross-linked with an anti-human IgG1 antibody of a different species (i.e., non-human)

A "death domain" or "DD" as used herein is a protein interaction module composed of a bundle of six alpha-helices. Some DD-containing proteins are involved in the regulation of apoptosis and inflammation through their activation of caspases and NF-kappaB, which typically involves interactions with TNF cytokine receptors.

The terms "polynucleotide", "oligonucleotide" and "nucleic acid" are used interchangeably throughout and include DNA molecules (e.g., cDNA or genomic DNA), RNA molecules (e.g., mRNA), analogs of the DNA or RNA generated using nucleotide analogs (e.g., peptide nucleic acids and non-naturally occurring nucleotide analogs), and hybrids thereof. The nucleic acid molecule can be single-stranded or double-stranded. In one embodiment, the nucleic acid molecules of the invention comprise a contiguous open reading frame encoding an antibody, or a fragment, derivative, mutein, or variant thereof, of the invention.

A "vector" is a nucleic acid that can be used to introduce another nucleic acid (or "construct") linked to it into a cell. One type of vector is a "plasmid", which refers to a linear or circular double stranded DNA molecule into which additional nucleic acid segments can be ligated. Another type of vector is a viral vector (e.g., replication defective retroviruses, adenoviruses and adeno-associated viruses), wherein additional DNA segments can be introduced into the viral genome. Certain vectors are capable of autonomous replication in a host cell into which they are introduced (e.g., bacterial vectors comprising a bacterial origin of replication and episomal mammalian vectors). Other vectors (e.g., non-episomal mammalian vectors) integrate into the genome of a host cell upon introduction into the host cell and culturing under selective pressure, and thereby are replicated along with the host genome. A vector can be used to direct the expression of a chosen polynucleotide in a cell.

A "host cell" is a cell that can be used to express a nucleic acid, e.g., a nucleic acid or DNA of the invention and the respective protein. A host cell can be a prokaryote, for example, E. coli, or it can be a eukaryote, for example, a single-cell eukaryote (e.g., a yeast or other fungus), a plant cell (e.g., a tobacco or tomato plant cell), an animal cell (e.g., a human cell, a monkey cell, a hamster cell, a rat cell, a mouse cell, or an insect cell) or a hybridoma. Exemplary host cells include Chinese hamster ovary (CHO) cell lines or their derivatives. Typically, a host cell is a cultured cell that can be transformed or transfected with a polypeptide-encoding nucleic acid, which can then be expressed in the host cell. It is understood that the term host cell refers not only to the particular subject cell but to the progeny or potential progeny of such a cell. Because certain modifications may occur in succeeding generations due to, e.g., mutation or environmental influence, such progeny may not, in fact, be identical to the parent cell, but are still included within the scope of the term as used herein.

The term "composition" means a mixture of substances. The term "pharmaceutical composition" means a mixture of substances including the therapeutically active substance for pharmaceutical use. The compositions/pharmaceutical compositions of the present invention may be used for therapy, in particular to treat cancer or other abnormal proliferative diseases.

The terms "of the invention", "in accordance with the invention", or "according to the invention" as used herein are intended to refer to all aspects and embodiments of the invention described and/or claimed herein.

As used herein, the term "comprising" is to be construed as encompassing both "including" and "consisting of", both meanings being specifically intended, and hence individually disclosed, embodiments in accordance with the present invention.

### Antibody variants

The present invention is based on the finding that when an antibody is formatted as IgG2 or IgG4 subtype instead of the more commonly used IgG1, the antibody's direct cytotoxicity induction potential dramatically increases. IgG subtypes differ mainly in their constant region, including the CH1 and hinge domains. Through a selection of novel hybrid antibody formats it has been determined that IgG1 antibodies carrying certain amino acid modifications corresponding to their IgG2 or IgG4 subtype counterparts display a superior direct cytotoxic activity compared to unmodified antibodies. In particular, specific amino acid modifications in either the CH1 region or the upper hinge region of an IgG1 antibody, or in both regions, result in superior direct cytotoxic activity. Thus, antibodies according to the invention are likely to have improved therapeutic potential. The antibody formats disclosed in the present invention are likely to be very valuable for the optimal design of a wide range of antibodies and other proteins comprising the IgG CH1 or upper hinge region intended to be applied in therapy. The terms "antibody variant" and "modified antibody" are used interchangeably herein.

It is an object of the present invention to provide antibody variants. The antibody of the invention has a variant human IgG1 heavy chain constant (CH) region comprising at least a CH1 and/or an upper hinge region, wherein the CH1 region has the amino acid sequence of SEQ ID NO: 5 corresponding to residues 118-215 of the Eu numbering and the upper hinge region has the amino acid sequence of SEQ ID NO: 6 corresponding to residues 216-225 of the Eu numbering; and one or more amino acid modification(s) within said CH1 and/or upper hinge region, wherein at least one of said one or more amino acid modification(s) is selected from the group consisting of (i) substitution S131C, (ii) substitutions I199T and N203D, (iii) substitution S219C, (iv) deletion of at least two of D221, K222 and T223, and (v) any combinations thereof, wherein the numbering is according to the Eu numbering. The antibody of the invention has an enhanced direct cytotoxic activity compared to an antibody without said amino acid modification(s). The enhanced direct cytotoxic activity can be detected by increased Annexin V staining.

For examples the antibody of the invention may comprise one of the above alternative modifications (i)-(iv): S131C; I199T, N203D; S219C or at least two of D221Δ, K222Δ, T223Δ, such as D221Δ, K222Δ, T223Δ; D221Δ, K222Δ; D221Δ, T223Δ or K222Δ, T223Δ.

Alternatively, the antibody of the invention may comprise two of the above alternative modifications (i)-(iv): S131C/I199T, N203D; S131C/S219C; S131C/at least two of D221Δ, K222Δ, T223Δ; S131C/D221Δ, K222Δ, T223Δ; S131C/D221Δ, K222Δ; S131C/D221Δ, T223Δ; S131C/K222Δ, T223Δ; I199T, N203D/S219C; I199T, N203D/at least two of D221Δ, K222Δ, T223Δ; I199T, N203D/D221Δ, K222Δ, T223Δ; I199T, N203D/D221Δ, K222Δ; I199T, N203D/D221Δ,T223Δ; I199T, N203D/K222Δ, T223Δ; S219C/D221Δ, K222Δ, T223Δ; S219C/D221Δ, K222Δ; S219C/D221Δ, T223Δ; or S219C/K222Δ, T223Δ.

Alternatively, the antibody may comprise three of the above alternative modifications (i)-(iv): S131C/I199T, N203D/S219C; S131C/S219C/at least two of D221Δ, K222Δ, T223Δ; S131C/S219C/D221Δ, K222Δ, T223Δ; S131C/S219C/D221Δ, K222Δ; S131C/S219C/D221Δ, T223Δ; S131C/S219C/K222Δ, T223Δ; I199T, N203D/S219C/at least two of D221Δ, K222Δ, T223Δ; I199T, N203D/S219C/D221Δ, K222Δ, T223Δ; I199T, N203D/S219C/D221Δ, K222Δ; I199T, N203D/S219C/D221Δ, T223Δ; I199T, N203D/S219C/K222Δ, T223Δ; S131C/I199T, N203D/at least two of D221Δ, K222Δ, T223Δ; S131C/I199T, N203D/D221Δ,K222Δ, T223Δ; S131C/I199T, N203D/D221Δ,K222Δ; S131C/I199T, N203D/D221Δ,T223Δ; or S131C/I199T, N203D/K222Δ,T223Δ.

Alternatively, the antibody may comprise all four of the above alternative modifications (i)-(iv): S131C/I199T, N203D/S219C/at least two of D221Δ, K222Δ, T223Δ; S131C/I199T, N203D/S219C/D221Δ, K222Δ, T223Δ; S131C/I199T, N203D/S219C/D221Δ, K222Δ; S131C/I199T, N203D/S219C/D221Δ, T223Δ; or S131C/I199T, N203D/S219C/K222Δ, T223Δ.

In one embodiment the at least one of said one or more amino acid modification is selected from the group consisting of (i) substitution S131C; (ii) substitutions I199T and N203D; (iv) deletion of at least two of D221, K222 and T223, and (v) any combinations thereof. In addition, the antibody may further comprise the substitution S219Y. In a specific embodiment the antibody comprises the substitution S219Y and the deletion of at least two of D221, K222 and T223.

For example the antibody of the invention may comprise the substitution S219Y in combination with one of the above alternatives (i), (ii) or (iv): S131C/S219Y; I199T, N203D/S219Y; S219Y/at least two of D221Δ, K222Δ, T223Δ; S219Y/D221Δ, K222Δ, T223Δ; S219Y/D221Δ, K222Δ; S219Y/D221Δ, T223Δ; or S219Y/K222Δ, T223Δ.

Alternatively, the antibody may comprise the substitution S219Y in combination with two of the above alternatives (i), (ii) or (iv): S131C/I199T, N203D/S219Y; S131C/S219Y/at least two of D221Δ, K222Δ, T223Δ; S131C/S219Y/D221Δ, K222Δ, T223Δ; S131C/S219Y/D221Δ, K222Δ; S131C/S219Y/D221Δ, T223Δ; S131C/S219Y/K222Δ, T223Δ; I199T, N203D/S219Y/at least two of D221Δ, K222Δ, T223Δ; I199T, N203D/S219Y/D221Δ, K222Δ, T223Δ; I199T, N203D/S219Y/D221Δ, K222Δ; I199T, N203D/S219Y/D221Δ, T223Δ; or I199T, N203D/S219Y/K222Δ, T223Δ.

Alternatively, the antibody may comprise the substitution S219Y in combination with three of the above alternatives (i), (ii) or (iv): S131C/I199T, N203D/ S219Y/at least two of D221Δ, K222Δ, T223Δ; S131C/I199T, N203D/ S219Y/D221Δ, K222Δ, T223Δ; S131C/I199T, N203D/ S219Y/D221Δ, K222Δ; S131C/I199T, N203D/ S219Y/D221Δ, T223Δ; or S131C/I199T, N203D/ S219Y/K222Δ, T223Δ.

The deletion of at least two of D221, K222 and T223 in the antibody of the invention may be a deletion of D221 and K222, D221 and T223 or K222 and T223, preferably D221, K222 and T223 are deleted in the antibody of the invention. More preferably the antibody has a deletion of at least two of D221, K222 and T223 in combination with at least one of the substitutions S131C; I199T and N203D; or S219C or in combination with at least one of the substitutions S131C; I199T and N203D; or S219Y.

Alternatively, the antibody comprises the substitution S131C, preferably in combination with at least one of the following modifications, a deletion of at least two of D221, K222 and T223; substitutions I199T and N203; or substitution S219C or in combination with at least one of the following modifications, a deletion of at least two of D221, K222 and T223; substitutions I199T and N203; or substitution S219Y. In a preferred embodiment the antibody comprises the substitution S131C and a deletion of at least two of D221, K222 and T223 and optionally further substitution S219C.

Alternatively, the antibody comprises the substitution S219C, preferably in combination with at least one of the following modifications, a deletion of at least two of D221, K222 and T223; substitutions I199T and N203; or substitution S131C. In a specific embodiment the antibody comprises the substitution S219C and the deletion of at least two of D221, K222 and T223.

In addition the antibody of the invention may further comprise substitutions R214T; P217R or P217S; H224V or H224 P; and/or T225E or T225P.

For example, without being limited thereto, the antibody of the invention may comprise the substitution P217R in combination with at least one of the above alternatives (i) to (iv): R217R/S219C/ at least two of D221Δ, K222Δ, T223Δ; R217R/S219C/D221Δ, K222Δ, T223Δ; R217R/S219C/D221Δ, K222Δ; R217R/S219C/D221Δ, T223Δ; R217R/S219C/K222Δ, T223Δ; R214T/R217R/S219C/ at least two of D221Δ, K222Δ, T223Δ; R217R/S219C/ at least two of D221Δ, K222Δ, T223Δ/H224V/T225E; R214T/R217R/S219C/ at least two of D221Δ, K222Δ, T223Δ/H224V/T225E; R217R/S219C/ at least two of D221Δ, K222Δ, T223Δ/H224P/T225P; R217R/S219Y/at least two of D221Δ, K222Δ, T223Δ; R217R/S219Y/D221Δ, K222Δ, T223Δ; R217R/S219Y/D221Δ, K222Δ; R217R/S219Y/D221Δ, T223Δ; R217R/S219Y/K222Δ, T223Δ; R214T/R217R/S219Y/ at least two of D221Δ, K222Δ, T223Δ; R217R/S219Y/ at least two of D221Δ, K222Δ, T223Δ/H224V/T225E; R214T/R217R/S219Y/ at least two of D221Δ, K222Δ, T223Δ/H224V/T225E; R217R/S219Y/ at least two of D221Δ, K222Δ, T223Δ/H224P/T225P; or R217R/S219Y/C220G/ at least two of D221Δ, K222Δ, T223Δ/H224P/T225P.

The antibody of the invention may further comprise, without being limited thereto the substitution P217S in combination with at least one of the above alternatives (i) to (iv): P217S/S219C/ at least two of D221Δ, K222Δ, T223Δ; P217S/S219C/D221Δ, K222Δ, T223Δ; P217S/S219C/D221Δ, K222Δ; P217S/S219C/D221Δ, T223Δ; P217S/S219C/K222Δ, T223Δ; P217S/S219C/ at least two of D221Δ, K222Δ, T223Δ; R214T/R217S/S219C/ at least two of D221Δ, K222Δ, T223Δ; R217S/S219C/ at least two of D221Δ, K222Δ, T223Δ/H224V/T225E; R214T/R217S/S219C/ at least two of D221Δ, K222Δ, T223Δ/H224V/T225E; R217S/S219C/ at least two of D221Δ, K222Δ, T223Δ/H224P/T225P; S131C/P217S/S219C/ at least two of D221Δ, K222Δ, T223Δ; S131C/R214T/R217S/S219C/ at least two of D221Δ, K222Δ, T223Δ; S131C/R217S/S219C/ at least two of D221Δ, K222Δ, T223Δ/H224V/T225E; S131C/R214T/R217S/S219C/ at least two of D221Δ, K222Δ, T223Δ/H224V/T225E; S131C/R217S/S219C/ at least two of D221Δ, K222Δ, T223Δ/H224P/T225P; P217S/S219Y/at least two of D221Δ, K222Δ, T223Δ; P217S/S219Y/D221Δ, K222Δ, T223Δ; P217S/S219Y/D221Δ, K222Δ; P217S/S219Y/D221Δ, T223Δ; P217S/S219Y/K222Δ, T223Δ; R214T/R217S/S219Y/ at least two of D221Δ, K222Δ, T223Δ; R217S/S219Y/ at least two of D221Δ, K222Δ, T223Δ/H224V/T225E; R214T/R217S/S219Y/ at least two of D221Δ, K222Δ, T223Δ/H224V/T225E; R217S/S219Y/ at least two of D221Δ, K222Δ, T223Δ/H224P/T225P; R217S/S219Y/C220G/at least two of D221Δ, K222Δ, T223Δ/H224P/T225P; S131C/R214T/R217S/S219Y/ at least two of D221Δ, K222Δ, T223Δ; S131C/R217S/S219Y/ at least two of D221Δ, K222Δ, T223Δ/H224V/T225E; S131C/R214T/R217S/S219Y/ at least two of D221Δ, K222Δ, T223Δ/H224V/T225E; S131C/R217S/S219Y/ at least two of D221Δ, K222Δ, T223Δ/H224P/T225P; or S131C/R217S/S219Y/C220G/at least two of D221Δ, K222Δ, T223Δ/H224P/T225P.

In a specific embodiment the antibody of the invention comprises the substitutions R214T, P217R, S219C, H224V and T225E and the deletion of D221, K222 and T223 (uch2) or the substitutions P217S, S219Y, H224P and T225P and the deletion of D221, K222 and T223 (uch4).

The skilled person will understand that the modifications in the hinge region of the antibody may be combined with any of the modifications in the CH1 domain disclosed herein, preferably, with the substitution S131C.

In addition, the antibody of the invention may further comprise the substitution K133R.

For example, without being limited thereto, the antibody of the invention may comprise the substitution K133R in combination with one of the above alternatives (i) to (iv): S131C/K133R; K133R/I199T, N203D; K133R/S219C; K133R/at least two of D221Δ, K222Δ, T223Δ; K133R/D221Δ, K222Δ, T223Δ; K133R/D221Δ, K222Δ; K133R/D221Δ, T223Δ; or K133R/K222Δ, T223Δ.Preferably, the antibody comprises the substitutions K133R in combination with S131C.

Alternatively, the antibody may comprise the substitution K133R in combination with two of the above alternatives (i) to (iv): S131C/K133R/I199T, N203D; S131C/K133R/S219C; S131C/K133R/at least two of D221Δ, K222Δ, T223Δ; S131C/K133R/D221Δ, K222Δ, T223Δ; S131C/K133R/D221Δ, K222Δ; S131C/K133R/D221Δ, T223Δ; S131C/K133R/K222Δ, T223Δ; K133R/I199T, N203D/S219C; K133R/I199T, N203D/at least two of D221Δ, K222Δ, T223Δ; K133R/I199T, N203D/D221Δ,K222Δ, T223Δ; K133R/I199T, N203D/D221Δ,K222Δ; K133R/I199T, N203D/D221Δ,T223Δ; K133R/I199T, N203D/K222Δ,T223Δ; K133R/S219C/at least two of D221Δ, K222Δ, T223Δ; K133R/S219C/D221Δ, K222Δ, T223Δ; K133R/S219C/D221Δ, K222Δ; K133R/S219C/D221Δ, T223Δ; K133R/S219C/K222Δ, T223Δ; S131C/K133R/S219Y; K133R/I199T, N203D/S219Y; K133R/S219Y/at least two of D221Δ, K222Δ, T223Δ; K133R/S219Y/D221Δ, K222Δ, T223Δ; K133R/S219Y/D221Δ, K222Δ; K133R/S219Y/D221Δ, T223Δ; or K133R/S219Y/K222Δ, T223Δ.

Alternatively, the antibody may comprise the substitution K133R in combination with three of the above alternatives (i) to (iv): S131C/K133R/I199T, N203D/S219C; S131C/K133R/S219C/at least two of D221Δ, K222Δ, T223Δ; S131C/K133R/S219C/D221Δ, K222Δ, T223Δ; S131C/K133R/S219C/D221Δ, K222Δ; S131C/K133R/S219C/D221Δ, T223Δ; S131C/K133R/S219C/K222Δ, T223Δ; K133R/I199T, N203D/S219C/at least two of D221Δ, K222Δ, T223Δ; K133R/I199T, N203D/S219C/D221Δ, K222Δ, T223Δ; K133R/I199T, N203D/S219C/D221Δ, K222Δ; K133R/I199T, N203D/S219C/D221Δ, T223Δ; K133R/I199T, N203D/S219C/K222Δ, T223Δ; S131C/K133R/I199T, N203D/at least two of D221Δ, K222Δ, T223Δ; S131C/K133R/I199T, N203D/D221Δ,K222Δ, T223Δ; S131C/K133R/I199T, N203D/D221Δ,K222Δ; S131C/K133R/I199T, N203D/D221Δ,T223Δ; S131C/K133R/I199T, N203D/K222Δ,T223Δ; S131C/K133R/I199T, N203D/S219Y; S131C/K133R/S219Y/at least two of D221Δ, K222Δ, T223Δ; S131C/K133R/S219Y/D221Δ, K222Δ, T223Δ; S131C/K133R/S219Y/D221Δ, K222Δ; S131C/K133R/S219Y/D221Δ, T223Δ; S131C/K133R/S219Y/K222Δ, T223Δ; K133R/I199T, N203D/S219Y/at least two of D221Δ, K222Δ, T223Δ; K133R/I199T, N203D/S219Y/D221Δ, K222Δ, T223Δ; K133R/I199T, N203D/S219Y/D221Δ, K222Δ; K133R/I199T, N203D/S219Y/D221Δ, T223Δ; or K133R/I199T, N203D/S219Y/K222Δ, T223Δ.

Alternatively, the antibody may comprise the substitution K133R in combination with all four of the above alternatives (i) to (iv): S131C/K133R/I199T, N203D/S219C/at least two of D221Δ, K222Δ, T223Δ; S131C/K133R/I199T, N203D/S219C/D221Δ, K222Δ, T223Δ; S131C/K133R/I199T, N203D/S219C/D221Δ, K222Δ; S131C/K133R/I199T, N203D/S219C/D221Δ, T223Δ; S131C/K133R/I199T, N203D/S219C/K222Δ, T223Δ; S131C/Kl33R/I199T, N203D/S219Y/at least two of D221Δ, K222Δ, T223Δ; S131C/K133R/I199T, N203D/S219Y/D221Δ, K222Δ, T223Δ; S131C/K133R/I199T, N203D/S219Y/D221Δ, K222Δ; S131C/K133R/I199T, N203D/S219Y/D221Δ, T223Δ; or S131C/K133R/I199T, N203D/S219Y/K222Δ, T223Δ.

In addition, the antibody of the invention may further comprise the substitutions G137E and/or G138S. For example, without being limited thereto, the antibody of the invention may comprise the substitution G137E and/or G138S in combination with at least one of the above alternatives (i) to (iv): S131C/G137E; S131C/G138S; S131C/G137E/G138S; S131C/G137E/I199T and N203D; S131C/G138S/I199T and N203D; S131C/G137E/G138S//I199T and N203D; S131C/K133R/G137E; S131C/K133R/G138S; S131C/K133R/G137E/G138S; S131C/K133R/G137E/I199T and N203D; S131C/K133R/G138S/I199T and N203D; S131C/K133R/G137E/G138S//I199T and N203D; S131C/G137E/ at least two of D221Δ, K222Δ, T223Δ; S131C/G138S/ at least two of D221Δ, K222Δ, T223Δ; S131C/G137E/G138S/ at least two of D221Δ, K222Δ, T223Δ; S131C/G137E/I199T and N203D/ at least two of D221Δ, K222Δ, T223Δ; S131C/G138S/I199T and N203D/ at least two of D221Δ, K222Δ, T223Δ; S131C/G137E/G138S//I199T and N203D/ at least two of D221Δ, K222Δ, T223Δ; S131C/K133R/G137E/ at least two of D221Δ, K222Δ, T223Δ; S131C/K133R/G138S/ at least two of D221Δ, K222Δ, T223Δ; S131C/K133R/G137E/G138S/ at least two of D221Δ, K222Δ, T223Δ; S131C/K133R/G137E/I199T and N203D/ at least two of D221Δ, K222Δ, T223Δ; S131C/K133R/G138S/I199T and N203D/ at least two of D221Δ, K222Δ, T223Δ; S131C/K133R/G137E/G138S//I199T and N203D/ at least two of D221Δ, K222Δ, T223Δ; S131C/G137E/S219C/ at least two of D221Δ, K222Δ, T223Δ; S131C/G138S/S219C/ at least two of D221Δ, K222Δ, T223Δ; S131C/G137E/G138S/S219C/ at least two of D221Δ, K222Δ, T223Δ; S131C/G137E/I199T and N203D/S219C/ at least two of D221Δ, K222Δ, T223Δ; S131C/G138S/I199T and N203D/S219C/ at least two of D221Δ, K222Δ, T223Δ; S131C/G137E/G138S//I199T and N203D/S219C/ at least two of D221Δ, K222Δ, T223Δ; S131C/K133R/G137E/S219C/ at least two of D221Δ, K222Δ, T223Δ; S131C/K133R/G138S/S219C/ at least two of D221Δ, K222Δ, T223Δ; S131C/K133R/G137E/G138S/S219C/ at least two of D221Δ, K222Δ, T223Δ; S131C/K133R/G137E/I199T and N203D/S219C/ at least two of D221Δ, K222Δ, T223Δ; S131C/K133R/G138S/I199T and N203D/S219C/ at least two of D221Δ, K222Δ, T223Δ; S131C/K133R/G137E/G138S//I199T and N203D/S219C/ at least two of D221Δ, K222Δ, T223Δ; S131C/G137E/S219Y/ at least two of D221Δ, K222Δ, T223Δ; S131C/G138S/S219Y/ at least two of D221Δ, K222Δ, T223Δ; S131C/G137E/G138S/S219Y/at least two of D221Δ, K222Δ, T223Δ; S131C/G137E/I199T and N203D/S219Y/ at least two of D221Δ, K222Δ, T223Δ; S131C/G138S/I199T and N203D/S219Y/ at least two of D221Δ, K222Δ, T223Δ; S131C/G137E/G138S//I199T and N203D/S219Y/ at least two of D221Δ, K222Δ, T223Δ; S131C/K133R/G137E/S219Y/ at least two of D221Δ, K222Δ, T223Δ; S131C/K133R/G138S/S219Y/ at least two of D221Δ, K222Δ, T223Δ; S131C/K133R/G137E/G138S/S219Y/ at least two of D221Δ, K222Δ, T223Δ; S131C/K133R/G137E/I199T and N203D/S219Y/ at least two of D221Δ, K222Δ, T223Δ; S131C/K133R/G138S/I199T and N203D/S219Y/ at least two of D221Δ, K222Δ, T223Δ; or S131C/K133R/G137E/G138S//I199T and N203D/S219Y/ at least two of D221Δ, K222Δ, T223Δ.

In a specific embodiment the antibody of the invention comprises at least the substitutions S131C, K133R, G137E and G138S and optionally further I199T and N203D. In another specific embodiment the antibody of the invention comprises at least the substitutions S131C, S219C and the deletion of at least two of D221, K222 and T223.

In another specific embodiment, the antibody of the invention comprises the CH1 region of IgG2 or IgG4P having the amino acid sequence of residues 1 to 98 of SEQ ID NO: 3 or SEQ ID NO: 4, respectively, and/or the upper hinge region of IgG2 or IgG4P having the amino acid sequence of residues 99-105 of SEQ ID NO: 3 or SEQ ID NO: 4, respectively. However, this is under the proviso that the antibody of the invention is not an IgG2 or IgG4P antibody or a fragment thereof. Thus the antibody comprises only the CH1 region or the upper hinge region of IgG2 or IgG4P or in the case that both the CH1 region and the upper hinge region are from IgG2 or IgG4P the antibody comprises a CH2 and/or CH3 region of IgG1.

Preferably, the antibody of the invention comprises a CH1 and/or hinge region each with no more than 6 amino acid modifications, preferably 5, more preferably 4, and even more preferably 3 or less amino acid modifications. Hence, the invention relates to antibodies wherein the CH1 region comprises 6 or less amino acid modifications, i.e., 6 or less, 5 or less, 4 or less, 3 or less, 2 or less or 1 amino acid modification(s) and/or the hinge region comprises 6 or less amino acid modifications, i.e., 6 or less, 5 or less, 4 or less, 3 or less, 2 or less or 1 amino acid modification(s). The modifications are usually additive or synergistic and hence an antibody comprising a combination of amino acid modifications of the invention typically shows a stronger direct cytotoxic activity compared to an antibody comprising only one of the amino acid modifications of the invention.

The antibody of the invention may be any antibody comprising the constant heavy chain of human IgG1 comprising at least one of the modifications of the invention, such as in a chimeric antibody, a humanized antibody or a human antibody. The antibody of the invention may further be a fragment of a chimeric antibody, a humanized antibody or a human antibody, as long as it comprises at least the IgG CH1, the IgG upper hinge region or the IgG CH1 and upper hinge region comprising the modifications of the invention. Preferably the antibody comprises the IgG1 CH1 and upper hinge region comprising at least one of the modifications of the invention. However, preferably the antibody also comprises the CH2 region and more preferably the CH2 and CH3 region of IgG1. In some embodiments the antibody is a single chain antibody, a bispecific antibody or an antibody fragment, preferably a F(ab')2 fragment. In a specific embodiment the antibody of the invention may also be an Fc fusion protein, as long as it contains a CH1 and/or upper hinge region.

The antibody of the present invention may bind to any target antigen as long as it is capable of inducing direct cytotoxicity. Preferably the antibody binds to a cell surface antigen. Examples of suitable target antigens are CD2, CD3, CD20, CD22, CD30, CD33, CD37, CD40, CD44, CD44v6, CD49d, CD52, EGFR1 (HER1), EGFR2 (HER2), GD3, IGF, VEGF, TNF, IL2, IL-5R and IgE. Suitable antibodies according to the invention are, without being limited thereto, anti-CD20 (e.g., rituximab, ofatumumab), anti-CD30 (e.g., brentuximab), anti-CD37, anti-CD52 (e.g., alemtuzumab), anti-CD44v6 (e.g., bivatuzumab), anti-EGFR1 (e.g, cetuximab) and anti-EGFR2 (e.g.,trastuzumab, pertuzumab, herceptin) antibodies.

Preferrably, the antibody binds to a cancer-related antigen, more preferably the antibody binds to CD20, CD37, HER2 and CD44v6. More preferably, the antibody variants of the invention are based on anti-CD20 antibody rituximab, anti-HER2 antibody herceptin, anti-CD44v6 antibody bivatuzumab or an anti-CD37 antibody, even more preferably the antibody variants are based on rituximab or an anti-CD37 antibody.

The antibody variants of the invention exhibit a superior direct cytotoxic activity compared to unmodified antibodies as measured by Annexin V staining. Preferably, said direct cytotoxic activity is apoptosis. Typically, the direct cytotoxic activity is induced by receptor crosslinking, receptor dimerization, blocking of receptor dimerization, by signalling involving a death domain or by blocking ligand receptor interactions.

The enhanced direct cytotoxicity of the antibody variants of the invention correspond to an increase in direct cytotoxic activity compared to an unmodified IgG1 antibody of at least 120%, of at least 150%, of at least 200%, preferably of at least 300%, more preferably of at least 400% and most preferably of at least 500%, as measured by Annexin V staining.

The antibody of the invention may further be engineered to provide altered effector functions, such as ADCC, ADCP and CDC. For example, antibodies mutated in the CH2 domain, such as S239D/A330L/I332E, G236A/S239D/I332E, S239D/I332E, G236A/I332E, I332E, F243L and G236A (Lazar et al, PNAS, 2006, Stewart et al, Protein Engineering, Desing and Selection, 2011, Richards et al, Mol Cancer Ther, 2008 and WO 2009/019312) show increased binding to FcγRIII and enhanced ADCC activity. Preferably, the antibodies of the invention further comprise the modification(s) I332E, S239D/I332E or S239D/I332E/G236A. Further, mutations of K326 and E333 result in a dramatic increase in C1q binding and CDC activity. Alternatively the antibody of the invention may be glycoengineered, such as carrying an afucosylated carbohydrate on the CH2 region, which exhibit greatly enhanced ADCC activity through increased binding to FcγRIIIa. The antibody of the invention may also be engineered to provide an enhanced serum half-life of IgG. Mutations identified to increase half-life of IgG antibodies are for example T250Q/M428L or M252Y/S254T/T256E (Hinton et al, J Biol Chem, 2004; Dall'Acqua et al., J Immunol, 2002, Dall'Acqua et al., J Biol Chem, 2006).

### Method of enhancing the direct cytotoxicity of an antibody

A method is provided for enhancing the direct cytotoxicity of an IgG1 antibody having a human IgG1 CH region comprising at least a CH1 and/or an upper hinge region. This method comprises a step of modifying one or more amino acid residue(s) of the CH1 and/or the upper hinge region of said IgG1 antibody. The method may further comprise the steps of providing an IgG1 antibody having a human IgG1 CH region comprising at least a CH1 and/or an upper hinge region prior to the modification step. Further, the method may comprise the steps of harvesting and purifying the modified antibody following the modification step.

In one embodiment, the CH1 region to be modified corresponds to residues 118-215 according to the Eu numbering and the upper hinge region to be modified corresponds to residues 216-225 of the Eu numbering, preferably, wherein the CH1 region to be modified comprises the amino acid sequence of SEQ ID NO: 5 and the upper hinge region to be modified comprises the amino acid sequence of SEQ ID NO: 6.

The modification of one or more amino acid(s) can be a modification to the corresponding residue of IgG2 (having the sequence of SEQ ID NO: 3) or a modification to the corresponding residue of IgG4P (having the sequence of SEQ ID NO:4) (see also Figures 2 and 4). Preferably, the method comprises the step of modifying one or more amino acids(s) within said CH1 and/or upper hinge region, wherein at least one of said one or more amino acid modification(s) is selected from the group consisting of: (i) substitution S131C, (ii) substitutions I199T and N203D, (iii) substitution S219C or S219Y, (iv) deletion of at least two of D221, K222 and T223, and (v) combinations thereof.

Preferably, the method comprises modifying one or more amino acid(s) within said CH1 and/or upper hinge region, wherein at least one of said one or more amino acid modifications is selected from the group consisting of: (i) substitution S131C, (ii) substitutions I199T and N203D, (iii) substitution S219C, (iv) deletion of at least two of D221, K222 and T223, and (v) combinations thereof.

More preferably, the method comprises modifying one or more amino acid(s) within said CH1 and/or upper hinge region, wherein at least one of said one or more amino acid modifications is selected from the group consisting of: (i) substitution S131C, (ii) substitutions I199T and N203D, (iv) deletion of at least two of D221, K222 and T223, and (v) combinations thereof. The method may further comprise substituting S219Y.

The method of the invention comprises modifying all amino acid modifications (substitutions and deletions) and combinations thereof as specified above for the antibodies of the present invention. Thus, the skilled person will understand that all of the modifications in the antibodies of the invention may be used in the method of the invention to enhance the direct cytotoxicity of an IgG1 antibody.

Preferably, the method of the invention comprises modifying no more than 6 amino acids in each of the CH1 and/or hinge region, preferably 5, more preferably 4, and even more preferably 3 or less amino acids. Hence, the method of the invention comprises modifying 6 or less amino acids, i.e., 6 or less, 5 or less, 4 or less, 3 or less, 2 or less or 1 amino acid(s) in the CH1 region and/or modifying 6 or less amino acids, i.e., 6 or less, 5 or less, 4 or less, 3 or less, 2 or less or 1 amino acid(s) in the hinge region.

The antibody used in the method of the invention may be a chimeric antibody, a humanized antibody or a human antibody or a fragment thereof. In some embodiments the antibody used in the methods of the invention is a single chain antibody, a bispecific antibody or an antibody fragment, preferably a F(ab')2 fragment. In a specific embodiment the antibody of the invention may also be an Fc fusion protein, as long as it contains a CH1 and/or upper hinge region.

The antibody used in the method of the present invention may bind to any target antigen as long as it is capable of inducing direct cytotoxicity. Preferably the antibody binds to a cell surface antigen. Examples of suitable target antigens are CD2, CD3, CD20, CD22, CD30, CD33, CD37, CD40, CD44, CD44v6, CD49d, CD52, EGFR1 (HER1), EGFR2 (HER2), GD3, IGF, VEGF, TNF, IL2, IL-5R and IgE. Suitable antibodies according to the invention are, without being limited thereto, anti-CD20 (e.g., rituximab, ofatumumab), anti-CD30 (e.g., brentuximab), anti-CD37, anti-CD52 (e.g., alemtuzumab), anti-CD44v6 (e.g., bivatuzumab), anti-EGFR1 (e.g, cetuximab), anti-EGFR2 (e.g.,trastuzumab, pertuzumab, herceptin) antibodies.

Preferably the antibody used in the method of the present invention binds to a cancer-related antigen, more preferably the antibody binds to CD20, CD37, HER2 and CD44v6. More preferably, the antibody variants of the invention are based on anti-CD20 antibody rituximab, anti-HER2 antibody herceptin, anti-CD44v6 antibody bivatuzumab or an anti-CD37 antibody, even more preferably the antibody variants are based on rituximab or an anti-CD37 antibody.

The method of the invention result in an antibody variant of the invention exhibiting a superior direct cytotoxic activity compared to unmodified antibodies as measured by Annexin V staining. Preferably, said direct cytotoxic activity is apoptosis. Typically, the direct cytotoxic activity is induced by receptor crosslinking, receptor dimerization, blocking of receptor dimerization, by signalling involving a death domain or by blocking ligand receptor interactions. Without wishing to be bound by theory, receptor crosslinking or receptor dimerization is likely to be the most common mechanism of action.

The enhanced direct cytotoxicity corresponds to an increase in direct cytotoxic activity compared to the unmodified IgG1 antibody of at least 120%, of at least 150%, of at least 200%, preferably of at least 300%, more preferably of at least 400% and most preferably of at least 500%, as measured by Annexin V staining.

The skilled person will understand that the direct cytotoxic activity of an unmodified IgG1 antibody may vary between different antibodies even if directed to the same target antigen. For example anti-CD20 antibodies have two major types of effector function profiles, termed type I or type II. The type II antibodies are generally better inducers of direct cytotoxicity than the type I antibodies. The majority of CD20 antibodies, including rituximab, veltuzumab, ocrelizumab and ofatumumab are of type I. The skilled person will further understand that the enhanced direct cytotoxicity of the antibody with the one or more amino acid modification(s) according to the invention is more pronounced in antibodies that have a low direct cytotoxicity without said amino acid modification(s). Thus, the invention is particularly useful for enhancing the direct cytotoxic activity of antibodies having a "% apoptosis induction" of 20% or less or even 10% or less as measured by Annexin V staining according to the method disclosed below. Alternatively the invention is particularly useful for enhancing the direct cytotoxic activity of IgG1 antibodies that have an at least 50% lower apoptosis induction compared to the corresponding IgG2 and/or IgG4P antibody, preferably compared to the corresponding IgG2 and IgG4P antibody, as measured by Annexin V staining.

Also provided is an antibody obtainable by the method of the invention.

### Antibody production

A method is provided for producing the antibody of the invention comprising (a) transfecting a mammalian host cell with the DNA of the invention or the vector of the invention, (b) optionally transfecting the mammalian host cell with a DNA comprising as sequence encoding an antibody light chain, (c) culturing the host cell under conditions suitable for expressing the antibody, and (d) recovering and purifying the antibody molecule.

For producing the recombinant antibody molecules of the invention, the DNA molecules encoding full-length light and heavy chains or fragments thereof are inserted into an expression vector such that the sequences are operatively linked to transcriptional and translational control sequences. Alternatively, DNA molecules encoding light chain variable regions and heavy chain variable regions can be chemically synthesized using the sequence information provided herein. Synthetic DNA molecules can be ligated to other appropriate nucleotide sequences, including, e.g., constant region coding sequences, and expression control sequences, to produce conventional gene expression constructs encoding the desired antibody. For manufacturing the antibodies of the invention, the skilled artisan may choose from a great variety of expression systems well known in the art, e.g. those reviewed by Kipriyanow and Le Gall, 2004. Expression vectors include plasmids, retroviruses, cosmids, EBV-derived episomes, and the like. The term "expression vector" comprises any vector suitable for the expression of a foreign DNA. Examples of such expression vectors are viral vectors, such as adenovirus, vaccinia virus, baculovirus and adeno-associated virus vectors. In this connection, the expression "virus vector" is understood to mean both a DNA and a viral particle. Examples of phage or cosmid vectors include pWE15, M13, λEMBL3, λEMBL4, λFIXII, λDASHII, λZAPII, λgT10, λgt11, Charon4A and Charon21A. Examples of plasmid vectors include pBR, pUC, pBluescriptll, pGEM, pTZ and pET groups. Various shuttle vectors may be used, e.g., vectors which may autonomously replicate in a plurality of host microorganisms such as *E. coli* and Pseudomonas sp. In addition, artificial chromosome vectors are considered as expression vectors. The expression vector and expression control sequences are selected to be compatible with the host cell. Examples of mammalian expression vectors include, but are not limited to, pcDNA3, pcDNA3.1 (+/-), pGL3, pZeoSV2(+/-), pSecTag2, pDisplay, pEF/myc/cyto, pCMV/myc/cyto, pCR3.1, pSinRepS, D H26S, D HBB, pNMT1, pNMT41, pNMT81, which are available from Invitrogen, pCI which is available from Promega, pMbac, pPbac, pBK-RSV and pBK-CMV which are available from Stratagene, pTRES which is available from Clontech, and their derivatives.

The antibody light chain gene and the antibody heavy chain gene can be inserted into separate vectors. In certain embodiments, both DNA sequences are inserted into the same expression vector. Convenient vectors are those that encode a functionally complete human CH or CL immunoglobulin sequence, with appropriate restriction sites engineered so that any VH or VL sequence can be easily inserted and expressed, as described above, wherein the CH1 and/or upper hinge region comprises at least one amino acid modification of the invention. The constant chain is usually kappa or lambda for the antibody light chain. The recombinant expression vector may also encode a signal peptide that facilitates secretion of the antibody chain from a host cell. The DNA encoding the antibody chain may be cloned into the vector such that the signal peptide is linked in-frame to the amino terminus of the mature antibody chain DNA. The signal peptide may be an immunoglobulin signal peptide or a heterologous peptide from a non-immunoglobulin protein. Alternatively, the DNA sequence encoding the antibody chain may already contain a signal peptide sequence. In addition to the DNA sequences encoding the antibody chains, the recombinant expression vectors carry regulatory sequences including promoters, enhancers, termination and polyadenylation signals and other expression control elements that control the expression of the antibody chains in a host cell. Examples for promoter sequences (exemplified for expression in mammalian cells) are promoters and/or enhancers derived from (CMV) (such as the CMV Simian Virus 40 (SV40) (such as the SV40 promoter/enhancer), adenovirus, (e. g., the adenovirus major late promoter (AdMLP)), polyoma and strong mammalian promoters such as native immunoglobulin and actin promoters. Examples for polyadenylation signals are BGH polyA, SV40 late or early polyA; alternatively, 3'UTRs of immunoglobulin genes etc. can be used.

The recombinant expression vectors may also carry sequences that regulate replication of the vector in host cells (e.g. origins of replication) and selectable marker genes. Nucleic acid molecules encoding the heavy chain or an antigen-binding portion thereof and/or the light chain or an antigen-binding portion thereof of an antibody of the present invention, and vectors comprising these DNA molecules can be introduced into host cells, e.g. bacterial cells or higher eukaryotic cells, e.g. mammalian cells, according to transfection methods well known in the art, including liposome-mediated transfection, polycation-mediated transfection, protoplast fusion, microinjections, calcium phosphate precipitation, electroporation or transfer by viral vectors.

It is within ordinary skill in the art to express the heavy chain and the light chain from a single expression vector or from two separate expression vectors. Preferably, the DNA molecules encoding the heavy chain and the light chain are present on two vectors which are co-transfected into the host cell, preferably a mammalian cell.

Mammalian cell lines available as hosts for expression are well known in the art and include, inter alia, Chinese hamster ovary (CHO, CHO-DG44, BI-HEX-CHO) cells, NSO, SP2/0 cells, HeLa cells, HEK293 cells, baby hamster kidney (BHK) cells, monkey kidney cells (COS), human carcinoma cells (e. g., Hep G2), A549 cells, 3T3 cells or the derivatives/progenies of any such cell line. Other mammalian cells, including but not limited to human, mice, rat, monkey and rodent cells lines, or other eukaryotic cells, including but not limited to yeast, insect and plant cells, or prokaryotic cells such as bacteria may be used. The antibody molecules of the invention are produced by culturing the host cells for a period of time sufficient to allow for expression of the antibody molecule in the host cells. Following expression, the intact antibody (or the antigen-binding fragment of the antibody) can be harvested and purified using techniques well known in the art, e.g., Protein A, Protein G, affinity tags such as glutathione-S-transferase (GST) and histidine tags.

### Protein purification

Antibody molecules are preferably recovered from the culture medium as a secreted polypeptide or it can be recovered from host cell lysates if for example expressed without a secretory signal. It is necessary to purify the antibody molecules using standard protein purification methods used for recombinant proteins and host cell proteins in a way that substantially homogenous preparations of the antibody are obtained. By way of example, state-of-the art purification methods useful for obtaining the antibody molecule of the invention include, as a first step, removal of cells and/or particulate cell debris from the culture medium or lysate. The antibody is then purified from contaminant soluble proteins, polypeptides and nucleic acids, for example, by fractionation on immunoaffinity or ionexchange columns, ethanol precipitation, reverse phase HPLC, Sephadex chromatography, chromatography on silica or on a cation exchange resin. Preferably, antibodies are purified by standard protein A chromatography, e.g., using protein A spin columns (GE Healthcare). Protein purity may be verified by reducing SDS PAGE. Antibody concentrations may be determined by measuring absorbance at 280nm and utilizing the protein specific extinction coefficient. As a final step in the process for obtaining an antibody molecule preparation, the purified antibody molecule may be dried, e.g. lyophilized, as described below for therapeutic applications.

### Direct cytotoxicity assay

Programmed cell death, also referred to as apoptosis, is characterized by distinct biochemical and morphological changes in a cell. *In vivo,* apoptotic cells are efficiently removed by phagocytes and do not trigger inflammatory processes. Apoptotic characteristics include the externalization of phosphatidylserine (PS), caspase activation, cell shrinkage, membrane blebbing, cell detachment and chromatin condensation and fragmentation (Henry et al., 2013).

These characteristics can be used to measure apoptotic events in cell populations *in vitro.* One method known in the art suitable for measuring apoptosis or direct cytotoxicity is the "Annexin V staining method", which involves the phospholipid binding protein Annexin V, which preferentially binds to PS. In healthy cells PS resides exclusively on the inner plasma membrane. In cells exposed to apoptotic stimuli, however, PS is quickly externalized (Martin et al., 1995). Hence, Annexin V can bind to the PS molecules appearing on the outer cell membrane. Labeled Annexin V is widely used in flow cytometric apoptosis assays. Preferably, the Annexin V is fluorescently labelled or biotin labelled. The biotin labelled Annexin V is detected in a further step using fluorescently labelled streptavidin, avidin or derivatives thereof as a secondary reagent. Fluorescent labels suitable for use in flow cytometry are known in the art and involve without being limited thereto R-Phycoerythrine (PE), Allophycocyanine (APC), Cy dyes such as Cy2 or Cy3, PE-Cy5, PE-Cy7, Fluorescein (FITC), TRITC and Alexa Fluor dyes such as Alexa Fluor 488. Cells are typically counterstained with the DNA intercalating dye propidium iodide (PI). PI can only enter the cells once membrane integrity is compromised, a symptom of either late apoptosis or cell death by the distinct mechanism of necrosis. In apoptosis (and other forms of direct cytotoxicity), cells will bind Annexin V first, before eventually turning PI positive. Necrosis is characterized by cells becoming Annexin V and PI positive simultaneously.

Following the treatment of cell lines with therapeutic antibodies, Annexin V and PI staining and flow cytometry, direct cytotoxic activity can be easily quantified. Three cellular populations can be readily identified, those being double negative (live cells), Annexin V positive but PI negative (early apoptotic) or double positive (late apoptotic or necrotic). The total percentage of early and late apoptotic cells, in relation to a control antibody, can be used to estimate an antibody's apoptotic potential (% normalized apoptosis induction).

The skilled person will appreciate that the Annexin V staining method is suitable to measure apoptosis, but also to measure non-apoptotic direct cell death, or non-classical apoptosis. Thus, Annexin V staining may be used to determine the direct cytotoxic activity of an antibody, wherein the antibody induces direct cytotoxicity in a target cell by inducing apoptosis, non-apoptotic direct cell death or non-classical apoptosis.

Typically antibody variants are incubated with target expressing cells for 24hr at about 1000-2000 ng per 100,000 cells. The exact antibody concentration should be titrated prior to the experiment. Subsequently cells are stained with fluorescently labelled Annexin V (e.g., Annexin V-FITC) and PI dyes (BD Biosciences) and measured in a flow cytometer, e.g. using a FACS Calibur flow cytometer (BD Biosciences) and analysing the data using FlowJo software package (Treestar).

A suitable target cell expresses the antigen to which the antibody binds on the cells surface. Ramos cells are derived from human Burkitt's lymphoma and express typical B cell markers, such as CD20 or CD37. Ramos cells (ATCC CRL-1596) are therefore suitable as target cells for detecting the cytotoxic activity of anti-CD20 antibodies, such as Rituximab. The skilled person is aware that the target cell depends on the antibody to be measured and he/she will know which target cell to use for a specific antibody. Suitable target cells for detecting the direct cytotoxic activity of anti-HER2 or anti-CD44v6 antibodies are, e.g., A431 (epidermoid carcinoma) or MDA-MB-468 cells (adenocarcinoma cell line). Suitable target cells for detecting the direct cytotoxic activity of anti-CD52 antibodies are, e.g., B lymphoid leukemia cells, such as Wien-133 cells. Suitable target cells for detecting the direct cytotoxic activity of anti-EGFR antibodies are, e.g., colon colorectal cancer cells, such as HT-29 cells. Suitable target cells for detecting the direct cytotoxic activity of anti-CD33 antibodies are, e.g., promyelocytic leukemia, such as HL-60 cells and suitable target cells for detecting the direct cytotoxic activity of anti-VEGF antibodies are, e.g., kidney carcinoma cells, such as A498 cells.

The antibody variants of the invention exhibit a superior direct cytotoxic activity compared to unmodified antibodies as measured by Annexin V staining. The improvement corresponds to an increase in direct cytotoxic activity compared to an unmodified IgG1 antibody of at least 120%, of at least 150%, of at least 200%, preferably of at least 300%, more preferably of at least 400% and most preferably of at least 500%, as measured by Annexin V staining.

Multiple other methods for measuring apoptosis exist. Confocal or phase microscopy can be used to observe membrane blebbing, cellular shrinkage and detachment. DNA intercalating dyes such as Hoechst 33342 can help visualize chromatin condensation and fragmentation. Alternatively, DNA fragmentation can be visualized by flow cytometry. PI staining allows characterizing a cell's replication profile (cell cycle) as fluorescence signals are proportional to the cell's DNA content. Resting cells in G1 have stable chromosomal contents, mitotic cells (G2/M) double their DNA content and thus the obtainable fluorescence signal, whereas fragmented DNA in apoptotic cells results in reduced (sub-G1) fluorescence intensity. Cellular DNA can also be isolated and assessed by gel electrophoresis. Apoptotic DNA fragments (ladders) can be readily distinguished from intact DNA of live cells. Terminal deoxynucleotidyl transferase dUTP nick end labeling (TUNEL) is an additional option to identify apoptotic DNA damage. Labeled dUTP is added to DNA fragment ends by the terminal deoxynucleotidyl transferase and can subsequently be measured or visualized. Further, the sequential activation caspases may occur. Several assay formats measure the activation state of such caspases by utilizing substrates, which, upon caspase cleavage, luminesce or fluoresce and can thus be easily quantified.

### Pharmaceutical compositions

To be used in therapy, the antibody may be formulated into a pharmaceutical composition appropriate to facilitate administration to animals or humans. Pharmaceutical compositions containing antibodies or antibody fragments of the invention can be presented in a dosage unit form and can be prepared by any suitable method. Typical formulations of an antibody molecule can be prepared by mixing the antibody molecule with physiologically acceptable carriers, excipients or stabilizers, in the form of lyophilized or otherwise dried formulations or aqueous solutions or aqueous or non-aqueous suspensions. Carriers, excipients, modifiers or stabilizers are nontoxic at the dosages and concentrations employed. They include buffer systems such as phosphate, citrate, acetate and other anorganic or organic acids and their salts; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); proteins, such as serum albumin, gelatin, or immunoglobulins; hydrophilic polymers such as polyvinylpyrrolidone or polyethylene glycol (PEG); amino acids such as glycine, glutamine, asparagine, histidine, arginine, or lysine; monosaccharides, disaccharides, oligosaccharides or polysaccharides and other carbohydrates including glucose, mannose, sucrose, trehalose, dextrins or dextrans; chelating agents such as EDTA; sugar alcohols such as, mannitol or sorbitol; salt-forming counter-ions such as sodium; metal complexes (e.g. Zn-protein complexes); and/or ionic or non-ionic surfactants such as TWEEN(TM) (polysorbates), PLURONICS(TM) or fatty acid esters, fatty acid ethers or sugar esters. Also organic solvents can be contained in the antibody formulation such as ethanol or isopropanol. The excipients may also have a release-modifying or absorption-modifying function.

The antibody molecules may also be dried (freeze-dried, spray-dried, spray- freeze dried, dried by near or supercritical gases, vacuum dried, air-dried), precipitated or crystallized or entrapped in microcapsules that are prepared, for example, by coacervation techniques or by interfacial polymerization using, for example, hydroxymethylcellulose or gelatin and poly-(methylmethacylate), respectively, in colloidal drug delivery systems (for example, liposomes, albumin microspheres, microemulsions, nano-particles and nanocapsules), in macroemulsions or precipitated or immobilized onto carriers or surfaces, for example by pcmc technology (protein coated microcrystals). Such techniques are disclosed in Remington: The Science and Practice of Pharmacy, 21st edition, Hendrickson R. Ed.

Naturally, the formulations to be used for *in vivo* administration must be sterile; sterilization may be accomplished be conventional techniques, e.g. by filtration through sterile filtration membranes. It may be useful to increase the concentration of the antibody to come to a so-called high concentration liquid formulation (HCLF); various ways to generate such HCLFs have been described.

The antibody molecule may also be contained in a sustained-release preparation. Such preparations include solid, semi-solid or liquid matrices of hydrophobic or hydrophilic polymers, and may be in the form of shaped articles, e.g. films, sticks or microcapsules and may be applied via an application device. Examples of sustained-release matrices include polyesters, hydrogels (for example, poly(2-hydroxyethyl-methacrylate) or sucrose acetate butyrate), or poly(vinylalcohol)), polylactides (US 3,773,919), copolymers of L-glutamic acid and [gamma] ethyl-L-glutamate, non-degradable ethylenevinyl acetate, degradable lactic acid-glycolic acid copolymers such as the LUPRON DEPOT(TM) (injectable microspheres composed of lactic acid-glycolic acid copolymer and leuprolide acetate), and poly-D-(-)-3-hydroxybutyric acid. While polymers such as ethylene- vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulf[iota]de interchange, stabilization may be achieved by modifying sulfhydryl residues, lyophilization (e.g. as described in WO 89/011297) from acidic solutions, controlling moisture content, using appropriate additives, and developing specific polymer matrix compositions. Formulations that may also be used for the antibody molecule of the invention are described in US 7,060,268 and US 6,991,790.

The antibody molecule can be incorporated also in other application forms, such as dispersions, suspensions or liposomes, tablets, capsules, powders, sprays, transdermal or intradermal patches or creams with or without permeation enhancing devices, wafers, nasal, buccal or pulmonary formulations, or may be produced by implanted cells or - after gene therapy - by the individual's own cells.

An antibody molecule may also be derivatized with a chemical group such as polyethylene glycol (PEG), a methyl or ethyl group, or a carbohydrate group. These groups may be useful to improve the biological characteristics of the antibody, e.g. to increase serum half-life or to increase tissue binding.

The preferred mode of application is parenteral, by infusion or injection (intraveneous, intramuscular, subcutaneous, intraperitoneal, intradermal), but other modes of application such as by inhalation, transdermal, intranasal, buccal, oral, may also be applicable.

For the prevention or treatment of disease, the appropriate dosage of antibody will depend on the type of disease to be treated, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments.

### Therapy

The antibodies and the pharmaceutical compositions of the invention can be used in therapy or for prophylaxis. An antibody having enhanced direct cytotoxicity is useful in the prevention and treatment of various diseases including primary or metastatic neoplastic disease (i.e., cancer), allergy, autoimmune diseases and viral or bacterial infection. Preferably, the antibody binds to a cancer-related antigen and is useful in treating cancer.

A method of treating a patient in need thereof comprising administering an antibody according to the invention to said patient is also provided. A "patient" for the purposes of the present invention includes both, humans and other animals, preferably mammals and most preferably humans. However, the antibody variants of the present invention have mainly human therapy applications. The term "treatment" in the present invention is meant to include therapeutic treatment, as well as prophylactic, or suppressive measures for a disease or disorder. Thus, for example, successful administration of an antibody variant prior to onset of the disease results in treatment of the disease. "Treatment" also encompasses administration of an antibody variant after the appearance of the disease in order to eradicate the disease. Successful administration of an antibody variant after onset and after clinical symptoms, with possible abatement of clinical symptoms and perhaps amelioration of the disease, comprises treatment of the disease. Those "in need of treatment" include mammals already having the disease or disorder, as well as those prone to having the disease or disorder, including those in which the disease or disorder is to be prevented.

The antibody variants of the present invention can be administered to a patient having a disease or disorder involving inappropriate expression of a protein, including diseases characterized by aberrant proteins, due for example to alterations in the amount of a protein present, protein localization, posttranslational modification, conformational state, or protein mutation, etc. Similarly, an antibody variant of the present invention can be administered to a patient having a disease or disorder characterized by alterations in molecules such as polysaccharides and gangliosides. An overabundance or reduction may be due to any cause, including but not limited to overexpression at the molecular level, prolonged or accumulated appearance at the site of action, or increased activity of a protein relative to normal.

In principle, any binding partner or antigen may be targeted by the antibody variants of the present invention, including but not limited to proteins and subunits, domains, motifs, and epitopes belonging to said proteins.

In cancer, the cells tend to hyperproliferate and cancer is typically characterized by unregulated cell growth. Conventional anticancer agents have a characteristic in inhibiting proliferation of cancer cells. Antibodies of the present invention having an enhanced direct cytotoxic activity can treat cancers by preventing proliferation of the target cancer cell through their cytotoxic effect more effectively than the corresponding unmodified antibody. Exemplary suitable cancer target antigens for the antibodies of the invention include, but are not limited to CD2, CD3, CD20, CD22, CD30, CD33, CD37, CD40, CD44, CD44v6, CD49d, CD52, EGFR1 (HER1 EGFR2 (HER2), IL-5R, IGF, VEGF, TNFalpha, IL2 Preferred cancer target antigens are CD20, CD37, CD44v6 and HER2. Specifically, anti-CD20 and anti-CD37 antibodies are suitable for treating B-cell malignancies, including B cell lymphomas, such as B-cell non-Hodgkin lymphoma, leukemias, such as acute lymphoblastic leukemia (ALL) and chronic lymphocytic leukemia (CLL) and myelomas, anti-CD44v6 antibodies are suitable for treating patients having metastases of cancers such as colon cancer, head and neck cancer and ovarian cancer and anti-HER2 antibodies are suitable for treating patients with breast cancer and gastric cancer.

Allergic reactions and autoimmune diseases are induced or accelerated by the release of a mediator molecule from immune cells. Thus, the antibody of the present invention having an enhanced direct cytotoxic activity are suitable for treating allergic reactions or autoimmune diseases by removing the responsible immune cells more effectively than the corresponding control antibody. Target antigens that are relevant for such diseases include but are not limited to α4β7 integrin, β2 integrin, BAFF (BLys), C5, CD2, CD3, CD4, CD11a, CD14, CD18, CD20, CD22, CD23, CD25, CD40L, CD52, CD64, CD80, CD147, E-selectin, gpIIb/IIIa, ICAM-3, ICE, FcR1, IgE, IL-4, IL-5, IL-8, IFNγ, TNF and VLA-4. Exemplary suitable target antigens for the antibodies of the invention and relevant for allergic reactions and autoimmune diseases include, but are not limited to BAFF (BLys), CD2, CD3, CD11a, CD20, CD22, CD52 and TNF. Specifically, anti-CD20 antibodies are suitable for treating rheumatoid arthritis and systemic lupus erythrematodes (SLE).

Viral and bacterial infections can be prevented and treated by killing the virus- or bacterium-infected cell. Thus, the antibodies of the present invention having an enhanced direct cytotoxic activity are suitable for treating viral or bacterial infections by removing the infected cells more effectively than the corresponding control antibody. Examplary suitable target antigens or infective agents for the antibodies of the invention that are relevant for such virus- or bacterium-infected cells include but are not limited to Hepatitis B virus (e.g., exbivirumab, tuvirumab), hsp90 (invasive candidiasis, e.g., efungumab), rabies virus (e.g., foravirumab), HIV or the primary receptor for HIV CD4 (e.g., ilbalizumab), CMV (e.g., regavirumab or sevirumab), toxin of *Bacillus anthracis* (e.g., raxibacumab), RSV, particularly the F protein of RSV (e.g., motavizumab or palivizumab) or *Clostridium difficile* enterotoxin A and B (e.g., actoxumab or bezlotoxumab).

Antibodies and antibody compositions disclosed herein can be used to treat various forms of cancer, including but not limited to the following: AIDS-related cancer, bone related cancer, brain related cancer, digestive/gastrointestinal related cancer, endocrine related cancer, eye related cancer, genitourinary related cancer, germ cell related cancer, gynaecologic related cancer, head and neck related cancer, hematologic/blood related cancer such as leukemias, lung related cancer such as non-small cell lung cancer and small cell lung cancer, musculoskeletal related cancer, neurologic related cancer, respiratory/thoracic related cancer, and skin related cancer. These disorders have been well characterized in man, but also exist with a similar etiology in other mammals, and can be treated by pharmaceutical compositions of the present invention. The cancer cells are exposed to a therapeutically effective amount of the antibody or antibody composition so as to inhibit or reduce proliferation of the cancer cells. In some embodiments, the antibody or antibody composition inhibit cancer cell proliferation by at least 40%, 50%, 60%, 70%, 80%, 90%, 95%, 98%, 99%, or 100%.

In some embodiments, the disclosed antibodies can be used in a method to inhibit tumour growth in a mammal, e.g. a human patient. The method comprises administering to the patient a therapeutically effective amount of the antibody or antibody composition.

Depending on the disorder to be treated, the antibody of the invention may be used on its own or in combination with one or more additional therapeutic agents, in particular selected from DNA damaging or tubulin binding agents or therapeutically active compounds that inhibit angiogenesis, signal transduction pathways or mitotic checkpoints in cancer cells. The additional therapeutic agent may be administered simultaneously with the antibody of the invention, optionally as a component of the same pharmaceutical preparation, or before or after administration of the antibody of the invention. Moreover the antibody of the invention may be used on its own or in the form of a conjugate, i.e., an antibody molecule that is chemically coupled to a cytotoxic agent that induces cytotoxicity (e.g., apoptosis or mitotic arrest) such as doxorubicin, mertansine, ricin A toxin, alkylating agents (e.g., CC-1065 analogs), anthracyclines, tubulysin analogs, duocarmycin analogs, myatansinoids (e.g., maytansine, maytansinol, C-3 ester of maytansinol) and auristatin E. Alternatively the antibody may also be coupled to a radioisotope such as Phosphorus-32, Strontium-89, Yttrium-90, Iodine-131, Samarium-153, Erbium-169, Ytterbium-175 and Rhenium-188.

As used herein, "therapy" is synonymous with treating a disease, which includes reducing symptoms of the disease, inhibiting progression of the disease, causing regression of the disease and/or curing the disease.

Generally, a therapeutically effective amount of active component is in the range of about 0.1 mg/kg to about 500 mg/kg, preferably in the range of about 1 mg/kg to about 100 mg/kg. The amount administered will depend on variables such as the type and extent of disease or indication to be treated, the overall health of the patient, the in vivo potency of the antibody, the pharmaceutical formulation, and the route of administration. The initial dosage can be increased beyond the upper level in order to rapidly achieve the desired blood-level or tissue level. Alternatively, the initial dosage can be smaller than the optimum, and the daily dosage may be progressively increased during the course of treatment. Human dosage can be optimized, e.g., in a conventional Phase I dose escalation study designed to run from 0.5 mg/kg to 20 mg/kg. Dosing frequency can vary, depending on factors such as route of administration, dosage amount and the disease being treated. Exemplary dosing frequencies are once per day, once per week and once every two weeks. Formulation of monoclonal antibody-based drugs is within ordinary skill in the art. In some embodiments of the invention a monoclonal antibody is lyophilized and reconstituted in buffered saline at the time of administration. Due to the enhanced cytotoxic activity of the antibody of the invention, the antibody may preferably be administered at a lower effective dose or less frequently compared to the corresponding unmodified antibody. The antibody of the present invention may further result in a reduced relapsing of the disease to be treated or reduce the incidence of drug resistance or increase the time until drug resistance is developing.

### Examples

### Example 1: IgG2 and IgG4 subtypes are more effective at inducing direct cytotoxicity than IgG1

Antibodies (mAb1; Figure 1) were formatted as IgG1, IgG2 or IgG4 subtypes -carrying an identical variable heavy chain domain, by cloning the variable region from IgG1 into existing plasmids encoding for IgG2, IgG4P or IgG4DM antibodies. All monoclonal antibodies were expressed in BI-HEX-CHO cells under serum-free conditions and purified via MabSelect Protein A affinity chromatography (GE Healthcare). The antibodies were then assessed for their apoptotic potential. In an *in vitro* direct cytotoxicity assay utilizing a target specific cell line (Ramos cells), IgG2 and IgG4 formats were more effective at inducing direct cytotoxicity than IgG1 a1 and IgG1 a2 (Figure 1).

**Table 1**

| **Antibody** | **Target** | **Equivalent to** | **Type** | **Target Cell** | **Sequence** |
|---|---|---|---|---|---|
| mAb1 | CD20 | rituximab | chimeric | Ramos | HC: SEQ ID NO: 161* |
| | | | | | LC: SEQ ID NO: 162* |

| | | | | | |
|---|---|---|---|---|---|
| *Sequences of heavy chain (HC) and light chain (LC) without signal peptide | | | | | |

Target cells were cultured in UltraCULTURE medium (Lonza) with 2 mM L-glutamine and seeded at 3-5x10⁵ cells/ml 24h prior to the assay. Antibody variants and control antibodies were incubated with target expressing cells (1x10⁶ cells/ml) in 100 µl for 24hr at 37°C. 2000 ng (mAb1) were used per 100,000 Ramos cells. Subsequently 100 µl 2x Annexin Binding buffer (10 x buffer: 0.1 M Hepes/NaOH pH 7.4; 1.4 M NaCl; 25 mM CaCl₂, filter sterilized) was added to each well and cells were incubated in the presence of Annexin V FITC (2.5 µl/well) and propidium iodide (PI; 1 µl/well) (both BD Biosciences) for 15 minutes at RT in the dark prior to analysis. As controls, cells were incubated without mAb1, with a negative control antibody (MT201, an antibody not binding to the target cell) and with a positive control antibody (e.g., Rit-IgG4P). Unstained controls and controls stained with Annexin V-FITC alone or PI alone were also added. Analysis was performed using a FACSCalibur flow cytometer (BD Biosciences) or a GUAVA 96w flow cytometer. Experiments were performed at least twice per antibody variant, preferably at least three times. Data were analyzed using the FlowJo software package (Treestar). Shown in Figure 1 is the calculated sum of Annexin V-FITC and Annexin V-FITC/PI double positive cells with the background apoptosis (untreated cells) substracted from all samples given as % apoptosis induction ± SD.

The IgG2, IgG4DM and IgG4P allotypes were more effective in inducing direct cytotoxicity than the corresponding IgG1 controls by about 5 times, about 4 times and about 3.5 times, respectively (Figure 1). The exact values are presented in Table 2.

**Table 2: Raw/Absolute Direct Cytotoxicity Data for mAb1 controls**

| **without cross-linking mAb** | **Mutant** | **Mean direct cytotoxic activity %** | **Standard deviation %** | **N** |
|---|---|---|---|---|
| **mAb1** | IgG1 a1 | 11 | 3 | 10 |
| | IgG1 a2 | 10 | 3 | 5 |
| | IgG2 | 49 | 6 | 9 |
| | IgG4P* | 41 | 8 | 3 |
| | IgG4DM** | 36 | 4 | 2 |
| | negative | 0.2 | 0.4 | 5 |
| *IgG4 subtype variant with hinge mutation S228P | | | | |
| **IgG4 subtype variant with hinge mutations S228P/L234E | | | | |

### Example 2: Investigating the key amino acid residues involved in direct cytotoxicity induction using mAb1

Based on the observations in Example 1, residues in the antibody's heavy chain which are responsible for the divergent apoptosis induction potential of the subtypes were investigated.

The mutation matrix in Figure 7 and Figures 2 and 4 illustrate the major differences between IgG1, IgG2 and IgG4P allotypes, which are predominantly located in the CH1 domain (residues 131 to 214) or the upper and lower hinge parts of the CH2 domain (residues 217 to 237). Using mAb1 as a model antibody a large variety of mutations, in combination or separately, were introduced into the CH1 domain and upper hinge region of IgG1 by inserting point mutations (Figure 7).

**Table 3: Mutant Classification**

| **Mutant** | **Region** | **SEQ ID NO:** |
|---|---|---|
| D | Hinge/CH2 | SEQ ID NO: 9¹ |
| K | Hinge/CH2 | SEQ ID NO: 10¹ |
| T | Hinge/CH2 | SEQ ID NO: 11¹ |
| DT | Hinge/CH2 | SEQ ID NO: 12¹ |
| DK | Hinge/CH2 | SEQ ID NO: 13¹ |
| KT | Hinge/CH2 | SEQ ID NO: 14¹ |
| DKT | Hinge/CH2 | SEQ ID NO: 15¹ |
| Ih2 | Hinge/CH2 | SEQ ID NO: 16¹ |
| Ih4 | Hinge/CH2 | SEQ ID NO: 17¹ |
| uch2 | Hinge/CH2 | SEQ ID NO: 18² |
| uch4 | Hinge/CH2 | SEQ ID NO: 19¹ |
| P217R | Hinge/CH2 | SEQ ID NO: 20¹ |
| P217S | Hinge/CH2 | SEQ ID NO: 21¹ |
| S219C | Hinge/CH2 | SEQ ID NO: 22¹ |
| S219Y | Hinge/CH2 | SEQ ID NO: 23¹ |
| C220G | Hinge/CH2 | SEQ ID NO: 24¹ |
| S219C-C220G | Hinge/CH2 | SEQ ID NO: 25¹ |
| DKT-S219C | Hinge/CH2 | SEQ ID NO: 26¹ |
| DK-S219C | Hinge/CH2 | SEQ ID NO: 27¹ |
| DT-S219C | Hinge/CH2 | SEQ ID NO: 28¹ |
| KT-219C | Hinge/CH2 | SEQ ID NO: 29¹ |
| H224V | Hinge/CH2 | SEQ ID NO: 30¹ |
| H224P | Hinge/CH2 | SEQ ID NO: 31¹ |
| T225P | Hinge/CH2 | SEQ ID NO: 32¹ |
| T225E | Hinge/CH2 | SEQ ID NO: 33¹ |
| H224P-T225P | Hinge/CH2 | SEQ ID NO: 34¹ |
| H224V-T225E | Hinge/CH2 | SEQ ID NO: 35¹ |
| ICH1 | CH1 | SEQ ID NO: 36³ |
| uCH1 | CH1 | SEQ ID NO: 37³ |
| S131C | CH1 | SEQ ID NO: 38³ |
| K133R | CH1 | SEQ ID NO: 39³ |
| S131C-K133R | CH1 | SEQ ID NO: 40³ |
| G137E-G138S | CH1 | SEQ ID NO: 41³ |
| S192N | CH1 | SEQ ID NO: 42³ |
| L193F | CH1 | SEQ ID NO: 43³ |
| S192N-L193F | CH1 | SEQ ID NO: 44³ |
| Q196K | CH1 | SEQ ID NO: 45³ |
| R214T | CH1 | SEQ ID NO: 46³ |
| uch2-S131C | Hinge/CH1 | SEQ ID NO: 47⁴ |
| uch4-S131C | Hinge/CH1 | SEQ ID NO: 48⁴ |
| DKT-S131C | Hinge/CH1 | SEQ ID NO: 49⁴ |
| DK-S131C | Hinge/CH1 | SEQ ID NO: 50⁴ |
| KT-S 131C | Hinge/CH1 | SEQ ID NO: 51⁴ |
| DT-S131C | Hinge/CH1 | SEQ ID NO: 52⁴ |
| S131C-S219C | Hinge/CH1 | SEQ ID NO: 53⁴ |
| DKT-S131C-S219C | Hinge/CH1 | SEQ ID NO: 54⁴ |
| DK-S131C-S219C | Hinge/CH1 | SEQ ID NO: 55⁴ |
| KT-S131C-S219C | Hinge/CH1 | SEQ ID NO: 56⁴ |
| DT-S131C-S219C | Hinge/CH1 | SEQ ID NO: 57⁴ |
| R214T-P217R | Hinge/CH1 | SEQ ID NO: 58⁴ |
| R214T-P217S | Hinge/CH1 | SEQ ID NO: 59⁴ |
| ¹SEQ ID NOs: 9 to 17 and 19 to 35 correspond to amino acid residues 216 to 238 according to the EU numbering, as shown in Figure 2. | | |
| ²SEQ ID NO: 18 corresponds to amino acid residues 216 to 238 according to the EU | | |
| numbering, as shown in Figure 2 and further includes amino acid residues 214 (T) and 215 (V). | | |
| ³SEQ ID NOs: 36 to 46 correspond to amino acid residues 131 to 214 of the modified CH1 region according to the EU numbering as shown in Figure 4, including amino acid residues 138-192. | | |
| ⁴SEQ ID NOs: 47 to 59 correspond to amino acid residues 131 to 238 according to the EU numbering, including amino residues 138-192 and residue 215 (valine). | | |

Expression vectors for the individual mAbs, with heavy and light chain carried on separate plasmids, were assembled using routine molecular biology methods (Sambrook and Russell, 2001). The point mutations required to effect amino acid changes in the hybrid antibodies were introduced into the expression plasmids by Quickchange mutagenesis (Cormack, 2001) using mutation specific primers (MWG, see Table 4) and a high processing, proof-reading polymerase with low strand displacement activity.

In more detail, point mutations were introduced using Phusion Hot Start Flex polymerase (NEB) (50 ng plasmid, 0.03 µM forward primer, 0.03 µM reverse primer, 12.5 ul 2 x Phusion Buffer, 1 µl DMSO, 0.2 mM dNTPs, 0.5 µl Phusion polymerase and water in 25 µl) and the following PCR cycling program: 1 x 98°C for 30 sec; 16-20 x 98°C for 10 sec, 55°C for 30 sec and 65°C for 30-60 sec/kb; 1 x 65°C for 300 sec and holding at 4°C. The amplified product was digested with Dpnl and cloned into the pTT5 plasmid encoding for the heavy chain of an IgG1 antibody (pTT5-Rit-HC-IgG1). pTT5 plasmid encoding for the modified heavy chain of the IgG1 antibody was further transformed into NEB10beta or Top10 cells. The forward and revers primers used for introducing point mutations to generate the mutants as shown in Table 3 are listed in Table 4.

**Table 4: Forward and Reverse Primer**

| **Mutant** | **Primer** | **Sequence** | **Based on** | **SEQ ID** |
|---|---|---|---|---|
| K | for | GAGCCCAAATCTTGTGACACTCACACATGCCCACCGTG | wt | 60 |
| | rev | CACGGTGGGCATGTGTGAGTGTCACAAGATTTGGGCTC | | 61 |
| T | for | CCCAAATCTTGTGACAAACACACATGCCCACCGTGCCC | wt | 62 |
| | rev | GGGCACGGTGGGCATGTGTGTTTGTCACAAGATTTGGG | | 63 |
| DT | for | CAAGAGAGTTGAGCCCAAATCTTGTAAACACACATGCCC ACCGTGCCCAGCAC | wt | 64 |
| | rev | GTGCTGGGCACGGTGGGCATGTGTGTTTACAAGATTTG GGCTCAACTCTCTTG | | 65 |
| DK | for | GGACAAGAGAGTTGAGCCCAAATCTTGTACTCACACATG CCCACCGTGCCCAGC | wt | 66 |
| | rev | GCTGGGCACGGTGGGCATGTGTGAGTACAAGATTTGGG CTCAACTCTCTTGTCC | | 67 |
| KT | for | GAGTTGAGCCCAAATCTTGTGACCACACATGCCCACCGT GCC | wt | 68 |
| | rev | GGCACGGTGGGCATGTGTGGTCACAAGATTTGGGCTCA ACTC | | 69 |
| DKT | for | GTTGAGCCCAAATCTTGTCACACATGCCCACCGTGCCC | wt | 70 |
| | rev | GGGCACGGTGGGCATGTGTGACAAGATTTGGGCTCAAC | | 71 |
| Ih2 | for | GCCCACCGTGCCCAGCACCTCCCGTGGCGGACCGTC AGTCTTCC | wt | 72 |
| | rev | GAGGAAGACTGACGGTCCCGCCACGGGAGGTGCTGGG CACGGTGG | | 73 |
| Ih4 | for | CCGTGCCCAGCACCTGAATTCCTGGGGGGACCGTCAGT C | wt | 74 |
| | rev | GACTGACGGTCCCCCCAGGAATTCAGGTGCTGGGCACG G | | 75 |
| uch2 | for | CCAAGGTGGACAAGACAGTTGAGAGGAAATGTTGTGTC GAATGCCCACCGTGCCCAGCAC | DKT | 76 |
| | rev | GTGCTGGGCACGGTGGGCATTCGACACAACATTTCCTC TCAACTGTCTTGTCCACCTTGG | | 77 |
| uch4 | for | GTGGACAAGAGAGTTGAGTCCAAATATGGTCCCCCATG CCCACCGTGCCCAGCAC | DKT | 78 |
| | rev | GTGCTGGGCACGGTGGGCATGGGGGACCATATTTGGAC TCAACTCTCTTGTCCAC | | 79 |
| P217R | for | GGTGGACAAGAGAGTTGAGCGCAAATCTTGTGACAAAA CTC | wt | 80 |
| | rev | GAGTTTTGTCACAAGATTTGCGCTCAACTCTCTTGTCCA CC | | 81 |
| P217S | for | GGACAAGAGAGTTGAGAGCAAATCTTGTGACAAAACTC | wt | 82 |
| | rev | GAGTTTTGTCACAAGATTTGCTCTCAACTCTCTTGTCC | | 83 |
| S219C | for | GAGAGTTGAGCCCAAATGTTGTGACAAAACTCACA | wt | 84 |
| | rev | TGTGAGTTTTGTCACAACATTTGGGCTCAACTCTC | | 85 |
| S219Y | for | GAGAGTTGAGCCCAAATATTGTGACAAAACTCACA | wt | 86 |
| | rev | TGTGAGTTTTGTCACAATATTTGGGCTCAACTCTC | | 87 |
| C220G | for | AGTTGAGCCCAAATCTGGTGACAAAACTCACACATG | wt | 88 |
| | rev | CATGTGTGAGTTTTGTCACCAGATTTGGGCTCAACT | | 89 |
| S219C-C220G | for | GGACAAGAGAGTTGAGCCCAAATGTGGTGACAAAACTC ACACATGC | wt | 90 |
| | rev | GCATGTGTGAGTTTTGTCACCACATTTGGGCTCAACTCT CTTGTCC | | 91 |
| DKT-S219C | for | GAGAGTTGAGCCCAAATGTTGTCACACATGCCCACC | DKT | 92 |
| | rev | GGTGGGCATGTGTGACAACATTTGGGCTCAACTCTC | | 93 |
| DK-S219C | for | GAGAGTTGAGCCCAAATGTTGTACTCACACATGCC | DK | 94 |
| | rev | GGCATGTGTGAGTACAACATTTGGGCTCAACTCTC | | 95 |
| DT-S219C | for | GAGAGTTGAGCCCAAATGTTGTAAACACACATGCC | DT | 96 |
| | rev | GGCATGTGTGTTTACAACATTTGGGCTCAACTCTC | | 97 |
| KT-S219C | for | GAGAGTTGAGCCCAAATGTTGTGACCACACATGCC | KT | 98 |
| | rev | GGCATGTGTGGTCACAACATTTGGGCTCAACTCTC | | 99 |
| H224V | for | AATCTTGTGACAAAACTGTCACATGCCCACCGTGCC | wt | 100 |
| | rev | GGCACGGTGGGCATGTGACAGTTTTGTCACAAGATT | | 101 |
| H224P | for | CTTGTGACAAAACTCCCACATGCCCACCGTGC | wt | 102 |
| | rev | GCACGGTGGGCATGTGGGAGTTTTGTCACAAG | | 103 |
| T225P | for | GTGACAAAACTCACCCATGCCCACCGTGCCCAG | wt | 104 |
| | rev | CTGGGCACGGTGGGCATGGGTGAGTTTTGTCAC | | 105 |
| T225E | for | GTGACAAAACTCACGAATGCCCACCGTGCCCAGCA | wt | 106 |
| | rev | TGCTGGGCACGGTGGGCATTCGTGAGTTTTGTCAC | | 107 |
| H224V-T225E | for | CCAAATCTTGTGACAAAACTGTCGAATGCCCACCGTGCC CAGC | wt | 108 |
| | rev | GCTGGGCACGGTGGGCATTCGACAGTTTTGTCACAAGA TTTGG | | 109 |
| H224P-T225P | for | CCAAATCTTGTGACAAAACTCCCCCATGCCCACCGTGCC CAGC | wt | 110 |
| | rev | GCTGGGCACGGTGGGCATGGGGGAGTTTTGTCACAAGA TTTGG | | 111 |
| uCH1 | for | CTTCCCCCTGGCACCCTGCTCCAGGAGCACCTCTGAGA GCACAGCGGCCCTGGGCTG | wt | 112 |
| | rev | CAGCCCAGGGCCGCTGTGCTCTCAGAGGTGCTCCTGGA GCAGGGTGCCAGGGGGAAG | | 113 |
| ICH1 | for | GGGCACCCAGACCTACACCTGCAACGTGGATCACAAGC CCAGCAACAC | wt | 114 |
| | rev | GTGTTGCTGGGCTTGTGATCCACGTTGCAGGTGTAGGT CTGGGTGCCC | | 115 |
| K133R | for | CTTCCCCCTGGCACCCTCCTCCAGGAGCACCTCTGGGG GCACAGC | wt | 116 |
| | rev | GCTGTGCCCCCAGAGGTGCTCCTGGAGGAGGGTGCCA GGGGGAAG | | 117 |
| S131C | for | CGGTCTTCCCCCTGGCACCCTGCTCCAAGAGCACCTCT GGGGGC | wt | 118 |
| | rev | GCCCCCAGAGGTGCTCTTGGAGCAGGGTGCCAGGGGG AAGACCG | | 119 |
| S131C-K133R | for | CCCCTGGCACCCTGCTCCAGGAGCACCTCTGGGG | wt | 120 |
| | rev | CCCCAGAGGTGCTCCTGGAGCAGGGTGCCAGGGG | | 121 |
| G137EG138S | for | GCACCCTCCTCCAAGAGCACCTCTGAGAGCACAGCGGC CCTGGGCTGCCTGG | wt | 122 |
| | rev | CCAGGCAGCCCAGGGCCGCTGTGCTCTCAGAGGTGCTC TTGGAGGAGGGTGC | | 123 |
| S192N | for | GGTGACCGTGCCCTCCAGCAACTTGGGCACCCAGACC | wt | 124 |
| | rev | GGTCTGGGTGCCCAAGTTGCTGGAGGGCACGGTCACC | | 125 |
| L193F | for | CGTGCCCTCCAGCAGCTTTGGCACCCAGACCTACATCT GC | wt | 126 |
| | rev | GCAGATGTAGGTCTGGGTGCCAAAGCTGCTGGAGGGCA CG | | 127 |
| S192N-L193F | for | GGTGACCGTGCCCTCCAGCAACTTTGGCACCCAGACCT ACATCTGC | wt | 128 |
| | rev | GCAGATGTAGGTCTGGGTGCCAAAGTTGCTGGAGGGCA CGGTCACC | | 129 |
| Q196K | for | CCAGCAGCTTGGGCACCCAAACCTACATCTGCAACG | wt | 130 |
| | rev | CGTTGCAGATGTAGGTTTGGGTGCCCAAGCTGCTGG | | 131 |
| R214T | for | GCAACACCAAGGTGGACAAGACAGTTGAGCCAAAATCTT GTGACAAAACTC | wt | 132 |
| | rev | GAGTTTTGTCACAAGATTTTGGCTCAACTGTCTTGTCCAC CTTGGTGTTGC | | 133 |
| uch2-S131C | for | CGGTCTTCCCCCTGGCACCCTGCTCCAAGAGCACCTCT GGGGGC | uch2 | 134 |
| | rev | GCCCCCAGAGGTGCTCTTGGAGCAGGGTGCCAGGGGG AAGACCG | | 135 |
| uch4-S131C | for | CGGTCTTCCCCCTGGCACCCTGCTCCAAGAGCACCTCT GGGGGC | uch4 | 136 |
| | rev | GCCCCCAGAGGTGCTCTTGGAGCAGGGTGCCAGGGGG AAGACCG | | 137 |
| DKT-S131C | for | CGGTCTTCCCCCTGGCACCCTGCTCCAAGAGCACCTCT GGGGGC | DKT | 138 |
| | rev | GCCCCCAGAGGTGCTCTTGGAGCAGGGTGCCAGGGGG AAGACCG | | 139 |
| DK-S131C | for | CGGTCTTCCCCCTGGCACCCTGCTCCAAGAGCACCTCT GGGGGC | DK | 140 |
| | rev | GCCCCCAGAGGTGCTCTTGGAGCAGGGTGCCAGGGGG AAGACCG | | 141 |
| KT-S131C | for | CGGTCTTCCCCCTGGCACCCTGCTCCAAGAGCACCTCT GGGGGC | KT | 142 |
| | rev | GCCCCCAGAGGTGCTCTTGGAGCAGGGTGCCAGGGGG AAGACCG | | 143 |
| DT-S131C | for | CGGTCTTCCCCCTGGCACCCTGCTCCAAGAGCACCTCT GGGGGC | DT | 144 |
| | rev | GCCCCCAGAGGTGCTCTTGGAGCAGGGTGCCAGGGGG AAGACCG | | 145 |
| S131C-S219C | for | GAGAGTTGAGCCCAAATGTTGTGACAAAACTCACA | S131C | 146 |
| | rev | TGTGAGTTTTGTCACAACATTTGGGCTCAACTCTC | | 147 |
| DKT-S131C-S219C | for | CGGTCTTCCCCCTGGCACCCTGCTCCAAGAGCACCTCT GGGGGC | DKT-S219C | 148 |
| | rev | GCCCCCAGAGGTGCTCTTGGAGCAGGGTGCCAGGGGG AAGACCG | | 149 |
| DK-S131C-S219C | for | CGGTCTTCCCCCTGGCACCCTGCTCCAAGAGCACCTCT GGGGGC | DK-S219C | 150 |
| | rev | GCCCCCAGAGGTGCTCTTGGAGCAGGGTGCCAGGGGG AAGACCG | | 151 |
| DT-S131C-S219C | for | CGGTCTTCCCCCTGGCACCCTGCTCCAAGAGCACCTCT GGGGGC | DT-S219C | 152 |
| | rev | GCCCCCAGAGGTGCTCTTGGAGCAGGGTGCCAGGGGG AAGACCG | | 153 |
| KT-S131C-S219C | for | CGGTCTTCCCCCTGGCACCCTGCTCCAAGAGCACCTCT GGGGGC | KT-s219C | 154 |
| | rev | GCCCCCAGAGGTGCTCTTGGAGCAGGGTGCCAGGGGG AAGACCG | | 155 |
| R214T-P217R | for | CCAAGGTGGACAAGACAGTTGAGCGCAAATCTTGTGAC AAAAC | wt | 156 |
| | rev | GTTTTGTCACAAGATTTGCGCTCAACTGTCTTGTCCACCT TGG | | 157 |
| R214T-P217S | for | CCAAGGTGGACAAGACAGTTGAGAGCAAATCTTGTGACA AAAC | wt | 158 |
| | rev | GTTTTGTCACAAGATTTGCTCTCAACTGTCTTGTCCACCT TGG | | 159 |

For antibody expression Chinese hamster ovary (CHO) DG44 cells were co-transfected with plasmids pTT5-Rit-HC-IgG1X comprising a sequence encoding the modified heavy chain of the IgG1 mAb1 antibody and pTT5-Rit-LC comprising a sequence encoding the kappa light chain of the IgG1 mAb1 antibody (comprising a constant region having the sequence of SEQ ID NO: 160).

Antibodies were either produced from stable MTX amplified in BI-HEX-CHO cell lines using a routine 11 day fed-batch procedure or utilizing an optimized transient episomal expression protocol in CHO-E cells in an 8 day batch process (Durocher and Loignon, 2009). The antibodies were further purified via MabSelect Protein A affinity chromatography (GE Healthcare).

For the direct cytotoxicity assay, cells were cultured in UltraCULTURE (Lonza) with 2 mM L-glutamine, and seeded at 3-5x10⁵ cells/ml 24 h before the assay. Antibody variants were incubated with target expressing cells (1x10⁶ cells/ml) in 100 µl for 24hr at 37°C. 2000 ng mAb1 were used per 100,000 Ramos cells. Subsequently 100µl 2x Annexin Binding buffer (10 x buffer: 0.1 M Hepes/NaOH pH 7.4; 1.4 M NaCl; 25 mM CaCl₂, filter sterilized) was added to each well and cells were incubated in the presence of Annexin V-FITC (2.5 µl per well) and PI (1µl/well) (both BD Biosciences) for 15 minutes at RT in the dark prior to analysis. As controls, cells were incubated without mAb1, with a negative control antibody (MT201) and with a positive control antibody (e.g., Rit-IgG4P). Unstained controls and controls stained with Annexin V-FITC alone or PI alone were also added. Analysis was performed using a FACS Calibur flow cytometer (BD Biosciences) or a GUAVA 96w flow cytometer. Experiments were performed at least twice per antibody variant, preferably at least three times. Data were analyzed using the FlowJo software package (Treestar). Shown is the calculated sum of Annexin V-FITC and Annexin V-FITC/PI positive cells with the background apoptosis (untreated cells) substracted from all samples given as % apoptosis induction ± SD relative to the unmodified non-crosslinked antibody.

### Example 2.1: mAb1 hinge mutants are more effective at inducing direct cytotoxicity than IgG1

One notable difference between IgG2/IgG4P and IgG1 is the lack of amino acids DKT at positions 221-223 in the upper hinge region. Consequently, variants of mAb1 were constructed in which these residues were deleted one at a time (ΔD, ΔK, ΔT), in combinations of two (ΔDT, ΔDK, ΔDT) or entirely (ΔDKT). The antibody variants were then assessed for their apoptotic potential (see Figure 3 and Table 5). Individual deletions of D and K only had a minor enhancing effect on the direct cytotoxic activity (ΔD 116%; ΔK 132%). Removal of T at position 223 moderately increased direct cytotoxicity induction (172%). The removal of two residues, irrespective of the combination, enhanced the observed direct cytotoxic activity levels threefold (ΔDT 302%; ΔDK 300%; ΔKT 300%). The strongest effect was seen when all three amino acids were deleted (ΔDKT 347%).

Additional mutations from either IgG2 or IgG4P to ΔDKT, resulting in mutants uch2 or uch4, respectively, further strengthened direct cytotoxic activity levels. uch2 (571%) was slightly more active than the corresponding IgG2 control (560%); whereas uch4 (419%) was slightly less active than the corresponding IgG4P control (441%). The S219C variant also resulted in a high induction of direct cytotoxicity (538%), as did the double mutant S219C-C220G (366%). The S219Y showed a less pronounced increase direct cytotoxic activity of 226%.

Another difference in the architecture of IgG2 and IgG4P is located in the lower hinge between residues 233-236. Transplanting those features onto IgG1 (mutants Ih2 and Ih4), however, only resulted in a modest increases in direct cytotoxic activity levels (Ih2 129%; Ih4 118%). Other variants P217R, P217S, H224V, T225P, T225E, and H224V-T225E also showed a mild to moderately increase in direct cytotoxic activity.

For cross-lining experiments, cells were incubated with cross-linking antibody (goat anti-human Fc, polyclonal) at a ratio of 1:1 to the test antibody. Notably, cross-linking enhanced the induction of direct cytotoxicity of the IgG1 control, but not the IgG2 or IgG4P controls. Regarding the antibody variants of the invention, cross-linking in many cases could enhance the direct cytotoxic activity of antibody variants (e.g., ΔD, ΔK or ΔT) but not for all. The mutants showing the strongest direct cytotoxic activity (e.g., ΔDKT, uch2, uch4, S219C) could generally not be further enhanced by cross-linking.

**Table 5: Normalised Direct Cytotoxic Activity Values (mAb1)**

| | | **without cross-linking mAb** | | | **with cross-linking mAb** | | |
|---|---|---|---|---|---|---|---|
| **Mutant ID** | **Type** | **Mean normalised apoptosis %** | **Standard deviation %** | **N** | **Mean normalised apoptosis %** | **Standard deviation %** | **N** |
| IgG1 | control | 100 | 0 | 32 | 278 | 104 | 31 |
| IgG2 | control | 560 | 160 | 31 | 570 | 181 | 31 |
| IgG4P | control | 441 | 123 | 17 | 260 | 97 | 17 |
| negative | control | 5 | 15 | 30 | 2 | 14 | 30 |
| D | hinge mutant | 116 | 33 | 6 | 198 | 87 | 5 |
| K | hinge mutant | 132 | 24 | 7 | 286 | 112 | 6 |
| T | hinge mutant | 172 | 56 | 7 | 288 | 116 | 6 |
| DT | hinge mutant | 302 | 84 | 4 | 386 | 114 | 4 |
| DK | hinge mutant | 300 | 63 | 4 | 339 | 93 | 4 |
| KT | hinge mutant | 300 | 74 | 4 | 405 | 37 | 4 |
| DKT | hinge mutant | 347 | 99 | 10 | 224 | 98 | 9 |
| Ih2 | hinge mutant | 129 | 31 | 4 | 292 | 129 | 4 |
| Ih4 | hinge mutant | 118 | 49 | 8 | 180 | 53 | 7 |
| uch2 | hinge mutant | 571 | 135 | 4 | 495 | 145 | 4 |
| uch4 | hinge mutant | 419 | 68 | 4 | 344 | 134 | 4 |
| P217R | hinge mutant | 138 | 29 | 5 | 293 | 105 | 5 |
| P217S | hinge mutant | 113 | 43 | 5 | 302 | 107 | 5 |
| S219C | hinge mutant | 538 | 159 | 5 | 538 | 132 | 5 |
| S219Y | hinge mutant | 226 | 80 | 4 | 345 | 67 | 4 |
| C220G | hinge mutant | 106 | 30 | 5 | 359 | 93 | 5 |
| S219C-C220G | hinge mutant | 366 | 69 | 5 | 421 | 91 | 5 |
| H224V | hinge mutant | 144 | 25 | 4 | 385 | 150 | 4 |
| H224P | hinge mutant | 106 | 17 | 4 | 322 | 107 | 4 |
| T225P | hinge mutant | 142 | 10 | 4 | 350 | 60 | 4 |
| T225E | hinge mutant | 137 | 9 | 4 | 386 | 81 | 4 |
| H224P-T225P | hinge mutant | 88 | 25 | 5 | 349 | 66 | 5 |
| H224V-T225E | hinge mutant | 138 | 18 | 4 | 389 | 65 | 4 |
| ICH1 | CH1 mutant | 205 | 87 | 7 | 335 | 124 | 7 |
| uCH1 | CH1 mutant | 383 | 78 | 6 | 308 | 87 | 6 |
| S131C | CH1 mutant | 334 | 47 | 4 | 367 | 96 | 4 |
| K133R | CH1 mutant | 140 | 36 | 4 | 306 | 93 | 4 |
| S131C-K133R | CH1 mutant | 406 | 61 | 4 | 380 | 77 | 4 |
| G137EG138S | CH1 mutant | 78 | 16 | 5 | 332 | 126 | 5 |
| S192N | CH1 mutant | 144 | 52 | 6 | 314 | 112 | 6 |
| L193F | CH1 mutant | 149 | 40 | 5 | 356 | 128 | 5 |
| S192N-L193F | CH1 mutant | 146 | 36 | 4 | 331 | 113 | 4 |
| Q196K | CH1 mutant | 131 | 36 | 4 | 324 | 122 | 4 |
| R214T | CH1 mutant | 116 | 13 | 5 | 314 | 88 | 5 |
| uch2-S131C | hinge/CH1 combination | 593 | 206 | 4 | 606 | 166 | 4 |
| uch4-S131C | hinge/CH1 combination | 556 | 189 | 4 | 510 | 116 | 4 |
| R214T-P217R | hinge/CH1 combination | 112 | 38 | 4 | 309 | 154 | 4 |
| R214T-P217S | hinge/CH1 combination | 121 | 39 | 2 | 351 | 139 | 2 |

### Example 2.2: mAb1 CH1 mutants are more effective at inducing direct cytotoxicity than IgG1

Further, the involvement of the CH1 domain (Figure 4) was analysed for mAb1. The major structural differences between IgG1, IgG2 and IgG4P are located between residues 131-138 (the upper CH1) and 192-214 (the lower CH1). Hence antibody variants uCH1 and ICH1 that incorporate several of those variations into an IgG1 backbone were constructed (see Figure 4). Both modifications increased direct cytotoxic activity of the mAb1 backbone (Figure 5 and Table 5), though uCH1 (383%) is stronger than ICH1 (205%).

Variants at other residues were also assessed: S131C, K133R, S131C-K133R and G137E-G138S for the upper CH1 domain and S192N, L193F, S192N-L193F, Q196K and R214T for the lower CH1 domain. S131C appears to be a critical mutant, as the direct cytotoxic activity was increased more than 3 fold (334%) by this single exchange. K133R showed only a modestly increase (140%), whereas G137E-G138S slightly decreases the antibody's apoptotic potential (78%). The double modification S131C-K133R showed an increase of 406%.

The lower CH1 domain mAb1 variants resulted in modest increases, ranging from 116% for R214T to 149% for L193F, although these levels were all significantly increased by cross-linking to levels of about 300%.

### Example 2.3: mAb1 hinaelCH1 mutant combinations are more effective at inducing direct cytotoxicity than IgG1

Combinations of the amino acid modifications in the hinge and CH1 regions of mAb1 were tested for their direct cytotoxic activity (Figure 6 and Table 5). The R214T-P217R and R214T-P217S combinations resulted in a modest increase in direct cytotoxic activity by 112% and 121%, respectively. However, the uch2-S131C and uch4-S131C combinations resulted in enhanced direct cytotoxic activity, with 593% for uch2-S131C and 556% for uch4-S131C, which is higher than S131C, uCH2 or uCH4 alone. Cross-linking did not further enhance the direct cytotoxic activity for uch2-S131C and uch4-S131C, but did so for R214T-P217R and R214T-P217S.

In view of the above, it will be appreciated that the present invention also relates to the following items:
1. An antibody having a variant human IgG1 heavy chain constant (CH) region comprising at least a CH1 and/or upper hinge region, wherein the CH1 region has the amino acid sequence of SEQ ID NO: 5 corresponding to residues 118-215 of the Eu numbering and the upper hinge region has the amino acid sequence of SEQ ID NO: 6 corresponding to residues 216-225 of the Eu numbering; and
   one or more amino acid modification(s) within said CH1 and/or upper hinge region, wherein at least one of said one or more amino acid modification(s) is selected from the group consisting of
   (i) substitution S131C,
   (ii) substitutions I199T and N203D,
   (iii) substitution S219C,
   (iv) deletion of at least two of D221, K222 and T223, and
   (v) any combinations thereof,
   wherein the numbering is according to the Eu numbering.
2. The antibody of item 1, wherein the at least one of said one or more amino acid modification(s) is selected from the group consisting of
   (i) substitution S131C,
   (ii) substitutions I199T and N203D,
   (iv) deletion of at least two of D221, K222 and T223, and
   (v) any combinations thereof.
3. The antibody of item 2, wherein the at least one of said one or more amino acid modification(s) is selected from the group consisting of
   (i) substitution S131C,
   (iv) deletion of at least two of D221, K222 and T223, and
   (v) substitution S131C and deletion of at least two of D221, K222 and T223.
4. The antibody of items 2 or 3, further comprising the substitution S219Y.
5. The antibody of any one of the preceding items, wherein the antibody comprises at least the substitution S219C or S219Y and the deletion of at least two of D221, K222 and T223.
6. The antibody of any one of the preceding items, wherein the deletion is a deletion of D221 and K222, of D221 and T223, of K222 and T223 or of D221, K222 and T223.
7. The antibody of any one of the preceding items, wherein the deletion is a deletion of D221, K222 and T223.
8. The antibody of any one of the preceding items further comprising the substitution
   (a) R214T,
   (b) P217R or P217S,
   (c) H224V or H224 P and/or
   (d) T225E or T225P.
9. The antibody of item 1, comprising at least the substitutions R214T, P217R, S219C, H224V and T225E and the deletions of D221, K222 and T223.
10. The antibody of item 4, comprising at least the substitutions P217S, S219Y, H224P and T225P and the deletions of D221, K222 and T223.
11. The antibody of any one of the preceding items, further comprising the substitution K133R.
12. The antibody of items 11, wherein the antibody comprises at least the substitutions S131C and K133R.
13. The antibody of any one of the preceding items, further comprising the substitutions G137E and/or G138S.
14. The antibody of any one of the preceding items, wherein the antibody comprises at least the substitutions S131C, K133R, G137E and G138S.
15. The antibody of any one of the preceding items, wherein the antibody comprises at least the substitutions S131C, K133R, G137E, G138S, 1199T and N203D.
16. The antibody of any one of items 1 to 3, wherein the antibody comprises at least the substitutions S131C, S219C and the deletion of at least two of D221, K222 and T223.
17. The antibody of any one of the preceding items, wherein said CH1 region comprises 6 or less amino acid modifications and/or said hinge region comprises 6 or less amino acid modifications.
18. The antibody of any one of the preceding items, wherein the antibody is a chimeric antibody, a humanized antibody or a human antibody.
19. The antibody of any one of the preceding items, wherein the antibody further comprises one or more substitutions in the Fc region to alter effector functions selected from ADCC, ADCP and CDC.
20. The antibody of any one of the preceding items, wherein the antibody is a single chain antibody, a bispecific antibody or an antibody fragment, preferably a F(ab')2 fragment.
21. The antibody of any one of the preceding items, wherein said antibody has an enhanced direct cytotoxic activity compared to an antibody without said amino acid modification(s).
22. The antibody of item 21, wherein
   (a) the direct cytotoxic activity is induced by receptor crosslinking, receptor dimerization, blocking of receptor dimerization, by signalling involving a death domain or by blocking ligand receptor interactions; and/or
   (b) the direct cytotoxic activity is apoptosis.
23. The antibody of any one of the preceding items, wherein the antibody binds to a cancer-related antigen.
24. The antibody of any one of items 1-23, wherein the antibody binds to a target antigen selected from the group consisting of CD20, CD22, CD30, CD33, CD37, CD44v6, CD52, EGFR2 (HER2), EGFR1 (HER1), preferably the antibody binds to a target antigen selected from the group consisting of CD20, CD37, EGFR1 (HER2) and CD44v6.
25. The antibody of any one of items 1-24, wherein the antibody is an anti-CD20, anti-CD30, anti-CD37, anti-CD52, anti-CD44v6, anti-EGFR1, anti-EGFR2 antibody.
26. The antibody of any one of the preceding items, wherein the antibody has an enhanced direct cytotoxic activity compared to an antibody without said amino acid modification(s) and preferably wherein said enhanced direct cytotoxic activity is detected by increased Annexin V staining.
27. A DNA molecule comprising a sequence encoding the heavy chain of an antibody of any one of items 1 to 26.
28. A vector comprising the DNA of item 27.
29. A pharmaceutical composition comprising the antibody of items 1 to 26 and a pharmaceutically acceptable carrier and optionally pharmaceutically acceptable excipients.
30. Use of the antibody of any one of items 1 to 26 for inducing direct cytotoxicity *in vitro.*
31. The antibody of any one of items 1 to 26 for use in therapy.
32. The antibody of any one of items 1 to 26 for use in promoting or inducing direct cytotoxicity in therapy.
33. The antibody of item 23 for use in treating cancer.
34. The antibody of item 23 for use in promoting or inducing direct cytotoxicity in a cancer cell.
35. A method of treating a mammal in need thereof comprising administering an antibody of any one of items 1 to 26 to said mammal.
36. A method of treating a mammal in need thereof, comprising administering an antibody of any one of items 1-26 to said mammal, wherein the antibody promotes or induces direct cytotoxicity in a cancer cell.
37. A method for enhancing the direct cytotoxicity of an antibody having a human IgG1 heavy chain constant (CH) region comprising at least a CH1 and/or upper hinge region, comprising the step of modifying one or more amino acid(s) of the CH1 and/or upper hinge region.
38. The method of item 33, wherein the CH1 region to be modified corresponds to residues 118-215 according to the Eu numbering and the upper hinge region to be modified corresponds to residues 216-225 of the Eu numbering, preferably, wherein the CH1 region to be modified comprises the amino acid sequence of SEQ ID NO: 5 and the upper hinge region to be modified comprises the amino acid sequence of SEQ ID NO: 6.
39. The method of items 37 or 38, wherein the modification of one or more amino acid(s) is a modification to the corresponding residue of IgG2 having the amino acid sequence of SEQ ID NO: 3 or a modification to the corresponding residue of IgG4P having the amino acid sequence of SEQ ID NO: 4.
40. The method of any one of items 37 to 39, comprising modifying one or more amino acid(s) within said CH1 and/or upper hinge region, wherein at least one of said one or more amino acid modification(s) is selected from the group consisting of:
   (i) substitution S131C,
   (ii) substitutions I199T and N203D,
   (iii) substitution S219C or S219Y,
   (iv) deletion of at least two of D221, K222 and T223, and
   (v) combinations thereof,
      wherein the numbering is according to the Eu numbering.
41. The method of item 40, comprising modifying one or more amino acid(s) within said CH1 and/or upper hinge region, wherein at least one of said one or more amino acid modification(s) is selected from the group consisting of
   (i) substitution S131C,
   (ii) substitutions I199T and N203D,
   (iii) substitution S219C,
   (iv) deletion of at least two of D221, K222 and T223, and
   (v) any combinations thereof.
42. The method of item 41, comprising modifying one or more amino acid(s) within said CH1 and/or upper hinge region, wherein at least one of said one or more amino acid modification(s) is selected from the group consisting of
   (i) substitution S131C,
   (ii) substitutions I199T and N203D,
   (iv) deletion of at least two of D221, K222 and T223, and
   (v) any combinations thereof.
43. The method of item 42, further comprising substituting S219Y.
44. The method of any one of items 37 to 43, wherein said antibody has an enhanced direct cytotoxic activity compared to an antibody without said amino acid modification(s) and preferably wherein the enhanced direct cytotoxicity is detected by increased Annexin V staining.
45. A method for producing an antibody of any one of items 1 to 26, comprising
   (a) transfecting a mammalian host cell with the DNA of item 27 or the vector of item 28,
   (b) optionally transfecting the mammalian host cell with a DNA comprising a sequence encoding an antibody light chain,
   (c) culturing the host cell under conditions suitable for expressing the antibody, and
   (d) recovering and purifying the antibody molecule.
46. An antibody obtainable by the method of any one of items 37-45.

Those skilled in the art will recognize, or be able to ascertain using no more than routine experimentation, numerous equivalents to the specific embodiments described herein both in the Examples and in the body of the entire patent description. Such equivalents are considered to be within the scope of this invention and are intended to be encompassed by the following claims.

## Claims

1. An antibody having a variant human IgG1 heavy chain constant (CH) region comprising at least a CH1 and/or upper hinge region, wherein the CH1 region has the amino acid sequence of SEQ ID NO: 5 corresponding to residues 118-215 of the Eu numbering and the upper hinge region has the amino acid sequence of SEQ ID NO: 6 corresponding to residues 216-225 of the Eu numbering; and
one or more amino acid modification(s) within said CH1 and/or upper hinge region, wherein at least one of said one or more amino acid modification(s) is selected from the group consisting of
(i) substitution S131C,
(ii) substitutions I199T and N203D,
(iii) substitution S219C,
(iv) deletion of at least two of D221, K222 and T223, and
(v) any combinations thereof,
wherein the numbering is according to the Eu numbering; and
wherein said antibody has an enhanced direct cytotoxic activity compared to an antibody without said amino acid modification(s).

2. The antibody of claim 1, wherein the at least one of said one or more amino acid modification(s) is selected from the group consisting of
(i) substitution S131C,
(ii) substitutions I199T and N203D,
(iv) deletion of at least two of D221, K222 and T223, and
(v) any combinations thereof.

3. The antibody of claim 2, further comprising the substitution S219Y.

4. The antibody of any one of the preceding claims further comprising the substitution
(a) R214T,
(b) P217R or P217S,
(c) H224V or H224 P and/or
(d) T225E or T225P.

5. The antibody of claim 1, comprising at least
(a) the substitutions R214T, P217R, S219C, H224V and T225E and the deletions of D221, K222 and T223,
(b) the substitutions S131C, S219C and the deletion of at least two of D221, K222 and T223, or
(c) the substitution S219C and the deletions of at least two of D221, K222 and T223.

6. The antibody of claim 3 comprising at least
(a) the substitutions P217S, S219Y, H224P and T225P and the deletions of D221, K222 and T223; or
(b) the substitution S219Y and the deletions of at least two of D221, K222 and T223.

7. The antibody of any one of the preceding claims, wherein the deletion is a deletion of D221 and K222, of D221 and T223, of K222 and T223 or of D221, K222 and T223, preferably wherein the deletion is a deletion of D221, K222 and T223.

8. The antibody of any one of the preceding claims, further comprising
(a) the substitution K133R, preferably wherein the antibody comprises at least the substitutions S131C and K133R; and/or
(b) the substitutions G137E and/or G138S.

9. The antibody of any one of the preceding claims, wherein said CH1 region comprises 6 or less amino acid modifications and/or said hinge region comprises 6 or less amino acid modifications.

10. The antibody of any one of the preceding claims, wherein
(a) the antibody is a chimeric antibody, a humanized antibody or a human antibody;
(b) the antibody further comprises one or more substitutions in the Fc region to alter effector functions selected from ADCC, ADCP and CDC;
(c) the antibody is a single chain antibody, a bispecific antibody or an antibody fragment, preferably a F(ab')2 fragment.

11. The antibody of any one of the preceding claims, wherein the antibody binds to a cancer-related antigen.

12. The antibody of any one of the preceding claims, wherein the enhanced direct cytotoxic activity is detected by increased Annexin V staining.

13. A DNA molecule comprising a sequence encoding the heavy chain of an antibody of any one of claims 1 to 12.

14. A vector comprising the DNA of claim 13.

15. A pharmaceutical composition comprising the antibody of claims 1 to 12 and a pharmaceutically acceptable carrier and optionally pharmaceutically acceptable excipients.

16. The antibody of any one of claims 1 to 12 for use in therapy.

17. The antibody of claim 11 for use in treating cancer.

18. A method for enhancing the direct cytotoxicity of an antibody having a human IgG1 heavy chain constant (CH) region comprising at least a CH1 and/or upper hinge region, comprising the step of modifying one or more amino acid(s) of the CH1 and/or upper hinge region.

19. The method of claim 18, comprising modifying one or more amino acid(s) within said CH1 and/or upper hinge region, wherein at least one of said one or more amino acid modification(s) is selected from the group consisting of:
(i) substitution S131C,
(ii) substitutions I199T and N203D,
(iii) substitution S219C or S219Y,
(iv) deletion of at least two of D221, K222 and T223, and
(v) combinations thereof,
wherein the numbering is according to the Eu numbering; and
wherein said antibody has an enhanced direct cytotoxic activity compared to an antibody without said amino acid modification(s).

20. The method of claims 18 or 19, wherein the enhanced direct cytotoxicity is detected by increased Annexin V staining.

21. A method for producing an antibody of any one of claims 1 to 12, comprising
(e) transfecting a mammalian host cell with the DNA of claim 13 or the vector of claim 14,
(f) optionally transfecting the mammalian host cell with a DNA comprising a sequence encoding an antibody light chain,
(g) culturing the host cell under conditions suitable for expressing the antibody, and
(h) recovering and purifying the antibody molecule.
